# EUROPEAN PATENT SPECIFICATION

(11) **EP 0 392 225 B1**
(45) Date of publication and mention of the grant of the patent: **28.05.2003**
(21) Application number: 90105336.3
(22) Date of filing: 21.03.1990
(51) Int. Cl.: C12N 15/82, C12N 15/29, C12N 15/56, C12N 15/53, A01N 65/00, A01H 5/00, A01H 1/00

(54) **Disease-resistant transgenic plants**
Krankheitsresistente transgene Pflanze
Plantes transgéniques résistantes aux maladies

(30) Priority: 24.03.1989 US 329018; 20.06.1989 US 368672; 20.10.1989 US 425504
(43) Date of publication of application: 17.10.1990
(73) Proprietor: Syngenta Participations AG, 4058 Basel (CH)
(72) Inventor: Ryals, John A., Dr., Durham, N.C. 27713 (US); Alexander, Danny C., Davis, C.A. 95616 (US); Goodman, Robert M., Davis, C.A. 95616 (US); Meins, Frederick, Prof., CH-4125 Riehen (CH); Payne, George B., Chapel Hill, N.C. 27516 (US); Stinson, Jeffrey R., Davis, C.A. 95616 (US); Neuhaus, Jean-Marc, Dr., CH-4054 Basle (CH); Moyer, Mary B,, Cary, N.C. 27511 (US); Ward, Eric Russell, Durham, N.C. 27701 (US); Williams, Shericca Cherrer, Cary, N.C. 27513 (US)
(74) Representative: Zumstein, Fritz, Dr.

(56) References cited:
- EP-A- 0 292 435
- EP-A- 0 307 841
- EP-A- 0 332 104
- EP-A- 0 418 695
- WO-A-88/00976
- WO-A-88/07585
- WO-A-90/07001
- JOURNAL OF CELLULAR BIOCHEMISTRY, Suppl. 12C, Y210, 28th February - 10th April 1988, page 264, Alan R. Liss Inc., New York, US; J. CUTT et al.: "Pathogenesis-related proteins of nicotiana tabacum"
- THE PLANT CELL, vol. 1, no. 3, March 1989, pages 285-291, Am. Soc. of Plant Physiologists; H.J.M. LINTHORST et al.: "Constitutive expression of pathogenesis-related proteins PR-1, GRP, and PR-S in tobacco has no effect on virus infection"
- EMBO JOURNAL, vol. 5, no. 9, 1986, pages 2057-2061, IRL Press Ltd, Oxford, GB; R.A.M. HOOFT et al.: "cDNA cloning of six mRNAs induced by TMV infection of tobacco and a chacterization of their translation products"
- PLANT MOL. BIOL., vol. 9, 1987, pages 411-420, Martinus Nijhoff Publishers, Dordrecht, NL; R.A.M. HOOFT et al.: "Homology between chitinases that are induced by TMV infection of tobacco"
- PROC. NATL. ACAD. SCI., vol 85, August 1988, pages 5738-5742; J.R. DUGUID et al.: "Isolation of cDNAs of scrapie-modulated RNAs by subtractive hybridization of a cDNA library"
- GENE, vol. 66, 1988, pages 121-134, Elsevier Science Publishers B.V. (Biomedical Division); S.C. PRUITT: "Expression vectors permitting cDNA cloning and enrichment for specific sequences by hybridization/selection"
- GENE, vol. 35, 1985, pages 45-54, Elsevier Science Publishers; J. KOWALSKI: "Vectors for the direct selection of cDNA clones corresponding to mammalian cell mRNA of low abundance"
- J. CELL. BIOCHEM., Suppl. 13D, M232, 27th March - 7th April 1989, page 283, Alan R, Liss Inc., New York, US; P.H. MORGENS et al.: "Cloning and sequencing of a cDNA clone encoding an ethylene-induced peroxidase from cucumber cotyledons"
- J. CELL. BIOCHEM., Suppl. 13D, M014, 27th March - 7th April 1989, page 234, Alan R, Liss Inc., New York, US; S. ROTHSTEIN et al.: "Studies of antisense inhibition of gene expression in tobacco: nopaline synthase and peroxidase"
- CELL, vol. 56, 27th January 1989, pages 215-224, Cell Press; C.J. LAMB et al.: "Signals and transduction mechanisms for activation of plant defenses against microbial attack"

## Description

The present invention relates to chimeric DNA constructs useful for producing transgenic disease-resistant plants and to genetic engineering of plants to produce the phenotype of disease resistance. In particular it relates to constitutive expression in transgenic plants of DNA sequences which encode pathogenesis-related proteins (PRPs). Wild-type plants express PRPs during pathogen-induced systemic acquired resistance.

Advances in recombinant DNA technology coupled with advances in plant transformation and regeneration technology have made it possible to introduce new genetic material into plant cells, plants or plant tissue, thus introducing new traits, e.g., phenotypes, that enhance the value of the plant or plant tissue. Recent demonstrations of genetically engineered plants resistant to pathogens (EP 0 240 332 and EP 0 223 452) or insects (Vaeck et al., 1987) and the production of herbicide tolerant plants (de Block et al., 1987) highlight the potential for crop improvement. The target crops can range from woody plants like trees and shrubs to ornamentals and to field crops.

Cutt et al. (1988) J. Cell Biochem., Suppl. 12c, Abstract Y210, investigates the pathogen resistance state of transgenic plants transformed with PR-protein constructs, but does not disclose results or data on such plants.

The production of transgenic plants which are disease resistant can now be realized by the present invention which is directed to, among other things, chimeric DNA constructions useful for producing transgenic disease-resistant plants. The chimeric DNA constructions contain a coding DNA sequence which encodes a plant PRP which is normally pathogen-induced in a wild type plant, and a promoter DNA sequence which provides for the constitutive expression of PRPs or anti-sense mRNA for PRPs in a transgenic plant containing the chimeric DNA construction.

### A. PATHOGENESIS-RELATED PROTEINS: SYSTEMIC ACQUIRED RESISTANCE (SAR).

So-called pathogenesis-related proteins (PRPs) are plant proteins induced following infection by a pathogen. It is believed that these proteins may have a role in providing systemic acquired resistance to the plant. These plant proteins are induced in large amounts in response to infection by various pathogens, including viruses, bacteria and fungi. PRPs were first discovered in tobacco plants (*Nicotiana tabacum*) reacting hypersensitively to infection with tobacco mosaic virus (TMV). Subsequently, PRPs have been found in many plant species (for reviews see Redolfi, 1983; van Loon, 1985) and are believed to be a common defensive response of plants to infection by pathogens.

As used herein, PRPs are proteins expressed in plants reacting hypersensitively towards pathogens. This term embraces, but is not limited to, SAR8.2a and SAR8.2b proteins, the acidic and basic forms of tobacco PR-1a, PR-1b, PR-1c, PR-1', PR-2, PR-N, PR-O, PR-O', PR-P, PR-Q, PR-S, PR-R proteins, the chitinase which is a basic counterpart of PR-P or PR-Q, and the β-1,3-glucanase (glucan endo-1,3-β-glucosidase, EC 3.2.1.39) which is a basic counterpart of PR-2, PR-N or PR-O and the pathogen-inducible chitinase from cucumber. A hypersensitve reaction is characterized by a local necrosis of the tissues immediately surrounding the pathogen infection site and a subsequent localization of the pathogen. This is in contrast to a sensitive reaction, wherein the pathogen spreads throughout the plant. Pathogens are, for example, viruses or viroids, e.g. tobacco or cucumber mosaic virus, ringspot virus or necrosis virus, pelargonium leaf curl virus, red clover mottle virus, tomato bushy stunt virus, and like viruses, fungi, e.g. *Phytophthora parasitica* or *Peronospora tabacina*, bacteria, e.g. *Pseudomonas syringae* or *Pseudomonas tabaci*, or aphids, e.g. *Myzus persicae.* This list is not intended to be limiting in any respect.

### B. GENERAL OVERVIEW OF PLANT TRANSFORMATION TECHNOLOGY

Various methods are known in the art to accomplish the genetic transformation of plants and plant tissues (i.e., the stable introduction of foreign DNA into plants). These include transformation by *Agrobacterium* species and transformation by direct gene transfer.

### 1. Afrobacterium-mediated Transformations

*Agrobacterium tumefaciens* is the etiologic agent of crown gall, a disease of a wide range of dicotyledons and gymnosperms, that results in the formation of tumors or galls in plant tissue at the site of infection. *Agrobacterium*, which normally infects the plant at wound sites, carries a large extrachromosomal element called the Ti (tumor-inducing) plasmid.

Ti plasmids contain two regions required for tumorogenicity. One region is the T-DNA (transferred-DNA) which is the DNA sequence that is ultimately found stably transferred to plant genomic DNA. The other region required for tumorogenicity is the *vir* (virulence) region which has been implicated in the transfer mechanism. Although the *vir* region is absolutely required for stable transformation, the *vir* DNA is not actually transferred to the infected plant. Transformation of plant cells mediated by infection with *A. tumefaciens* and subsequent transfer of the T-DNA alone have been well documented (Bevan and Chilton, 1982).

After several years of intense research in many laboratories, the *Agrobacterium* system has been developed to permit routine transformation of a variety of plant tissue. Representative tissues transformed in this manner include tobacco, tomato, sunflower, cotton, rapeseed, potato, soybean, and poplar. While the host range for Ti plasmid transformation using *A. tumefaciens* as the infecting agent is known to be very large, tobacco has been a host of choice because of its ease of manipulation.

*Agrobacterium rhizogenes* has also been used as a vector for plant transformation. This bacterium, which incites hairy root formation in many dicotyledonous plant species, carries a large extrachromosomal element called an Ri (root-inducing) plasmid which functions in a manner analogous to the Ti plasmid of *A. tumefaciens*. Transformation using *A. rhizogenes* has developed analogously to that of *A. tumefaciens* and has been successfully utilized to transform, for example, alfalfa, *Solanum nigrum* L., and poplar.

### 2. Direct Gene Transfer

Several so-called direct gene transfer procedures have been developed to transform plants and plant tissues without the use of an *Agrobacterium* intermediate. In the direct transformation of protoplasts, the uptake of exogenous genetic material into a protoplast may be enhanced by use of a chemical agent or electric field. The exogenous material may then be integrated into the nuclear genome. The early work was conducted in the dicot tobacco where it was shown that the foreign DNA was incorporated and transmitted to progeny plants (Paszkowski et al., 1984; Potrykus et al., 1985).

Monocot protoplasts have also been transformed by this procedure in, for example, *Triticum monococcum, Lolium multiflorum* (Italian ryegrass), maize, and Black Mexican sweet corn.

Alternatively exogenous DNA can be introduced into cells or protoplasts by microinjection. A solution of plasmid DNA is mechanically injected directly into the cell, usually using a finely pulled glass needle. In this manner alfalfa protoplasts have been transformed by a variety of plasmids (Reich et al., 1986).

A more recently developed procedure for direct gene transfer involves bombardment of cells by microprojectiles carrying DNA (Klein et al., 1987). In this procedure tungsten particles coated with the exogenous DNA are accelerated toward the target cells, resulting in at least transient expression in the example reported (onion).

### C. REGENERATION OF TRANSFORMED PLANT TISSUE

Just as there is a variety of methods for the transformation of plant tissue, there is a variety of methods for the regeneration of plants from plant tissue. The particular method of regeneration will depend on the starting plant tissue and the particular plant species to be regenerated. In recent years it has become possible to regenerate many species of plants from callus tissue derived from plant explants. The plants which can be regenerated from callus include monocots, such as corn, rice, barley, wheat and rye, and dicots, such as tomato, sunflower, soybean, cotton, rapeseed and tobacco.

Regeneration of plants from tissue transformed with *A. tumefaciens* has been demonstrated for several species of plants. These include sunflower, tomato, white clover, rapeseed, cotton, tobacco, and poplar. The regeneration of alfalfa from tissue transformed with *A. rhizogenes* has also been demonstrated. Plant regeneration from protoplasts is a particularly useful technique (Evans and Bravo, 1983). When a plant species can be regenerated from protoplasts, direct gene transfer procedures can be utilized, and transformation is not dependent on the use of *A. tumefaciens.* Regeneration of plants from protoplasts has been demonstrated for rice, tobacco, rapeseed, potato, eggplant, cucumber, poplar, and corn.

Various plant tissues may be utilized for transformation with foreign DNA. For instance, cotyledon shoot cultures of tomato have been utilized for *Agrobacterium* mediated transformation and regeneration of plant (EP 0 249 432). Further examples include *Brassica* species (WO 87/07299) and woody plant species, particularly poplar (US 4,795,855).

### D. FIELD OF THE INVENTION

One of the objects of the present invention is to provide transgenic plants constitutively expressing induced levels of plant PRPs or substantially homologous proteins. A further object is to provide transgenic plants constitutively expressing such proteins, providing an enhanced disease-resistant phenotype with respect to wild-type plants. Another object of the present invention is to provide transgenic plants constitutively transcribing sense or antisense mRNA strands of DNA sequences encoding plant PRPs or transcribing sense or antisense mRNA strands of DNA sequences substantially homologous to genomic or cDNA sequences encoding plant PRPs, such transgenic plants thus having an enhanced disease-resistant phenotype with respect to wild-type plants.

Accordingly, to meet these objectives and others, the present invention disclosed herein includes
(a) chimeric DNA constructions useful for producing transgenic disease-resistant plants which comprise a first DNA sequence which promotes in a plant the constitutive transcription of the second DNA sequence, and a second DNA sequence which is a coding sequence of an inducible plant PRPs or a coding sequence having substantial sequence homology to a coding sequence of an inducible plant PRPs;
(b) vectors containing such chimeric DNA constructions; and
(c) transgenic plants, transgenic plant tissue, propagules and seeds of transgenic plants containing the chimeric DNA constructions for producing disease-resistant plants.

In particular, the present invention discloses a chimeric DNA sequence which comprises:
(a) a first DNA sequence which promotes in a plant the constitutive transcription of a second DNA sequence, and
(b) a second DNA sequence comprising a coding sequence of an inducible plant pathogenesis-related protein, or a coding sequence having substantial sequence homology to a coding sequence of an inducible plant pathogenesis-related protein, said second DNA sequence being in either a sense or antisense orientation with respect to said first DNA sequence.

Preferred is a chimeric DNA sequence, wherein the first DNA sequence is of heterologous origin with respect to the second DNA sequence.

Especially preferred is a chimeric DNA sequence, wherein the first DNA sequence comprises the promoter of the small subunit of tobacco ribulose bis-phosphate carboxylase (RUBISCO) or a double CaMV 35S promoter.

The second DNA sequence of the chimeric DNA preferably encodes a plant pathogenesis-related protein which is selected from the group consisting of PR-1A, PR-1B, PR-1C, PR-R major, PR-R minor, PR-P, PR-Q, PR-2, PR-N, PR-O, PR-O', SAR8.2a, SAR8.2b, cucumber chitinase/lysozyme, cucumber basic peroxidase, tobacco basic glucanase and tobacco basic chitinase.

Also preferred is a chimeric DNA sequence, wherein the second DNA sequence encodes a plant pathogenesis-related protein selected from the group consisting of PR-1A, PR-1B, PR-1C, PR-R major, PR-R minor, PR-P, PR-Q, PR-O', SAR8.2a, SAR8.2b, cucumber chitinase/lysozyme, tobacco basic glucanase and tobacco basic chitinase.

Especially preferred is a chimeric DNA sequence, wherein the second DNA sequence encodes a plant pathogenesis-related protein selected from the group consisting of PR-R major, PR-R minor, PR-P, PR-Q, PR-2, PR-N, PR-O, PR-O', SAR8.2a, SAR8.2b, cucumber chitinase/lysozyme, cucumber basic peroxidase, tobacco basic glucanase and tobacco basic chitinase.

Further disclosed are recombinant DNA molecules which contain a chimeric DNA sequence according to the present invention.
Preferably, a recombinant DNA molecule is characterised in that it is a vector molecule. Especially preferred is a vector molecule comprising pCGN1703.

A method for producing the above chimeric DNA sequences, which method comprises:
(a) preparing from a suitable source such as, for example, a plant RUBISCO gene or a CaMV genome a first DNA sequence which promotes in a plant the constitutive transcription of a second DNA sequence;
(b) preparing a DNA library from pathogen infected or from infected and uninfected plant tissue, the said DNA library thus comprising induced or both induced and uninduced DNA populations and isolating therefrom a second DNA sequence comprising a coding sequence of an inducible plant pathogenesis-related protein, or a coding sequence having substantial sequence homology to a coding sequence of an inducible plant pathogenesis-related protein; and
(c) operably linking the first DNA seqeuence of step (a) with the second DNA sequence of step (b) such that the said first DNA sequence promotes the transcription of the second DNA sequence using methods known in the art.

Linking the first and second DNA sequence is preferably carried out such that the second DNA sequence is in either a sense or an antisense orientation with respect to said first DNA sequence.

Also comprised by the present invention are plants, plant tissue, plant propagules or plant seeds containing anyone of the above described chimeric DNA sequences as well as methods for producing same.

Preferred within the present invention is a transgenic plant constitutively expressing induced levels of plant pathogenesis-related proteins which provide a disease-resistant phenotype.

Further preferred is a transgenic plant constitutively transcribing sense or antisense strands of DNA sequences encoding a plant pathogenesis-related protein which provides a disease-resistant phenotype.

Especially preferred is a plant, plant tissue, plant propagules or plant seed containing a chimeric DNA sequence comprising a first DNA sequence comprising the promoter of the small subunit of tobacco ribulose bis-phosphate carboxylase (RUBISCO) and a second DNA sequence comprising the coding sequence of PR-1A pathogenesis-related protein, wherein the second DNA sequence is oriented with respect to the first DNA sequence such that the first DNA sequence promotes transcription of the antisense strand of the second DNA sequence.

Also provided herein is a method for producing a transgenic plant cell or plant tissue having the potential to regenerate into a whole plant exhibiting a disease-resistant phenotype, comprising transforming the plant cell or tissue, which may exist in form of an *in vitro* culture or as part of an embryo or a whole plant, with a chimeric DNA sequence comprising a first DNA sequence which promotes in a plant the constitutive transcription of a second DNA sequence, and a second DNA sequence comprising a coding sequence of an inducible plant pathogenesis-related protein, or a coding sequence having substantial sequence homology to a coding sequence of an inducible plant pathogenesis-related protein, said first DNA sequence being linked to the second DNA sequence such that it promotes the transcription of the second DNA sequence.

Transformation of plant cells or plant tissue can be carried out by means of the transformation methods described hereinbefore using either an isolated plant cell or plant tissue being part of a cell or tissue culture or a plant cell or plant tissue being part of a plant embryo or a whole plant.

Further preferred is a method for producing a transgenic plant cell or plant tissue, wherein the second DNA sequence is in either a sense or an antisense orientation with respect to said first DNA sequence.

Also provided herein are novel cDNA clones coding for plant PRPs. Within the present invention the following substantially pure cDNA sequences are preferred:
(1) A substantially pure cDNA sequence encoding SAR8.2a, or a DNA sequence having substantial sequence homology to cDNA encoding SAR 8.2a, but in particular plasmid pCIB/SAR8.2a, ATCC accession number 40584, deposited March 22, 1989.
(2) A substantially pure cDNA sequence encoding SAR8.2b, or a DNA sequence having substantial sequence homology to cDNA encoding SAR 8.2b, but in particular plasmid pCIB/SAR8.2b, ATCC accession number 40585, deposited March 22, 1989.
(3) A substantially pure cDNA sequence encoding PR-P, or a DNA sequence having substantial sequence homology to cDNA encoding PR-P, but in particular plasmid pBScht28, ATCC accession number 40588, deposited March 24, 1989.
(4) A substantially pure cDNA sequence encoding PR-2, or a DNA sequence having substantial sequence homology to cDNA encoding PR-2, but in particular plasmid pBSGL117, ATCC accession number 40691, deposited Octpber 19, 1989.
(5) A substantially pure cDNA sequence encoding PR-N, or a DNA sequence having substantial sequence homology to cDNA encoding PR-N, but in particular plasmid pBSGL148, ATCC accession number 40689, deposited October 19, 1989.
(6) A substantially pure cDNA sequence encoding PR-O, or a DNA sequence having substantial sequence homology to cDNA encoding PR-O, but in particular plasmid pBSGL134, ATCC accession number 40690, deposited October 19, 1989.
(7) A substantially pure cDNA sequence encoding PR-2', or a DNA sequence having substantial sequence homology to cDNA encoding PR-2', but in particular plasmid pBSGL135, ATCC accession number 40685, deposited October 19, 1989.
(8) A substantially pure cDNA sequence encoding cucumber peroxidase, or a DNA sequence having substantial sequence homology to cDNA encoding cucumber peroxidase, but in particular plasmid pBPERI, ATCC accession number 40686, deposited October 19, 1989 or plasmid pBPERB24, respectively ATCC accession number 40687, deposited October 19, 1989.
(9) A substantially pure DNA sequence of "Sequence 6" or having substantial sequence homology to the sequence shown in "Sequence 6."
(10) A substantially pure DNA sequence of "Sequence 9" or having substantial sequence homology to the sequence shown in "Sequence 9."
(11) A substantially pure DNA sequence of "Sequence 10" or having substantial sequence homology to the sequence shown in "Sequence 10."

Further provided is a novel method for differential screening and enriching cDNA populations comprising
(a) providing single-stranded cDNA from induced and uninduced cDNA populations, the single-stranded cDNA from the induced and uninduced populations having opposite DNA polarity, and the cDNA from the uninduced population having a biotin-affinity tag;
(b) hybridizing the single-stranded cDNA populations of step (a) with each other; and
(c) separating the hybridized mixture of step (b) by biotin-avidin chromatography to enrich for single-stranded cDNAs from the induced population which are not hybridized to the cDNA from the uninduced population.

Further provided herein is a method for cloning cDNA's encoding disease-resistance proteins comprising
(a) providing tissue induced to systemic acquired resistance or localized acquired resistance using biological inducers, and
(b) isolating cDNA clones encoding disease-resistance proteins. Although cDNAs have been produced previously from RNA isolated from pathogen-infected material, this is the first example of producing cDNAs from RNA isolated from uninfected portions of the plant that have been induced to resistance by pathogens. That is, the tissue is uninfected by pathogens, and is demonstrating acquired resistance.

The chimeric genes of the present examples which are described below comprise plant cDNA sequences encoding PRPs. However, this invention applies equally to genomic clones and synthetic homologous sequences which encode PRPs or proteins having substantial homology thereto. Thus the scope of the claims of the present invention are not intended to be limited to the disclosed specific embodiments.

### E. EXAMPLES

### Deposits with ATCC

The following deposits have been made with the ATCC in accordance with the Budapest Treaty:

| Plasmid | ATCC Accession No. | Date of Deposit |
|---|---|---|
| pCGN783 | 67868 | December 23,1988 |
| pBS-Gluc 39.1 | 40526 | December 29, 1988 |
| pBScucchi/chitinase | 40528 | December 29, 1988 |
| (aka pBScuccht5) | | |
| pBSGL6e. | 40535 | January 18, 1989 |
| pCIB/SAR8.2a | 40584 | March 22, 1989 |
| pCIB/SAR8.2b | 40585 | March 22, 1989 |
| pCGN1540 | 40586 | March 22, 1989 |
| pCGN2113 | 40587 | March 22, 1989 |
| pBScht28 | 40588 | March 24, 1989 |
| pBSGL135 | 40685 | October 19, 1989 |
| pBSPER1 | 40686 | October 19, 1989 |
| pBSPERB24 | 40687 | October 19, 1989 |
| pBSPERB25 | 40688 | October 19, 1989 |
| pBSGL148 | 40689 | October 19, 1989 |
| pBSGL134 | 40690 | October 19, 1989 |
| pBSGL117 | 40691 | October 19, 1989 |
| pBSGL125 | 40692 | October 19, 1989 |
| λ tobcDNAGL162* | 40693 | October 19, 1989 |
| λ tobcDNAGL153* | 40694 | October 19, 1989 |
| λ tobcDNAGL161* | 40695 | October 19, 1989 |

| | | |
|---|---|---|
| * in *Escherichia coli* | | |

### Definitions

In order to provide a clear and consistent understanding of the specification and the claims, including the scope given to such terms, the following definitions are provided:

Chimeric Sequence or Gene: A DNA sequence containing at least two heterologous parts, e.g., parts derived from naturally occurring DNA sequences which are not associated in their naturally occurring states, or containing at least one part that is of synthetic origin and not found in nature.

Coding DNA Sequence: A DNA sequence which, when transcribed and translated, results in the formation of a cellular polypeptide.

Constitutive transcription: transcription of substantially fixed amounts of a DNA sequence, irrespective of environmental conditions.

Gene: A discrete chromosomal region which is responsible for a discrete cellular product.

Inducers: Molecules that cause the production of larger amounts of macromolecules, compared to the amounts found in the absence of the inducer.

Inducible Protein: Proteins whose rate of production can be increased by the presence of inducers in the environment.

Non-coding DNA Sequence: A DNA sequence which is not translated resulting in the formation of a cellular polypeptide when associated with a particular coding DNA sequence. Thus, for example, a sequence that is non-coding when associated with one coding sequence may actually be coding when associated with another coding or non-coding sequence.

Phenotypic Trait: An observable property resulting from expression of a gene.

Plant Tissue: Any tissue of a plant in planta or in culture. This term includes, but is not limited to, whole plants, plant cells, plant organs, plant seeds, protoplasts, callus, cell cultures and any groups of plant cells organized into structural and/or functional units. The use of this term in conjunction with, or in the absence of, any specific type of plant tissue as listed above or otherwise embraced by this definition is not intended to be exclusive of any other type of plant tissue.

Substantially Pure DNA Sequence: A DNA sequence isolated in substantially pure form from a natural or non-natural source. Such a sequence may occur in a natural system, for example, in bacteria, viruses or in plant or animal cells, or may be provided, for example, by synthetic means or as a cDNA sequence. Substantially pure DNA sequences are usely isolated in form of a vector comprising the intended DNA. Substantially pure means that other DNA sequences than the ones intended are present only in marginal amounts, for example less than 5 %, less than 1 %, or preferably less than 0.1 %. Substantially pure DNA sequences and vectors containing them may be, and typically are, provided in solution, for example in aqueous solution containing buffers or in the usual culture media.

Substantial Sequence Homology: Substantial sequence homology means close structural relationship between sequences of nucleotides or amino acids. For example, substantially homologous DNA sequences may be 60 % homologous, preferably 80 % or 90 % homologous, and substantially homologous amino acid sequences may typically be 50 % homologous, or more. Homology also includes a relationship wherein one or several subsequences of nucleotides or amino acids are missing, or subsequences with additional nucleotides or amino acids are interdispersed.

### Abbreviations

- bp: base pair
- kb: kilo base pair
- ATCC: American Type Culture Collection, Rockville, MD
- TMV: Tobacco Mosaic Virus
- TNV: Tobacco Necrosis Virus
- HPLC: high pressure liquid chromatography
- Cvcpm: Cerenkov counts per minute
- w/v: weight/volume except of where otherwise stated
- ICF: intracellular fluid
- PAGE: polyacrylamide gel electrophoresis
- PCR: Polymerase-catalyzed Chain Reaction
- PCV: packed cell volume: settled cell volume in a pipette
- MW: molecular weight
- LGT: low gelling temperature
- ATP: adenosine triphosphate
- BSA: bovine serum albumin
- CETAB: hexadecyltrimethylammonium bromide
- 2,4-D: 2,4-dichlorophenoxyacetic acid
- DTT: dithiothreitol
- dicamba: 3,6-dichloro-2-methoxybenzoic acid
- EDTA: ethylendiamine N,N,N',N'-tetraacetic acid
- MES: 2-(N-morpholino)ethanesulfonic acid
- MU: 4-methyl umbelliferyl glucuronide
- PEG: polyethylene glycol
- picloram: 4-amino-3,5,6-trichloropicolinic acid
- SDS: sodium dodecyl sulfate
- TFA: trifluoroacetic acid
- Tris-HCl: tris(hydroxymethyl)methylamine hydrochloride
- PRP: pathogenesis-related proteins
- CIAP: calf intestinal alkaline phosphatase
- PTH: Phenylthiohydantoin
- RUBISCO: ribulose-1,5-bis-phosphate carboxylase
- NAA: α-naphthaleneacetic acid
- BAP: 6-benzylaminopurine

### Media and buffers

Enzyme reactions are conducted in accordance with the manufacturer's recommended procedures unless otherwise indicated. The chimeric genes and vectors of the present invention are constructed using techniques well known in the art. Suitable techniques have been described in Maniatis et al. (1982); Methods in Enzymology, Volumes 68, 100, 101 and 118 (1979, 1983, 1983 and 1986); and Glover (1985). Medium compositions have been described in Miller (1972), as well as in the references previously identified.

SH-0 medium: Medium of Schenk and Hildebrandt (1972) without hormones. SH medium can be liquid or solidified with 0.8 % agar or with 0.5 % GelRite®. The medium is normally sterilized by heat in an autoclave at about 125°C for 15 to 20 min.

SH-30 medium: SH-0 medium containing 30 µm Dicamba.

SH-45 medium: SH-0 medium containing 45 µm Dicamba.

OMS medium: Medium of Murashige and Skoog (1962). The medium can be solidified with 0.8 % agar or agarose or with 0.5 % GelRite®.

| | | |
|---|---|---|
| KM-8p and N6 medium: Macroelements^{a}, microelements^{a} and Fe-EDTA are as given in the literature: KM medium according to Kao and Michayluk (1975); N6 medium according to Chu et al. (1975) | | |

| Media: | KM-8p^{b,c,d} | N6 |
|---|---|---|
| Organics and vitamins^{e} [mg/l]: | | |
| biotin | 0.01 | |
| pyridoxine-HCl | 1.00 | 0.5 |
| thiamine-HCl | 10.00 | 0.1 |
| nicotinamide | 1.00 | |
| nicotinic acid | 0.10 | 0.5 |
| folic acid | 0.40 | |
| D-Ca-pantothenate | 1.00 | |
| p-aminobenzoic acid | 0.02 | |
| choline chloride | 1.00 | |
| riboflavin | 0.20 | |
| Vitamin B 12 | 0.02 | |
| glycine | 0.10 | 2.0 |
| | | |
| Sugars and sugar alcohols [g/l]: | | |
| sucrose | 0.25 | 30.0 |
| glucose | 68.40 | |
| mannitol | 0.25 | |
| sorbitol | 0.25 | |
| cellobiose | 0.25 | |
| fructose | 0.25 | |
| mannose | 0.25 | |
| rhamnose | 0.25 | |
| ribose | 0.25 | |
| xylose | 0.25 | |
| myo-inositol | 0.10 | |
| | | |
| Final pH | 5.8 | 5.6 |
| Sterilization | filter | autoclaved |

| | | |
|---|---|---|
| ^{a}Macroelements are usually made up as a 10 X concentrated stock solution, and microelements as a 1000 X concentrated stock solution. | | |
| ^{b}Citric, fumaric and malic acid (each 40 mg/l final concentration) and sodium pyruvate (20 mg/l final concentration) are prepared as a 100 X concentrated stock solution, adjusted to pH 6.5 with NH₄OH, and added to this medium. | | |
| ^{c}Adenine (0.1 mg/l final concentration), and guanine, thymidine, uracil, hypoxanthine and cytosine (each 0.03 mg/l final concentration) are prepared as a 1000 X concentrated stock solution, adjusted to pH 6.5 with NH₄OH, and added to this medium. | | |
| ^{d}The following amino acids are added to this medium using a 10 X stock solution (pH 6.5 with NH₄OH) to yield the given final concentrations: glutamine (5.6 mg/l), alanine, glutamic acid (each 0.6 mg/l), cysteine (0.2 mg/l), asparagine, aspartic acid, cystine, histidine, isoleucine, leucine, lysine, methionine, phenylalanine, proline, serine, threonine, tryptophan, tyrosine and valine (each 0.1 mg/l). | | |
| ^{e}Vitamin stock solution is normally prepared 100 X concentrated. | | |

### Materials

Agarose: Preparation and purification of agarose are described by Guiseley and Renn (1975). Agarose is one of the constituents of agar. Commercially available agar normally consists of a mixture of neutral agarose and ionic agaropectin with a large number of side groups. Usually a certain number of side chains remains intact and determines the physicochemical properties of the agarose such as gel formation and melting temperature. Low-melting agarose, especially SeaPlaque® agarose, is a preferred solidifying agent.

Casein hydrolysate: Casein Hydrolysate - Enzymatic Hydrolysate from bovine milk, Type 1, Sigma Co., PO. Box 14508, St. Louis, MO 63178, USA.

Cellulase RS: Cellulase RS, Yakult Honsha Co. Ltd., 1.1.19 Higashi-Shinbashi, Minato-ku, Tokyo, 105 Japan.

GelRite®: GelRite Gellan Gum, Scott Laboratories Inc., Fiskerville, R.I. 02823, USA.

IBI Random primer kit: 'Prime Time' random primer kit, International Biotechnologies Inc., PO. Box 1565, New Haven, CT 07606, USA.

Nalgene® filter: Nalge Co., Division of Sybron Corp. Rochester, N.Y. 14602, USA.

Parafilm®: Parafilm® laboratory film - American Can Co. Greenwich, CT 06830, USA.

T4 DNA ligase: New England BioLabs

Gene-Screen Plus: DuPont

Dialog Electroporator: (DIA-LOG GmbH, D-4000 Düsseldorf 13, Federal Republic of Germany)

### SECTION 1. GENERAL TECHNIQUES

This group of examples describes general manipulations used to carry out the following detailed examples.

### Example 1: Ligation in Agarose

Following restriction endonuclease digestion of DNA and electrophoretic separation of the fragments on a low melting TAE agarose gel, the bands containing appropriate fragments are excised, placed in a test tube and heated to 65°C to melt the agarose. 2 to 5 µl are added to 15 µl water and the solution is left at 65°C for 10 minutes. This solution is cooled to 37°C and left for five minutes to temperature equilibrate. 2 µl of 10 X ligase buffer (200 mM Tris, pH 8.0, 100 mM MgCl₂, 100 mM DTT, 10 mM ATP) are added along with 1 µl T4 DNA ligase, and this solution is allowed to solidify and incubate at 15°C overnight.

### Example 2: Transformation From Agarose

The agarose containing the appropriate DNA is melted by incubating at 65°C for 10 minutes. 10 µl of this solution are added to 30 µl of TE buffer (10 mM Tris pH 7.5, 1 mM EDTA), mixed and allowed to stand at room temperature. Frozen competent cells (*E. coli* strain DH5α) are placed on wet ice to thaw. The diluted DNA solution from above is added to 200 µl of cells and allowed to stand on ice for 20 minutes. The cells plus the DNA are then heat-shocked for 90 seconds at 41°C. The cells are then left at room temperature for 10 minutes. 0.8 ml of SOC medium (Hanahan, 1983) is added and the culture is incubated at 37°C for one hour. 100 µl of the culture is plated on LB plates (Miller, 1972) containing 100 µg/ml ampicillin (L-amp) and the plates are incubated overnight at 37°C. Surviving colonies are picked and restreaked to a second antibiotic plate and the plates are incubated overnight at 37°C.

### Example 3: Labeling DNA restriction fragments

DNA is treated with the appropriate restriction enzymes and fragments are separated by electrophoresis on a LGT agarose gel. A band containing the fragment of interest is excised and the DNA purified by standard techniques. 50 ng of the DNA fragment is labelled using the IBI Random primer kit according to the manufacturers directions.

### Example 4: Southern Blotting of Genomic DNA

3 µg of tobacco DNA is digested with various restriction enzymes under the conditions suggested by the supplier. The DNA is extracted with phenol, precipitated with ethanol and then resuspended in gel-loading buffer (15 % ficoll, 0.025 % bromophenol blue, 10 mM EDTA, pH 8). Samples are loaded on to the gel and electrophoresed on a 0.5 % agarose gel at 5 Vcm⁻¹ until the bromophenol blue dye reaches the end of the gel. Following this, the DNA is transferred to Gene-Screen Plus using the alkaline transfer procedure as described by the supplier. Pre-hybridization, hybridization and washing are according to the manufacturer's recommendation. Hybridization is detected by autoradiography.

### Example 5: Molecular Adaptors

A typical molecular adaptor is the sequence 5'-GGGATCCCTGCA-3' for the conversion of a PstI site to a BamHI site. This molecule is synthesized on an Applied Biosystems Synthesizer using β-cyanoethylphosphoramidite chemistry and purified by reverse-phase HPLC. About 2 µg of this oligonucleotide is kinased according to Maniatis et al. (1982, p. 125). Following this, the oligonucleotide solution is heated to 65°C in a water bath and allowed to cool to room temperature over a period of about 30 minutes. An approximately 10-fold molar excess of this annealed adapter is added to the digested DNA along with 10 X ligase buffer, T4 DNA ligase, and an appropriate amount of water. A typical reaction is:
DNA to be adapted: 1-2 µl (∼ 1 pmol)
Adaptor: 1 µl (∼ 10 pmol)
10 X ligase buffer: 1 µl
T4 DNA ligase: 1 µl
Water: 5-6 µl
This solution is incubated at 12° to 15°C for 30 minutes, and heated to 65°C for 30 minutes to inactivate the ligase. The salt concentration and volume are adjusted for the appropriate restriction enzyme digest and the adapted DNA is digested to expose the adapted "sticky end." Unincorporated adaptors are removed either by electrophoresis on an agarose gel or by sequential isopropanol precipitations.

### Example 6: Primer Extension Mapping

A. Synthesis and 5' End-Labeling of Primers for Primer Extension. The following primer oligomers are synthesized using an Applied Biosystems Synthesizer and β-cyanoethyl-phosphoramidite chemistry:

PR-1: 5'-ATAGTCTTGTTGAGAGTT-3'

The oligonucleotides are purified by reverse-phase HPLC. 5 pmol of each oligonucleotide is kinased (Maniatis et al., 1982, p. 125) using 200 µCi of ³²P-ATP (6000 Ci/mmol, 10 µCi/ µl). After incubation at 37°C for 30 minutes, the reaction is diluted to 100 µl, extracted with phenol/chloroform and then precipitated three times with 50 µg carrier RNA. The final precipitate is resuspended in 1 X reverse-transcriptase buffer (50 mM Tris-HCl, pH 7.5, 40 mM KCl, 3 mM MgCl₂) at a concentration of 2 nM. The specific activity of the labeled oligonucleotide is determined to be about 3 X 10⁶ Cvcpm/pmol.

B. Total RNA Preparation. Total RNA is prepared essentially as described by Lagrimini et al. (1987). Tissue is ground under liquid nitrogen in a mortar and pestle. The ground tissue is added to grinding buffer (Lagrimini et al., 1987) using 2.5 ml/g tissue. An equal volume of phenol is then added and the emulsion is homogenized in a Brinkman polytron. A one-half volume of chloroform is added and the emulsion is gently mixed for 15 minutes. The phases are separated by centrifugation and the aqueous phase is removed. RNA is precipitated by the addition of sodium acetate to 0.3 M and 2.5 volumes ethanol. The precipitate is collected by centrifugation and resuspended in 2 ml sterile water. Lithium chloride is added to a final concentration of 3 M and left at 4°C overnight. The precipitate is collected by centrifugation and the pellet is washed with ice-cold 80 % ethanol. The pellet is dried and resuspended in 500 µl sterile water. The concentration of this total RNA preparation is determined spectrophotometrically.

Alternatively, RNA is extracted from callus as described above except that the callus tissue is cut into cubes approximately 3 mm in size, and added to pre-chilled mortars and pestles for grinding in liquid nitrogen prior to the polytron step.

C. Primer Extension. 50 µg of total RNA is lyophilized in a 500 µl Eppendorf tube. The RNA is resuspended in 30 µl of radiolabeled probe solution and heated to 70°C for 10 minutes. The tube is slowly cooled to 37°C and allowed to incubate overnight. Without removing the tube from the 37°C water bath, 2 µl of 10 X reverse-transcriptase buffer (500 mM Tris-HCl, pH 7.5, 400 mM KCl, 30 mM MgCl₂), 1 µl 5 mg/ml BSA, 5 µl 100 mM DTT, 5 µl 10 X dNTPs (10 mM of each dNTP in H₂O), 3 µl H₂O, 2 µl RNAase (80 units), and 2 µl reverse transcriptase (400 units) are added and the reaction is incubated at 37°C for 30 minutes. To stop the reaction, 5 µl of 3 M sodium acetate pH 5, and 150 µl absolute ethanol are added. The tube is left at -20°C for 30 minutes, the precipitate collected by centrifugation, washed with 80 % ethanol and allowed to air-dry. The precipitate is resuspended in 10 to 20 µl of loading dye (90 % formamide, 0.05 % bromophenol blue, 0.05 % xylene cyanol, 1 mM EDTA) and the extension products are separated on a 6 % sequencing gel (Maniatis et al., 1982). Extension products are visualized by autoradiography.

### SECTION 2. PROTEIN IDENTIFICATION AND CHARACTERIZATION

The PRPs relevant to these examples are isolated, purified and sequenced, for the first time in some cases, and in accordance with literature procedures in other, for the purpose of allowing the isolation of the corresponding cDNAs and ultimately for confirming the identities of the cDNAs.

### Example 7: General techniques for peptide generation, purification and automated sequencing

A. Reduction and Alkylation. Purified, lyophilized protein is dissolved in 6 M guanidine-HCl containing 1 M Tris-HCl, pH 8.6, 10 mM EDTA. DTT is added to 20 mM and 4-vinylpyridine is added to a final concentration of 50 mM. The sample is then incubated for 1.5 hours under nitrogen. The pyridylethylated material is desalted on HPLC using an Aquapore phenyl column (2.1 x 10 cm, Brownlee). The column is eluted with a linear, 5 to 80 % gradient of acetonitrile : isopropanol (1:1) in 0.1 % TFA.
B. Cyanogen Bromide Cleavage and Removal of Pyroglutamate. Cyanogen bromide cleavage is performed in situ according to Simpson and Nice (1984). Digestion of PR-1 protein with pyroglutamate aminopeptidase (Boehringer Mannheim) is carried out according to Allen, G. (1981).
C. LysC digestion. Protein is digested with endoproteinase Lys-C (Boehringer Mannheim) in 0.1 M Tris-HCl, pH 8.5, for 24 hours at room temperature using an enzyme : substrate ratio of 1 10. Resulting peptides are isolated by HPLC using an Aquapore C-8 column (1 x 22 cm, Brownlee) eluted with a linear acetonitrile: isopropanol (1:1 ratio) gradient (0 to 60%) in 0.1 %TFA.
D. Trypsin Digestion. Digestion with trypsin (Cooper) is performed in 0.1 M ammonium bicarbonate, pH 8.2, containing 0.1 M calcium chloride for five hours at 37°C using an enzyme : substrate ratio of 1 : 100. Peptides generated are separated on HPLC using the same conditions as with the Lys-C peptides or performed in 0.1 M Tris-HCl pH 8.5 for 24 hours at 37°C using an enzyme : substrate ratio of 1 : 50. Peptides are isolated by HPLC using a Vydac C-18 column (2.1 x 150 mm) with a linear 0 to 60 % acetonitrile : isopropanol (1 : 1) gradient in 0.1 % TFA.
E. Sequencing. Automated Edman degradations are performed with an Applied Biosystems 470A gas-phase sequencer. PTH amino acids are identified using an Applied Biosystems 120A PTH analyzer.

### Example 8: Purification and sequence of PR-1a and PR-1b Protein

A correlation of the DNA sequences of three cDNA clones with the PR-1a, -1b and - 1c proteins is originally made by Cornelissen, B.J.C. et al. (1986), based on a comparison of the published protein sequence data of three peptides derived from PR-1a (Lucas et al., 1985) and the primary structure of the protein deduced from the cDNA clones. However, the cDNA clone designated as PR-1a is truncated at the 5' end and can be compared to only two of the three peptides with a mismatch of one residue.
The encoded amino acid sequence deduced from cDNA as prepared and analyzed in the example below, and the PR-1a cDNA sequence from Pfitzner, U.M. et al., (1987), mismatch the published protein sequence at the tryptophan residue as reported. They also do not match at three other positions of the amino-terminal protein sequence. This anomaly places the previous identification of the PR-1 clones in question. In order to confirm the identity of our cDNA clones as either PR-1a, PR-1b or PR-1c, a large portion of the primary structure of the purified PR-1a and PR-1b protein and peptides derived from the proteins is determined by amino acid sequencing. This data is then compared to the protein sequence deduced from the nucleotide sequence of the cDNAs in order to identify which of the cDNA clones corresponds to which protein.

Plants of *N. tabacum* cv. Xanthi are grown in a glasshouse and when eight weeks old are infected by gently rubbing the leaves with a suspension of a common strain (UI) of TMV (0.5 µg/ml). Leaves are harvested seven days after infection and the ICF is washed out of the leaves and collected according to Parent and Asselin (1984). 250 ml of ICF are concentrated to 50 ml by lyophilization and loaded on an Ultragel ACA54 column equilibrated with Tris-HCl, pH 8.0, and 1 mM EDTA.

Eluates of this column are analyzed by electrophoresis on 10 % native polyacrylamide gels. Fractions containing PR-1 proteins are pooled, lyophilized, resuspended in 3 ml water and then dialyzed overnight against water. This preparation is further purified by HPLC anion exchange chromatography on a TSK-DEAE 5PN column. The column is eluted with a 0 to 0.6 M NaCl gradient in 50 mM Tris-HCl, pH 8.0, 1 mM EDTA. Fractions are analyzed by PAGE. PR-1b elutes first from the column at 0.18 M NaCl and PR-1a elutes at 0.28 M NaCl. Fractions containing each protein are pooled, dialyzed against water and lyophilized.
The purity of the PR-1a and PR-1b protein preparation is confirmed by reverse-phase HPLC using an Aquapore phenyl column (2.1 x 100 mm, Brownlee) and eluting with a linear acetonitrile : isopropanol (1:1) gradient (5 to 80 %) in 0.1 % TFA.

Protein sequence is derived from either the deblocked amino terminus of the intact protein, from peptides derived from cyanogen bromide cleavage of the proteins, from peptides derived from LysC digestion of the proteins or from peptides derived from trypsin digestion of the protein using techniques detailed above or using other methods known in the art. A summary of the data resulting from this analysis is shown below for PR-1a and PR-1b.

### PR-1a

A. (Q)NSQQDYLDAHNTARAD
B. (R)ADVGVEPLTWDDQVAAYAQNYASQLAADCNLVHSHGQYGENLAE
C. (M)TAAKAVEMWVDEKQYY
D. (K)QYYDHDSNTCAQGQVCGHYTQVVWRNSVR
E. (D)PPGNYRGESPY

### PR-1b

A. (D)FMTAAKAVEMWVD
B. (M)TAAKAVEMWVDEKQY
C. (N)GGYVVS
D. (D)PPGNVIGQSPY

### Example 9: Purification and sequence of PR-R major and PR-R minor

Plants of *N. tabaccum* cv. Xanthi are grown in a glasshouse and infected when eight weeks old by gently rubbing the leaves with a suspension of a common strain (U1) of TMV (0.5 µg/ml). Leaves are harvested seven days after infection and the ICF is washed out of the leaves and collected according to Parent and Asselin (1984). 250 ml of ICF are concentrated to 50 ml by lyophilization and loaded on an Ultragel ACA54 column equilibrated with Tris-HCl, pH 8.0, and 1 mM EDTA. Eluates are analyzed by electrophoresis on 10 % polyacrylamide gels.

Fractions containing the PR-R protein and several minor contaminating proteins are pooled, lyophilized, resuspended in 3 ml water and then dialyzed overnight against water. This preparation is further purified by HPLC reverse phase chromatography using a Brownlee Aquapore phenyl column (2.1 x 100 mm). The column is eluted with a linear gradient of 20 to 80 % acetonitrile : isopropanol (1:1) in 0.1 % TFA using a flow rate of 50 µl/minute over 80 minutes. It is found that the major proteins present are isoforms of PR-R which are given the names PR-R major, for the most abundant protein eluting at 46 minutes and PR-R minor for the less abundant protein eluting at 47.5 minutes.
The peaks containing PR-R major and PR-R minor are collected and the samples are reduced to dryness. The proteins are then resuspended in 6 M guanidine-HCl, 1 M Tris-HCl, reduced and alkylated as described above and subjected to automated sequencing as described above.

A summary of the data obtained is presented below.

PR-R Major: ATFDIVNKCTYTVWAAASPGGGRR

PR-R Minor: ATFDIVNQCTYTVWAAASPGGGRQLN

### Example 10: Purification and sequence of PR-P and PR-Q

Plants of *N. tabacum* cv. Xanthi are grown in a glasshouse and infected when eight weeks old by gently rubbing the leaves with a suspension of a common strain (UI) of TMV (0.5 µg/ml). Leaves are harvested seven days after infection and the ICF is washed out of the leaves and collected according to Parent and Asselin (1984). 250 ml of ICF are concentrated to 50 ml by lyophilization and loaded on an Ultragel ACA54 column equilibrated with Tris-HCI, pH 8.0, and 1 mM EDTA. Eluates are analyzed by electrophoresis on 10 % polyacrylamide gels. Fractions from the Ultragel ACA54 chromatography containing PR-O, PR-P, PR-Q and PR-R are pooled and concentrated by lyophilization. The proteins are further purified by PAGE followed by electroblotting onto PVDF membrane (Matsudaira, 1987). The blotted protein bands containing PR-P and PR-Q are excised and treated with 0.5 % PVP-40 in 100 mM acetic acid according to the Applied Biosystems User Bulletin No. 36, March 21, 1988. Deblocking of the protein is carried out with pyroglutamate aminopeptidase as described and the protein is sequenced from the PVDF by automated Edman degradation as decribed above.

The sequences of the amino terminus of the PR-P and PR-Q proteins are described below.

To obtain the protein sequence from peptides derived from PR-P and PR-Q, the fractions from the Ultragel column, which contain PR-proteins, are pooled, lyophilized, dissolved in water and then dialyzed against water prior to chromatography on DEAE-Sephacel. The DEAE-Sephacel column is equilibrated with 50 mM Tris-HCl (pH 8.0), 1 mM EDTA and eluted with a linear, 0 to 0.4 M gradient of sodium chloride. Fraction 6, which contains a mixture of PR-R major, PR-R minor, PR-P, PR-Q, PR-O and several minor contaminants is lyophilized and then resuspended in distilled water.

The proteins from Fraction 6 are further purified by HPLC using a reverse phase phenyl column and eluted with a linear 20 to 80 % gradient of acetonitrile : isopropanol (1 : 1) in 0.1 % TFA. PR-P and PR-Q proteins co-elute as a single peak which is collected and concentrated almost to dryness in vacuo, resuspended in 10 mM ammonium acetate, pH 7.0 and applied to a Brownlee Labs AX-300 HPLC ion-exchange column (2.1 mm X 10 cm) equilibrated in 10 mM ammonium acetate (pH 7.0). The proteins are eluted from this column using a linear gradient of 10 to 250 mM ammonium acetate (pH 7.0). PR-P and PR-Q elute as single distinct peaks at ca. 75 mM and ca. 95 mM ammonium acetate, respectively.

Peptides are generated by either cyanogen bromide cleavage, trypsin digestion or LysC digestion and purified as described in Example 7. Amino acid sequence is determined as described in Example 7, and as summarized below:

### PR-P

amino terminus | (Q)GIGSIVTNDLFNEML
CNBR | RNDGR?PANGF
Tryp 46.4 | (R/K)GPIQLTNQNNYEK
Tryp 64.7 | (R/K)QDLVNNPDLVATDATI
Tryp 60.2 | (R/K)Y?GMLNVAPGDNLD?YNQ(R/K)

### PR-Q

amino terminus | (Q)GIGSIVT?DLFNEML
CNBR | (M)LKNRNDGR?PANGFYTYDAFIA
Tryp 74.2 | (R/K)?PANGFYTYDAF
Tryp 44.7 | (R/K)GPIQLTN(R/K)
Tryp 38.6 | (R/K)WTPSAADQAAN(R/K)
Tryp 37.5 | (R/K)IGYY(R/K)
Tryp 50.3 | (R/K)YCGMLNVAPGENLDCYN

### Example 11: Protein purification and sequence of PR-O'

Plants of *N. tabacum* cv. Xanthi are grown in a glasshouse and infected when eight weeks old by gently rubbing the leaves with a suspension of a common strain (U1) of TMV (0.5 µg/ml). Leaves are harvested seven days after infection and the ICF is washed out of the leaves and collected according to Parent and Asselin (1984). 250 ml of ICF are concentrated to 50 ml by lyophilization and loaded on an Ultragel ACA54 column equilibrated with Tris-HCl, pH 8.0, and 1 mM EDTA. Eluates are analyzed by electrophoresis on 10 % polyacrylamide gels. Fractions from the Ultragel ACA54 chromatography containing PR-proteins as determined by gel electrophoresis are pooled, lyophilized, dissolved in water and dialzyzed against water prior to chromatography on DEAE-Sephacel. The DEAE-Sephacel column is equilibrated with 50 mM Tris-HCl (pH 8.0), 1 mM EDTA and eluted with a linear 0 to 0.4 M gradient of sodium chloride. Fraction 6 which contains a mixture of PR-R major, PR-R minor, PR-P, PR-Q and several minor contaminants is lyophilized and then resuspended in distilled water. The individual proteins are further purified by HPLC reverse phase chromotography using a Vydac phenyl column (4.6 x 250 mm). Proteins are eluted using a linear 20 to 80 % gradient of acetonitile : isopropanol (1:1) in 0.1 % TFA. The results of this purification step reveal that the mixture of proteins contains at least nine individual proteins. One of these proteins, PR-O', eluting at 46 minutes is collected and concentrated by lyophilization. The protein is resuspended in 6 M guanidine-HCl, 1 M Tris-HCl and reduced and alkylated as described above. Peptides are generated by trypsin digestion and separated as decribed above. A summary of the amino acid sequences of these peptides is presented below.

A. VSTAIETGLTTDTSPP
B. TYNNNLI
C. YVPVLLNAMR
D. QFIEPIINFLVTN
E. NLFDAILDATYSALEK

### Example 11B: Purification and protein sequence of PR-2, PR-N and PR-O

Plants of *N. tabacum* c. Xanthi.nc are grown in a glasshouse and infected when eight weeks old by gently rubbing the leaves with a suspension of a common strain (U1) of TMV (0.5 µg/ml). Leaves are harvested seven days after infection and the intercellular fluid (ICF) is washed out of the leaves and collected according to Parent and Asselin (1984). 250 ml of ICF are concentrated to 50 ml by lyophilization and loaded on an Ultragel ACA54 column equilibrated with Tris-HCI, pH 8.0 and 1 mM EDTA. Eluates are analyzed by electrophoresis on 10 % polyacrylamide gels. Fractions containing PR-proteins as determined by gel electrophoresis are pooled, lyophilized, dissolved in water and dialyzed against water prior to chromatography on DEAE-Sephacel. The DEAE-Sephacel column is equilibrated with 50 mM Tris-HCl (pH 8.0), 1 mM EDTA and eluted with a linear 0 to 0.4 M gradient of sodium chloride. Fraction 6, which contains a mixture of PR-R major, PR-minor, PR-P, PR-Q, PR-O and several minor contaminants, is lyophilized. Fraction 3, which contains a mixture of PR-2, PR-N and several minor contaminants, is also collected separately and concentrated by lyophilization.

PR-O is further purified from other Fraction 6 proteins by first resuspending Fraction 6 in 2 ml water and then separating the proteins by HPLC reverse phase chromtography using a Vydac phenyl column (4.6 X 250 mm). Proteins are eluted using a linear 20 to 80 % gradient of acetonitrile : isopropanol (1:1) in 0.1 % TFA. The results of this purification step reveal that the mixture contains at least nine proteins. One of these proteins, eluting in one run at 51 minutes is PR-O as determined by gel electrophoresis. The peak containing PR-O is collected and concentrated by lyophilization. The protein is resuspended in 6 M guanidine-HCl, 1 M Tris-HCl and reduced and alkylated as described above. Peptides are generated by trypsin and LysC digestion and are purified as described above. The protein sequence is determined as described in Example 7. A summary of the sequencing data is provided below.

The proteins PR-2 and PR-N are purified from Fraction 3 using a Brownlee Aquapore AX-300 (2.1 x 100 mm) anion exchange column. The proteins are eluted from the column using a linear gradient of 10 mM to 350 mM ammonium acetate pH 7.0. PR-N elutes at 37.5 minutes and PR-2 elutes at 50.0 minutes as single, uniform peaks. The identity of the proteins is confirmed by gel electrophoresis. The proteins are collected, concentrated by lyophilization and then reduced and alkylated as described above. Peptides are generated by trypsin digestion and purified as described in Example 7. The protein sequencing data is summarized below.

### PR-2

Peptide | Peptide
Designation | Sequence
49.2 | DSIFR
54/69.5 | YQLNFN
54/72 | VSTATYSGILANTNP
58.9 | HFGLFSPDQR
65.9 | IYNPDTNVFN 57/94.5
ANGRVQDNIIN
78.8 | APGNAIETYLFAMFDENN.EGD
82.5 | YIAVGNEVSPGNNGQYAPF
91.8 | GSNIEII

### PR-N

Peptide | Peptide

Designation | Sequence
44.8 | YQLNFN
46.1 | VSTATYSGILAN.YP
50.4 | DNLPPDQQVINLYNA
53.3 | IYNPDTNVFNAL
65.3 | AGGQNVEIIVSESG.PSE
65.3B | DIETYLFAMFD.NN.EGD
65.6A | AGGQNLEII
65.6B | DIE.YVFAM
67 | IYNPDTNVFNAL
79 | GSNIEIILDVPLQDLQSLTDP

### PR-O

Peptide | Peptide
Designation | Sequence
39.1 | NLIDHV
43.2 | YQLNF
46.7 | HFGLFSPDQR
51.5 | IANNLPSDQDVINLYNANGI
54.5 | PENTNVFNAL
57.6 | NNLPLLANVYP
64.9 | GSNIEIILDVPNQDLESLTDPS
70.9 | YIAVGNEVSPTN
83.3 | AGGPNVEIIVSESG.P

### Example 12: Purification and protein sequence of cucumber chitinase/lysozyme

A pathogen-inducible chitinase protein is isolated from infected cucumber leaves as described (Métraux et al., 1988). Peptides are generated from this homogeneous protein preparation essentially as described in the art and as described above. The amino acid sequence of these peptides is summarized below.

Amino Terminus AGIAIYWGQNGNEGSLASTCATGNYEFVNIAFL

LysC Peptides | (K)NFGQVILSAAPQCPIPDAHLDAAIK
(K)TGLFDSVWVQFYNNPPCMFADNADNLLS

(K)LYMGLPAAREAAPSGGFIPAD
(K)ASSNYGGVMLWSK

CNBR Peptides (M)FADNADNLLS
(M)GLPAAREAAPSGGFIPADVLISQVLPTI

Tryptic Peptides | VLLSIGGGA
 | TGLFD?V
 | LYMGLPAA
 | ASSNYGGV
 | AFDNGY

### Example 12B: Purification and protein sequence of the cucumber peroxidase

The pathogen-induced, acidic, cucumber peroxidase protein is purified as described by Smith, J.A., PH. D. Thesis, Department of Botany and Plant Pathology, Michigan State University, Lansing, Michigan (1988), from the uninfected leaves of cucumber plants that have been infected seven days previously with a spore suspension of *Colletotrichum lagenarium.*
The purified protein is reduced and alkylated and amino acid sequence from the amino terminus and from peptides derived from either LysC or trypsin digestion is determined as described in Example 7. The results of the amino acid sequencing are summarized below.

Peptide Designation | Peptide Sequence

Amino terminal | (Q)LSPTFYNTTWPNV
LysC 84.0/60.7 | TAVENVCPGVVSCADILALGSRDAVT
LysC 71.6 | DAVTLASGQGWTVQLGR
LysC 54.1/55.1 | IIRLHFHDCFVDGCDGSVLLEDQDGIT.E
LysC 28.6 | TGTTGEIRTNCRRLN
Tryp 66.4 | LNNNPNADDSPIDSTAYASQL

### SECTION 3. ISOLATION OF NOVEL cDNA CLONES

This section describes the isolation of novel cDNA clones. It is divided into 3 sections: Section A covers the construction of libraries used in the isolation of clones. Section B covers the identification and purification of clones thus isolated. Section C covers the development of a novel cDNA cloning technology.

### Section 3A: Construction of cDNA libraries

### Example 13: Preparation of cDNA library from TMV-infected tobacco leaves

*N. tabacum* cv. Xanthi-nc leaves are infected with TMV and harvested five days post-infection. Total RNA is prepared as described above and poly A+ RNA is isolated using standard techniques. A cDNA library is constructed using this poly A+ RNA in the Lambda ongC cloning vector (Stratagene) essentially as described (Gubler and Hoffman, 1983).

### Example 14A: Preparation of a cDNA library from uninfected leaves of TMV inoculated tobacco using a novel cDNA cloning vector

pCGN1703, a plasmid cDNA cloning vector of the vector-primer type described by Okayama and Berg (1982) is constructed as an improved vector to simplify the cloning process and allow easy shuttling of libraries into a phage vector, as well as to provide additional functions that are outside the present use.

A. Construction of the pCGN1703 cloning vector. Bluescribe M13- (Stratagene, Inc.) is used as a starting plasmid. The BamHI site is deleted by BamHI digestion and mungbean nuclease treatment, followed by ligation with T4 DNA ligase to yield pCGN1700. This plasmid is digested with EcoRI and SacI and then ligated with a double stranded synthetic polylinker created by annealing two oligonucleotides of the sequence

[oligo #47] 5'-AATTTCCCGGGCCCTCTAGACTGCAGTGGATCCGAGCT-3'

[oligo #46] 5'-CCGATCCACTGCAGTCTAGAGGGCCCGGGA-3'.

The resulting plasmid thus has additional restriction sites for SmaI, ApaI, XbaI, PstI, and BamHI, and is designated pCGN1702. Note that the EcoRI site is not reconstructed.

pCGN1702 is digested to completion with HindIII and made blunt ended with T4 polymerase. The resultant DNA is subjected to partial digestion with PvuII and then ligated with T4 DNA ligase. A transformant is selected that has deleted the 214 bp HindIII-PvuII fragment which includes the *lac* operator-promoter region; this plasmid is designated pCGN1703.

Use of vector-primer plasmids such as pCGN1703 was described previously (Alexander, 1987). As described in the ADDENDUM section of that work, the present vector is a monomer vector. The T-tracts used to prime cDNA synthesis are present on both ends of the vector during the reverse transcriptase and terminal transferase (G-tailing) reactions, and the linker DNA used to circularize the final products of cDNA cloning with pCGN1703 has the generalized structure as follows: T7 promoter, a multiple cloning site (SmaI, ApaI, XbaI, PstI, BamHI, NotI, EcoRI, SacI), C:G homopolymer tract (10 to 15 residues), cDNA insert (5'-3' of mRNA-sense strand), A:T homopolymer tract (40 to 100 residues), and another multiple cloning site (KpnI, SmaI, XbaI, Sail, PstI and SphI), all contained within the plasmid backbone derived from Bluescribe M13- as described above.

B. Construction of the cDNA library using pCGN1703. Xanthi.nc tobacco plants are grown in a phytotron until they are approximately 10 to 12 inches tall. Two leaves near the bottom of each plant are inoculated with either a mock buffer-only sample (10 mM sodium phosphate, pH 7.0) or a sample of TMV (10 µg/ml in the same buffer). Eleven days later 3 to 4 upper leaves, which have not been inoculated, are harvested and frozen in liquid nitrogen. Poly-A+ mRNA is isolated by methods previously described (Hall et al., 1978, p. 3196-3200; Manitatis et al., 1982, p. 297-209). A cDNA library is constructed, using the poly-A+ RNA isolated from TMV-induced leaves, in the cDNA vector pCGN1703 by methods previously described (Alexander, 1987). Plasmid DNA of the amplified cDNA library (Alexander, 1987) is digested to completion with EcoRI and sub-cloned into the EcoRI site of lgt-10 (Stratagene, Inc.). Note that the plasmid vector remains attached to the cDNAs and is also cloned into the phage vector. Therefore, using this process, two cDNA libraries are constructed, one in which the library is contained in a plasmid vector and the other in which the cDNA library is contained in a phage vector.

### Example 14B: Preparation of a cDNA library from TNV infected cucumber leaves

Cucumber leaves are infected with Tobacco Necrosis Virus (TNV) and RNA is isolated 5 days after infection as described above. Poly A+ RNA is isolated by standard techniques and a cDNA library is constructed in the lambda Zap cloning vector (Stratagene), essentially as described (Gubler and Hoffman, 1983).

Section 3B: Identification, isolation and characterization of cDNA clones

### Example 15: Isolation of cDNAs encoding PR-1a, PR-1b and PR-1c

About 300,000 plaques from the cDNA library prepared above are screened with an oligonucleotide of the sequence:

5' CAAAACTCTCAACAAGACTATTTGGATGCCC 3'.

25 positive plaques are purified by standard techniques and DNA is prepared from the phage. A fragment of the recombinant phage which contains the cDNA is subcloned into the bluescript plasmid. A partial cDNA sequence of each clone is determined by DNA sequencing. It is found that the 26 clones can be typed into three classes of cDNAs. Class 1 is represented by the clone pBSPR1-207, class 2 is represented by the clone pBSPR1-1023 and class 3 is represented by the clone pBSPR1-312.

In order to determine the identity of the three clones relative to the known PR-1 proteins, the amino acid sequence data for the PR-1a and PR-1b proteins determined above is compared to the amino acid sequences deduced from the three representative cDNA clones. The comparison is summarized below.

The experimentally determined amino acid sequence from above is shown on the top line with the first (inferred) amino acid shown in parentheses. The sequence deduced from clones pBSPR1-207 (= a), pBSPR1-1023 (= b), and pBSPR1-312 (= c) is shown below the peptide data with agreements indicated with (-). Residues that do not agree are designated by their amino acid symbol.

### PR-1a

A.  (Q)NSQQDYLDAHNTARAD
a - ----------------
b - ----------------
c - ----------------

B.   (R) ADVGVEPLTWDDQVAAYAQNYASQLAADCNLVHSHGQYGENLAE
    a - --------------------------------------------
    b - -----------NG--------V---------------------Q
    c - -------------------------------------------W

C.   (M) TAAKAVEMWVDEKQYY
    a - ----------------
    b - ----------------
    c L ----------N-----

D.   (K) QYYDHDSNTCAQGQVCGHYTQVVWRNSVR
    a - -----------------------------
    b - -----------------------------
    c - ---A-------------------------

E.   (D) PPGNYRGESPY
    a - -----------
    b - ----VI-Q---
    c - ----VI-K---

### PR-1b

A.  (D)FMTAAKAVEMWVD
    a - -------------
    b - -------------
    c - -L----------N

B.   (M)TAAKAVEMWVDEKQY
    a - ---------------
    b - ---------------
    c L ----------N----

C.   (N)GGYVVS
    a - ------
    b - ------
    c - ---I--

D. (D)PPGNVIGQSPY
a - ----YR-E---
b - -----------
c - -------K---

When the protein sequence for PR-1a is compared to the deduced amino acid sequence derived from pBSPR1-20 there is agreement at every residue. However, when the deduced amino acid sequence derived from the pBSPR1-1023 or pBSPR1-312 peptides is compared to the protein sequence for PR-1a there are seven and six mismatches, respectively. Thus, the evidence clearly demonstrates that the clone pBSPR1-207 encodes the PR-1a protein.

When the amino acid sequence for the PR-1b protein is compared to the deduced amino acid sequence derived from the pBSPR1-1023 protein there is agreement at every residue. However, in comparisons to the sequence derived from pBSPR1-207 or pBSPR1-312 there are three or six mismatches, respectively. Thus, the data clearly demonstrates that the clone pBSPR1-1023 encodes the PR-1b protein. Further, by default, the clone pBSPR1-312 is determined to encode the PR-1c protein. The sequences of the cDNAs encoding PR-1a, PR-1b and PR-1c are as follows:

### Sequence 1: PR-1a cDNA cloned into the plasmid pBSPR1-207

  1 ATTCAAGATA CAACATTTCT CCTATAGTCA TGGGATTTGT TCTCTTTTCA
 51 CAATTGCCTT CATTTCTTCT TGTCTCTACA CTTCTCTTAT TCCTAGTAAT
101 ATCCCACTCT TGCCGTGCCC AAAATTCTCA ACAAGACTAT TTGGATGCCC
151 ATAACACAGC TCGTGCAGAT GTAGGTGTAG AACCTTTGAC CTGGGACGAC
201 CAGGTAGCAG CCTATGCGCA AAATTATGCT TCCCAATTGG CTGCAGATTG
251 TAACCTCGTA CATTCTCATG GTCAATACGG CGAAAACCTA GCTGAGGGAA
301 GTGGCGATTT CATGACGGCT GCTAAGGCTG TTGAGATGTG GGTCGATGAG
351 AAACAGTATT ATGACCATGA CTCAAATACT TGTGCACAAG GACAGGTGTG
401 TGGACACTAT ACTCAGGTGG TTTGGCGTAA CTCGGTTCGT GTTGGATGTG
451 CTAGGGTTCA GTGTAACAAT GGAGGATATG TTGTCTCTTG CAACTATGAT
501 CCTCCAGGTA ATTATAGAGG CGAAAGTCCA TACTAATTGA AACGACCTAC
551 GTCCATTTCA CGTTAATATG TATGGATTGT TCTGCTTGAT ATCAAGAACT
601 TAAATAATTG CTCTAAAAAG CAACTTAAAG TCAAGTATAT AGTAATAGTA

651 CTATATTTGT AATCCTCTGA AGTGGATCTA TAAAAAGACC AAGTGGTCAT
701 AATTAAGGGG AAAAATATGA GTTGATGATC AGCTTGATGT ATGATCTGAT
751 ATTATTATGA ACACTTTTGT ACTCATACGA ATCATGTGTT GATGGTCTAG
801 CTACTTGCG

### Sequence 2: PR-1b cDNA cloned into the plasmid pBSPR1-1023

1 GTTCAAAATA AAACATTTCT CCTATAGTCA TGGGATTTTT TCTCTTTTCA
51 CAAATGCCCT CATTTTTTCT TGTCTCTACA CTTCTCTTAT TCCTAATAAT
101 ATCTCACTCT TCTCATGCCC AAAACTCTCA ACAAGACTAT TTGGATGCCC
151 ATAACACAGC TCGTGCAGAT GTAGGCGTGG AACCATTAAC TTGGGACAAC
201 GGGGTAGCAG CCTATGCACA AAATTATGTT TCTCAATTGG CTGCAGACTG
251 CAACCTCGTA CATTCTCATG GCCAATACGG CGAAAACCTA GCTCAGGGAA
301 GTGGCGATTT TATGACGGCT GCTAAGGCCG TCGAGATGTG GGTCGATGAG
351 AAACAGTACT ATGACCATGA CTCAAATACT TGTGCACAAG GACAGGTGTG
401 TGGACACTAT ACTCAGGTGG TTTGGCGTAA CTCGGTTCGT GTTGGATGTG
451 CTAGGGTTAA GTGCAACAAT GGAGGATATG TTGTCTCTTG CAACTATGAT
501 CCTCCAGGTA ATGTCATAGG CCAAAGTCCA TACTAATTGA AATGAATGTC
551 CATTTCACGT TATATATGTA TGGACTTCTG CTTGATATAT ATAAACAACT
601 TAAATAATTG CACTAAAAAG CAACTTATAG TTAAAAGTAT ATAATATTTG
651 TAATCCTCTG AAGAACTGGA TCTGTAAAAA GTCCAAGTGG TCTTAATTAA
701 GGGGGGGAGG ATATATGAAT TCAGCTTGAT GTATGATCTG ATATTATTAT
751 GAACTCTTTA GTACTCTTAC G

### Sequence 3: PR-1c cDNA cloned into the plasmid pBSPR1-312

1 GTTCAAAATA AAACATTTCT CCTATAGTCA TGGAATTTGT TCTCTTTTCA
51 CAAATGTCTT CATTTTTTCT TGTCTCTACG CTTCTCTTAT TCCTAATAAT
101 ATCCCACTCT TGTCATGCTC AAAACTCTCA ACAAGACTAT TTGGATGCCC
151 ATAACACAGC TCGTGCAGAT GTAGGTGTAG AACCTTTGAC CTGGGACGAC
201 CAGGTAGCAG CCTATGCACA AAATTATGCT TCCCAATTGG CTGCAGATTG
251 TAACCTCGTA CATTCTCATG GTCAATACGG CGAAAACCTA GCTTGGGGAA
301 GTGGCGATTT CTTGACGGCC GCTAAGGCCG TCGAGATGTG GGTCAATGAG
351 AAACAGTATT ATGCCCACGA CTCAAACACT TGTGCCCAAG GACAGGTGTG
401 TGGACACTAT ACTCAGGTGG TTTGGCGTAA CTCGGTTCGT GTTGGATGTG
451 CTAGGGTTCA GTGTAACAAT GGAGGATATA TTGTCTCTTG CAACTATGAT

501 CCTCCAGGTA ATGTTATAGG CAAAAGCCCA TACTAATTGA AAACATATGT
551 CCATTTCACG TTATATATGT GTGGACTTCT GCTTGATATA TATCAAGAAC
601 TTAAATAATT GCGCTAAAAA GCAACTTATA GTTAAGTATA TAGTACTATA
651 TTTGTAATTC TCTGAAGTGG ATATATAATA AGACCTAGTG CTCTTG

### Example 16: Isolation of cDNA clones encoding PR-R major and PR-R minor

About 300,000 plaques from the library constructed above are screened with an oligonucleotide probe of the sequence

5'-GAACTTCCTAAAAGCTTCCCCTTTTATGCC-3'.

Fifteen positive plaques are purified by standard techniques and DNA is prepared from the phage. A fragment of the recombinant phage which contains the cDNA is subcloned into the bluescript plasmid. Partial DNA sequence of the cDNA insert reveals that the clones can be typed into two classes. The sequence of one of these clones, pBSPRR-401, which encodes the major form of PR-R (Pierpoint et al., 1987; and above) is as follows:

### Sequence 4: PR-R major cDNA cloned into the plasmid pBSPRR-401

1 AAAAAGAAAA AAAAAATGAA CTTCCTCAAA AGCTTCCCCT TTTTTGCCTT
51 CCTTTATTTT GGCCAATACT TTGTAGCTGT TACTCATGCT GCCACTTTTG
101 ACATTGTCAA CAAATGCACC TACACAGTCT GGGCCGCGGC CTCTCCAGGT
151 GGAGGCAGGC GGCTCGACTC AGGCCAATCT TGGAGCATTA ATGTGAACCC
201 AGGAACAGTC CAGGCTCGCA TTTGGGGTCG AACCAATTGC AACTTCGATG
251 GCAGTGGCCG AGGTAATTGT GAGACTGGAG ACTGTAACGG GATGTTAGAG
301 TGTCAAGGCT ATGGAAAAGC ACCTAACACT TTAGCTGAAT TTGCACTTAA
351 TCAACCCAAT CAGGACTTTG TCGACATCTC TCTTGTTGAT GGATTTAACA
401 TCCCCATGGA ATTCAGCCCG ACCAATGGAG GATGTCGTAA TCTCAGATGC
451 ACAGCACCTA TTAACGAACA ATGCCCAGCA CAGTTGAAAA CACAAGGTGG
501 ATGTAACAAC CCATGTACTG TGATAAAAAC CAATGAATAT TGTTGTACAA
551 ATGGGCCTGG ATCATGTGGG CCTACTGATT TGTCGAGATT TTTTAAGGAA
601 AGATGCCCTG ATGCTTATAG TTATCCACAG GATGATCCAA CCAGTTTGTT
651 TACGTGTCCT TCTGGTACTA ATTACAGGGT TGTCTTCTGC CCTTGAAATT
701 GAAGCCTGCA AAATTATGAC TATGTAATTT GTAGTTTCAA ATATATAAGC
751 TACACAAGTA GTACTAAGCA CTATTAAATA AAAAAGAGAG TGACAAAGAG
801 GAGAGGCTGT GGGTCAGATT CTCTTGTTCG CTGTTGTCGT TGTTGTAGCA

851 TTCTGGTTTT AAGAAATAAA GAAGATATAT ATCTGCTAAA TTATTAAATG

The identity of this cDNA as encoding the major form of PR-R major is confirmed by comparing the encoded protein sequence to the amino terminal sequence determined experimentally above and by comparing to the sequence presented for the PR-R major isoform by Pierpoint et al. (1987). This encoded protein has a very strong homology to a known trypsin/α-amylase inhibitor isolated from maize (Richardson et al., 1987). The cloned PR-R may be an inhibitor of either proteases or α-amylases and as such will confer insecticidal, viricidal or fungicidal activity to plants when expressed transgenically.

### Example 17: Isolation of cDNA clones encoding PR-P and PR-Q

About 300,000 plaques of the cDNA library prepared above are screened using a labeled cDNA probe encoding the tobacco basic chitinase gene (Shinshi et al., 1987) and washing filters at 50°C in 0.125 mM NaCl, 1 % SDS, 40 mM sodium phosphate (pH 7.2), 1 mM EDTA. 24 positive plaques are purified and the DNA sequence of one clone named pBScht15 is as follows:

### Sequence 5: PR-Q cDNA cloned into the plasmid pBScht15

1 AAAAAAAAAA AAAAACATAA GAAAGTACAG AGGAAAATGG AGTTTTCTGG
51 ATCACCAATG GCATTGTTTT GTTGTGTGTT TTTCCTGTTC TTAACAGGGA
101 GCTTGGCACA AGGCATTGGT TCTATTGTAA CGAGTGACTT GTTCAACGAG
151 ATGCTGAAGA ATAGGAACGA CGGTAGATGT CCTGCCAATG GCTTCTACAC
201 TTATGATGCA TTCATAGCTG CTGCCAATTC CTTTCCTGGT TTTGGAACTA
251 CTGGTGATGA TACTGCCCGT AGGAAAGAAA TTGCTGCCTT TTTCGGTCAA
301 ACTTCTCACG AAACTACTGG TGGATCCCTG AGTGCAGAAC CATTTACAGG
351 AGGGTATTGC TTTGTTAGGC AAAATGACCA GAGTGACAGA TATTATGGTA
401 GAGGACCCAT CCAATTGACA AACCGAAATA ACTATGAGAA AGCTGGAACT
451 GCAATTGGAC AAGAGCTAGT TAACAACCCT GATTTAGTGG CCACAGATGC
501 TACTATATCA TTCAAAACAG CTATATGGTT TTGGATGACA CCACAGGACA
551 ACAAGCCATC TTCCCACGAC GTTATCATCG GTCGTTGGAC TCCGTCTGCC
601 GCGGATCAGG CGGCGAATCG AGTACCAGGT TACGGTGTAA TTACCAACAT
651 CATTAACGGT GGAATTGAAT GTGGCATAGG ACGGAATGAC GCAGTGGAAG
701 ATCGAATTGG ATACTACAGG AGGTATTGTG GTATGTTAAA TGTTGCTCCG
751 GGGGAAAACT TGGACTGTTA CAACCAAAGG AACTTCGGCC AGGGCTAGGC

801 TTCGTTACAT AGAATGCAGA TCATGTTATG TATACAAGTT ATATTTGTAT
851 TAATTAATGA ATAAGGGGAT TGTGTATCCA TTAAGAATTA GGTGAAATAT
901 TTCTGTTATT TGTCTTCTTG GGAAGAACCA ATAGCTCCTA TATATGAGGC
951 GCTTTTAAGT GATGAGGCTA CTGCATTGAT GAAAACGAAA TTTCTATCCA
1001 GAAATAAAAG TTCCTTGTCT

The protein encoded in the clone of this sequence is determined to be the PRP Q based on: (1) limited structural homology to the the basic tobacco chitinase and (2) identity to the amino-terminal protein sequence of PR-Q and identity to the sequence of a number of internal peptides derived from PR-Q as determined by protein sequencing (see above). The isolated clone appears to be a truncated cDNA. In order to isolate the 5' end of the cDNA, the end of the mRNA is first determined.

An oligonucleotide primer of the sequence: 5' CAGCAGCTATGAATGCAT 3' referred to as oligo A, is synthesized by β-cyanoethylphosphoramidite chemistry and purified by standard techniques. This primer is used in a primer extension experiment as above using RNA isolated from TMV infected leaves. Two extension products are visualized by autoradiography corresponding to mRNA end points that are 43 bp and 53 bp longer than the pBScht15 cDNA.

In order to isolate the 5' end of the PR-Q cDNA, a novel method of cloning is developed based on PCR technology. Essentially, two primers are used to amplify and then clone the 5' end of the cDNA clone from the cDNA library. One primer (oligo A) which is complementary to the sense-strand of the cDNA and located about 160 bases into the pBScht15 cDNA and a second primer (either oligo B or Oligo C, below) which will prime into the cDNA from either side of the λ cloning vector are used in this procedure.

Oligo A is complementary to the PR-Q mRNA and contains a sequence recognized by the endonuclease NsiI. Two other oligonucleotides with the sequence 
5' GGCAGGGATATTCTGGC 3' and
5' TGCAAAGCTTGCATGCC 3'
 are synthesized, purified and named oligo B and oligo C. Oligo B has the same sequence as part of the λ OngC cloning vector and Oligo C is complementary to the polylinker of the λ OngC cloning vector. In order to clone the 5' end of the PR-Q cDNA, two PCR*s* are carried out, one using oligos A and B and the other using oligos A and C. An aliquot of the cDNA library is used as a template for the reaction. The two reactions are necessary to amplify clones that have been ligated into the λ OngC vector in either direction. As a control, reactions are also performed on aliquots of the purified phage lysate using the chitinase 15 isolate. After amplification, the DNA is purified and digested with NsiI and EcoRI and run on a 1.5 % LGT agarose gel. Gel slices containing DNA fragments longer than the control are excised and ligated into pBluescript which is digested with both EcoRI and PstI as described above. After transformation, positive colonies are isolated and the DNA insert analyzed by DNA sequencing. Several inserts contain the 5' end of the PR-Q cDNA and others contain the 5' end of PR-P (as determined by comparing the amino acid sequence deduced from the clones to the protein sequence determined above).

The 3' end of PR-P is then isolated from the cDNA library by screening about 100,000 clones of the cDNA library with a probe from one of the 5' isolates of PR-P, pBScht5'-5. Positive phage are isolated and purified and the insert is subcloned into pBluescript. Several inserts are sequenced and one, pBSCht28, is determined to encode PR-P. The DNA sequence of PR-P is as follows:

### Sequence 6: PR-P cDNA cloned into the plasmid pBScht28

1 GAAAATGGAG TTTTCTGGAT CACCACTGAC ATTGTTTTGT TGTGTGTTTT
51 TCCTGTTCCT AACAGGGAGC TTGGCACAAG GCATTGGCTC AATTGTAACG
101 AATGACTTGT TCAACGAGAT GCTGAAGAAT AGGAACGACG GTAGATGTCC
151 TGCCAATGGC TTCTATACTT ATGATGCATT CATAGCTGCT GCCAATTCCT
201 TTCCTGGTTT TGGAACTAGT GGTGATGATA CTGCCCGTAG GAAAGAAATT
251 GCTGCCTTTT TCGGTCAAAC TTCTCATGAA ACTACAGGTG GTTCCCTGAG
301 TGCAGAACCT TTTACAGGAG GATATTGCTT TGTTAGGCAA AATGACCAGA
351 GTGACAGATA TTATGGTAGA GGACCCATCC AATTGACAAA CCAAAATAAC
401 TATGAGAAAG CTGGAAATGC AATTAGACAA GACCTAGTTA ACAACCCAGA
451 TTTAGTAGCT ACAGATGCTA CTATATCATT CAAAACAGCT ATATGGTTCT
501 GGATGACACC ACAGGATAAT AAGCCATCAA GCCACGACGT TATCATCGGT
551 AGTTGGACTC CGTCCGCCGC TGATCAGTCG GCGAATCGAG CACCTGGTTG
601 CGGTGTAATT ACCAACATTA TTAACGGTGG AATTGAATGT GGCGTAGGTC
651 CGAATGCCGC AGTGGAAGAT CGAATTGGAT ACTACAGGAG GTATTGTGGT
701 ATGTTGAATG TTGCTCCTGG GGACAACTTG GACTGTTACA ACCAAAGGAA
751 CTTCGCCCAA GGCTAGGATT CGTTAGATCA TGTTATGTGT ACACAAGTTA
801 TATTTGTATG TAATGAATAA GGGGATTGTG TACCCATTTA GAATAAGGGG

851 AAATATTTCT GTTATTTGTC TTCTTCGAAA GAATAACCAG TAGTTCCTAT
901 ATATCTGGTG CTTCGAGTGA AAACGAATAT TCTATCCGGA AATAAATACT
951 GTATGTTTCT TGTCTTAT

### Example 18: Isolation of cDNA clones encoding PR-O'

About 300,000 plaques of the cDNA library described above are screened using a labeled cDNA probe encoding the basic form of β-1,3-glucanase (Shinshi et al., 1988) and washing filters at 50°C in 0.125 M NaCl, 1 % SDS, 40 mM sodium phosphate (pH 7.2), 1 mM EDTA. 15 positive plaques are isolated and the insert is subcloned into the bluescript plasmid. Partial DNA sequencing reveals that pBSGL5 and pBSGL6e encode identical cDNAs which have about 55 % homology to the known DNA sequence of the basic form of β-1,3 glucanase. The sequence of the cDNA in clone 5 and 6 is determined and shown as sequence 7 for the cDNA in the plasmid pBSGL6e.

### Sequence 7: PR-O' cDNA cloned into the plasmid pBSGL6e

1- 40 CAGGTGCTCA AGCAGGAGTT TGTTATGGAA GGCAAGGGAA
41- 80 TGGATTACCA TCTCCAGCAG ATGTTGTGTC GCTATGCAAC
81- 120 CGAAACAACA TTCGTAGGAT GAGAATATAT GATCCTGACC
121- 160 AGCCAACTCT CGAAGCGCTT AGAGGCTCCA ACATTGAGCT
161- 200 CATGCTAGGT GTCCCGAATC CGGACCTTGA GAATGTTGCT
201- 240 GCTAGCCAAG CCAATGCAGA TACTTGGGTC CAAAACAATG
241- 280 TTAGGAACTA TGGTAATGTC AAGTTCAGGT ATATAGCAGT
281- 320 TGGAAATGAA GTTAGTCCCT TAAATGAAAA CTCTAAGTAT
321- 360 GTACCTGTCC TTCTCAACGC CATGCGAAAC ATTCAAACTG
361- 400 CCATATCTGG TGCTGGTCTT GGAAACCAGA TCAAAGTCTC
401- 440 CACAGCTATT GAAACTGGAC TTACTACAGA CACTTCTCCT
441- 480 CCATCAAATG GGAGATTCAA AGATGATGTT CGACAGTTTA
481- 520 TAGAGCCTAT CATCAACTTC CTAGTGACCA ATCGCGCCCC
521- 560 TTTGCTTGTC AACCTTTATC CTTACTTTGC AATAGCAAAC
561- 600 AATGCAGATA TTAAGCTTGA GTATGCACTT TTTACATCCT
601- 640 CTGAAGTTGT TGTAAATGAT AACGGAAGAG GATACCGAAA
641- 680 CCTTTTTGAT GCCATCTTAG ATGCCACATA CTCGGCCCTT
681- 720 GAAAAGGCTA GTGGCTCGTC TTTGGAGATT GTTGTATCAG
721- 760 AGAGTGGTTG GCCTTCAGCT GGAGCAGGAC AATTAACATC

761- 800 CATTGACAAT GCCAGGACTT ATAACAACAA CTTGATTAGT
801- 840 CACGTGAAGG GAGGGAGTCC CAAAAGGCCT TCCGGTCCAA
841- 880 TAGAGACCTA CGTTTTCGCT CTGTTTGATG AAGATCAGAA
881- 920 AGACCCTGAA ATTGAGAAGC ATTTTGGACT ATTTTCAGCA
921- 960 AACATGCAAC CAAAGTACCA GATCAGTTTT AACTAGTTAA
961-1000 AAGCAAGAGG AGAGCATTAA TAGGAATAAG GACTTTCCTT
1001-1040 TGTATGAAGA GAAAGTAGTC CATTGGCACT ATGTACTGAA
1041-1080 ACTATATATC ATGCTCATAA AGAAAGCAGT TATTACAATA
1081-1108 ATGAAACACT TACAAGAAAA GCCATCAA

### Sequence 7A: Full-length PR-O' cloned into the plasmid pBSGL5B-12

1 CTCAATTCTT GTTTTCCTTA CAAATGGCAC ATTTAATTGT CACACTGCTT
51 CTCCTTAGTG TACTTACATT AGCTACCCTG GATTTTACAG GTGCTCAAGC
101 AGGAGTTTGT TATGGAAGGC AAGGGAATGG ATTACCATCT CCAGCAGATG
151 TTGTGTCGCT ATGCAACCGA AACAACATTC GTAGGATGAG AATATATGAT
201 CCTGACCAGC CAACTCTCGA AGCGCTTAGA GGCTCCAACA TTGAGCTCAT
251 GCTAGGTGTC CCGAATCCGG ACCTTGAGAA TGTTGCTGCT AGCCAAGCCA
301 ATGCAGATAC TTGGGTCCAA AACAATGTTA GGAACTATGG TAATGTCAAG
351 TTCAGGTATA TAGCAGTTGG AAATGAAGTT AGTCCCTTAA ATGAAAACTC
401 TAAGTATGTA CCTGTCCTTC TCAACGCCAT GCGAAACATT CAAACTGCCA
451 TATCTGGTGC TGGTCTTGGA AACCAGATCA AAGTCTCCAC AGCTATTGAA
501 ACTGGACTTA CTACAGACAC TTCTCCTCCA TCAAATGGGA GATTCAAAGA
551 TGATGTTCGA CAGTTTATAG AGCCTATCAT CAACTTCCTA GTGACCAATC
601 GCGCCCCTTT GCTTGTCAAC CTTTATCCTT ACTTTGCAAT AGCAAACAAT
651 GCAGATATTA AGCTTGAGTA TGCACTTTTT ACATCCTCTG AAGTTGTTGT
701 AAATGATAAC GGAAGAGGAT ACCGAAACCT TTTTGATGCC ATCTTAGATG
751 CCACATACTC GGCCCTTGAA AAGGCTAGTG GCTCGTCTTT GGAGATTGTT
801 GTATCAGAGA GTGGTTGGCC TTCAGCTGGA GCAGGACAAT TAACATCCAT
851 TGACAATGCC AGGACTTATA ACAACAACTT GATTAGTCAC GTGAAGGGAG
901 GGAGTCCCAA AAGGCCTTCC GGTCCAATAG AGACCTACGT TTTCGCTCTG
951 TTTGATGAAG ATCAGAAAGA CCCTGAAATT GAGAAGCATT TTGGACTATT
1001 TTCAGCAAAC ATGCAACCAA AGTACCAGAT CAGTTTTAAC TAGTTAAAAG
1051 CAAGAGGAGA GCATTAATAG GAATAAGGAC TTTCCTTTGT ATGAAGAGAA
1101 AGTAGTCCAT TGGCACTATG TACTGAAACT ATATATCATG CTCATAAAGA
1151 AAGCAGTTAT TACAATAATG AAACACTTAC AAGAAAAGCC ATCAA

This cDNA encodes the PR-O' protein (an acidic form of β-1,3 glucanase) based on the comparison to the amino acid sequence of peptides derived from the PR-O' protein.

The dot matrix comparison to the basic β-1,3-glucanase shows that the cDNA sequence 7 is probably missing about 80 bases from the 5' end. In order to isolate the full-length form of the PR-O' cDNA, the library is rescreened with a labeled EcoRI restriction fragment derived from the pBSGL6e plasmid. Six positive clones are isolated, purified and subcloned into the bluescript plasmid. The sequences of the inserts in the plasmids are determined by DNA sequencing. One clone, pBSGL5B-12, is 87 bp longer than the cDNA in pBSGL6e (sequence 7A).

### Example 18B: Isolation of cDNA clones encoding PR-2, PR-N, PR-O and PR-2'

About 300,000 plaques of the cDNA library prepared as described from RNA isolated from TMV infected tobacco leaves are screened probe comprising a mixture of labelled oligonucleotides (JR138) of the formula: ATGTTYGAYGARAAYAA, wherein each Y is independently selected from a pyrimidine C or T and each R is independently selected from a purine G or C. This probe is 17 bases long with 16 fold redundancy in the mixture. The probe design is based on an analysis of the protein data in Example 11B. The probe will recognize clones containing the PR-2, PR-N or basic β-1,3-glucanase but not PR-O' or PR-O. 30 positively hybridizing plaques are isolated and purified and their inserts are subcloneed into the bluescript plasmid. The sequence of the inserts is determined by DNA sequencing and the results indicate that at least three distinct cDNAs have been isolated. When comparing to the protein data in Example 11B, it is clear that one type of clone contains a full-length cDNA that encodes the PR-2 protein (pBSGL117), one type encodes the PR-O protein (pBSGL134), one type encodes the PR-N protein (pBSGL125 and pBSGL148) and one type encodes a highly related protein which is neither PR-2, PR-N or PR-O. We have named this type of cDNA PR-2' (pBSGL135).

In order to isolate a cDNA clone encoding PR-O and also to isolate more full-length cDNA clones, the library is screened for a second time with a PstI restriction fragment from pBSGL125. pBSGL125 is a 500 bp clone encoding a PR-N, which is truncated at the 5' end. About 300,000 plaques of the library are screened an 17 positive plaques are isolated, purified and the inserts subcloned into bluescript. The sequences of the inserts are determined by DNA sequencing.

To ensure that full-length clones are isolated from all of the acidic glucanases, two final strategies are employed. First, a final round of screening is carried out using a 210 bp, PVuII-TaqI, restriction fragment derived from pBSGL117 as a probe. In this screen, 17 clones are isolated, purified, subcloned into bluescript and their sequence is determined by DNA sequencing.

The second strategy is to amplify the 5' end of the cDNA out of the library by a PCR strategy described in Example 17. In this case, the two oligonucleotides B and C are used along with a third oligonucleotide JR209 which is complementary to positions 152 to 169 of the PR-2 cDNA sequence. In this experiment, two PCRs are carried out; one containing an aliquot of the cDNA library as template and the primers JR209 and DP01 (oligo B from Example 17) and the other using an aliquot of the cDNA library as a template and using JR209 and GP38 (Oligo C from Example 17) as primers. The sequence of JR209 is as follows:

JR209 5' AACATCTTGGTCTGATGG 3'.

A positive control in the amplification experiment is an aliquot of the purified GL117 clone encoding the full-length PR-2 cDNA amplified with JR209 and DP01. A negative control in the experiment is an amplification using JR209 and GP38. Aliquots of the products of the PCR are analyzed by agarose gel electrophoresis and it is found that a band about the size of the positive control amplified from the library for each set of primers. This result suggests that the procedure is successful and so the remaining DNA is purified and then treated with the Klenow fragment of DNA polymerase I in the presence of all four dNTPs, as described by Maniatis et al. (1982) to make the ends of the DNA molecules "flush". The Klenow enzyme is inactivated by heating to 65°C for 15 minutes and the DNA is then restricted with EcoRI. The DNA is purified and then electrophoresed on a 1.5 % LGT agarose gel. The band of DNA of the correct size is excised from the gel and used to ligate into the bluescript plasmid, which has been restricted with both EcoRI and EcoRV. The ligation is used to transform bacteria and positive colonies are selected and analyzed by DNA sequencing.

The result of the preceeding procedures is the isolation of clones comprising the full-length cDNA clones for PR-2, PR-N, PR-O and a fourth type of acidic glucanase, PR-2', which encodes an unknown protein. The sequence of PR-2 is as follows:

### Sequence 8: PR-2 cloned in the plasmid pBSGL117

1 GTGTTTCTTA CTCTCTCATT TCCATTTTAG CTATGACTTT ATGCATTAAA
51 AATGGCTTTC TTGCAGCTGC CCTTGTACTT GTTGGGCTGT TAATTTGCAG
101 TATCCAAATG ATAGGGGCAC AATCTATTGG AGTATGCTAT GGAAAACATG
151 CAAACAATTT ACCATCAGAC CAAGATGTTA TAAACCTATA CGATGCTAAT
201 GGCATCAGAA AGATGAGAAT CTACAATCCA GATACAAATG TCTTCAACGC
251 TCTCAGAGGA AGTAACATTG AGATCATTCT CGACGTCCCA CTTCAAGATC
301 TTCAATCCCT AACTGATCCT TCAAGAGCCA ATGGATGGGT CCAAGATAAC
351 ATAATAAATC ATTTCCCAGA TGTTAAATTT AAATATATAG CTGTTGGAAA
401 TGAAGTCTCT CCCGGAAATA ATGGTCAATA TGCACCATTT GTTGCTCCTG
451 CCATGCAAAA TGTATATAAT GCATTAGCAG CAGCAGGGTT GCAAGATCAA
501 ATCAAGGTCT CAACTGCAAC ATATTCAGGG ATCTTAGCGA ATACCAACCC
551 GCCCAAAGAT AGTATTTTTC GAGGAGAATT CAATAGTTTC ATTAATCCCA
601 TAATCCAATT TCTAGTACAA CATAACCTTC CACTCTTAGC CAATGTCTAT
651 CCTTATTTTG GTCACATTTT CAACACTGCT GATGTCCCAC TTTCTTATGC
701 TTTGTTCACA CAACAAGAAG CAAATCCTGC AGGATATCAA AATCTTTTTG
751 ATGCCCTTTT GGATTCTATG TATTTTGCTG TAGAGAAAGC TGGAGGACAA
801 AATGTGGAGA TTATTGTATC TGAAAGTGGC TGGCCTTCTG AAGGAAACTC
851 TGCAGCAACT ATTGAAAATG CTCAAACTTA CTATGAAAAT TTGATTAATC
901 ATGTGAAAAG CGGGGCAGGA ACTCCAAAGA AACCTGGAAA TGCTATAGAA
951 ACTTATTTAT TTGCCATGTT TGATGAAAAT AATAAGGAAG GAGATATCAC
1001 AGAGAAACAC TTTGGACTCT TTTCTCCTGA TCAGAGGGCA AAATATCAAC
1051 TCAATTTCAA TTAATTAATG CATGGTAACA TTTATTGATA TATATAGTGA
1101 TATGAGTAAT AAGGAGAAGT AGAACTGCTA TGTTTTTCTC TTCAATTGAA
1151 AATGTAACTC TGGTTTCACT TTGATATTTA TATGACATGT TTATTGAGAT
1201 CTAA

pBSGL134 is the isolated plasmid containing a cDNA insert encoding a PR-O protein.
The λ phages λtobcDNAGL153 and λtobcDNAGL162 are clones containing the full-length PR-O cDNA. The phage λtobcDNAGL161 is a clone containing the full length PR-2' cDNA.
The determination of the protein encoded in the cDNA insert is based on a comparison between the protein data in 11B and the deduced protein sequence encoded by the cDNA insert.

### Example 19: Isolation of cDNA clones encoding SAR8.2a and SAR8.2b

Filter lifts of the sub-cloned library prepared above are screened with labeled cDNA probes in a method described previously (St. John and Davis, 1979) except that the same filter lifts are screened sequentially rather than by screening replicate lifts. The first probe is synthesized as described, using reverse transcriptase and [α³²P]-dCTP, from mRNA of the mock-induced RNA sample isolated above; following probing and exposure to X-ray film the same filters are probed again (without stripping) using probe synthesized as above but from the TMV-induced RNA sample isolated above. Following exposure the two X-ray films are compared using a computerized digital image analysis system (Biological Vision, Inc., San Jose, California) according to the manufacturer's specifications, and plaques are selected that yield an increased signal when probed with the TMV-induced probe. The selected plaques are purified by a second round of probing at a selected plaque density of approximately 100/plate, and the cDNA inserts are recovered from the phage as follows: a small amount of isolated phage DNA is digested with EcoRI followed by inactivation of the restriction enzyme, dilution, re-ligation with T4 DNA ligase, and transformation into *E. coli* strain DH-5α, (Bethesda Research Laboratories, Bethesda, Maryland) followed by selective growth on ampicillin-containing culture plates. This procedure allows direct recovery of the cDNA contained in the plasmid vector from the phage vector. The DNA sequences of these two slightly different clones are shown as sequence 9 and sequence 10 for the cDNA in the plasmids pCIB/SAR8.2a (eg cDNA SAR8.2a) and pCIB/SAR8.2b (eg cDNA SAR8.2b).

### Sequence 9: SAR8.2a cDNA cloned into the plasmid pCIB/SAR8.2a

1- 40 ACAGTAAAAA ACTGAAACTC CAAATAGCTC ATCAAAATGT
41- 80 TTTCCAAAAC TAACCTTTTT CTTTGCCTTT CTTTGGCTAT
81-120 TTTGCTAATT GTAATATCCT CACAAGCTGA TGCAAGGGAG
121-160 ATGTCTAAGG CGGCTGTTCC AATTACCCAA GCAATGAATT
161-200 CAAACAACAT TACTAATCAG AAGACGGGTG CCGGAATCAT
201-240 CCGTAAGATA CCGGGTTGGA TACGAAAAGG TGCAAAACCA
241-280 GGAGGCAAAG TCGCCGGCAA AGCTTGTAAA ATTTGCTCAT
281-320 GTAAATACCA GATTTGCAGC AAATGTCCTA AATGTCATGA
321-360 CTAAAGTTAG GCCTTGAGAC TATGTACTTG TGCTGGTGTG
361-400 AGTTTAGTTT TGAGAGTAAA GGGAAAGTTA TGAATAGCCT

401-440 AATATAATTG TATTCACTAT GTTTTCTTAG TAATTCTTAT
441-480 TGTTGAAACT TGGAACAGGT CTTTGGGTCA AAATGTACCT
481-520 CTTGTCTTGT AGTCTTTCAA CTGTATAGTA TTGTACTGTA
521-560 TTTTTCTTT

### Sequence 10: SAR8.2b cDNA cloned into the plasmid pCIB/SAR8.2b

1- 40 GACAGTAAAA AACTGAAACT CCAAATAGCT CATCAAAATG
41- 80 TTTTCCAAAA CTAACCTTTT TCTTTGCCTT TCTTTGGCTA
81-120 TTTTGCTAAT TGTAATATCC TCACAAGCTG ATGCAAGGCA
121-160 GATTTCTAAG GCGGCTGCTC CAATTACCCA TGCAATGAAT
161-200 TCAAACAACA TTACTAATCA GAAGACGGGT GCCGGAATCA
201-240 TCCGTAAGAT ACCGGGTTGG ATACGAAAAG GTGCAAAACC
241-280 AGGAGGCAAA GTCGCCGGCA AAGCTTGTAA AATTTGCTCA
281-320 TGTAAATACC AGATTTGCAG CAAATGTCCT AAATGTCATG
321-360 ACTAAAGTTA GGCCTTGAGA CTATGTACTT GTGCTGGTGT
361-400 GAGTTTAATT TTGAGAGTAA AGGGAAAGTT ATGAATAGCC
401-440 TAATATAATT CTATTCACTA TGTTTTCTTA GTAATTCTTA
441-480 TTGTTGAAAC TTGGAACAGG TCTTTGGGTC AAAATGTACC
481-520 TCTTGTCTTG TAGTCTTTCA ACTGTATGGT ATTGTACTGT
521-560 ATCTTTCTTT AGCCACTTGA TATCAAATCC GATTAAATCT

Analysis of RNA from mock-induced vs TMV-induced tobacco leaves, using Northern analysis and Primer extension assay confirms that steady-state levels of the pSAR8.2 family mRNAs are increased by TMV induction.

### Example 20: Isolation of cDNA clones encoding the chitinase/lysozyme from cucumber

Two regions of the protein sequence determined in Example 12, are selected and oligonucleotide probes are synthesized that are complementary to all possible combinations of mRNA capable of encoding the peptides. The sequences of the probes are: 
                  G  T           A
Probe 1:    5'-CCATTCTGNCCCCAGTA-3'
 
G G G G C
Probe 2: 5'-GGATTATTATAAAATTGNACCCA-3'.
 About 300,000 plaques are plated from the library constructed above and duplicate plaque lifts are probed either with ³²P labeled oligonucleotide mixture 1 (probe 1) or mixture 2 (probe 2). Plaques are isolated that show positive results when screened with either probe. Isolation of phage and automatic excision are carried out as described in the Stratagene Lambda Zap laboratory manual, Stratagene, San Diego, USA.

Once the chitinase cDNA clones are isolated in the bluescript plasmid they are sequenced by dideoxy sequencing. The sequence of the chitinase cDNA contained in the plasmid pBScuccht5 (aka pBScucchi/chitinase) is as follows:

### Sequence 11: Cucumber chidnase/lysozyme cDNA cloned into the plasmid pBScucchi/chitinase. (aka pBScuccht5)

1 AAAGAAAGCT CTTTAAGCAA TGGCTGCCCA CAAAATAACT ACAACCCTTT
51 CCATCTTCTT CCTCCTTTCC TCTATTTTCC GCTCTTCCGA CGCGGCTGGA
101 ATCGCCATCT ATTGGGGTCA AAACGGCAAC GAGGGCTCTC TTGCATCCAC
151 CTGCGCAACT GGAAACTACG AGTTCGTCAA CATAGCATTT CTCTCATCCT
201 TTGGCAGCGG TCAAGCTCCA GTTCTCAACC TTGCTGGTCA CTGCAACCCT
251 GACAACAACG GTTGCGCTTT TTTGAGCGAC GAAATAAACT CTTGCAAAAG
301 TCAAAATGTC AAGGTCCTCC TCTCTATCGG TGGTGGCGCG GGGAGTTATT
351 CACTCTCCTC CGCCGACGAT GCGAAACAAG TCGCAAACTT CATTTGGAAC
401 AGCTACCTTG GCGGGCAGTC GGATTCCAGG CCACTTGGCG CTGCGGTTTT
451 GGATGGCGTT GATTTCGATA TCGAGTCTGG CTCGGGCCAG TTCTGGGACG
501 TACTAGCTCA GGAGCTAAAG AATTTTGGAC AAGTCATTTT ATCTGCCGCG
551 CCGCAGTGTC CAATACCAGA CGCTCACCTA GACGCCGCGA TCAAAACTGG
601 ACTGTTCGAT TCCGTTTGGG TTCAATTCTA CAACAACCCG CCATGCATGT
651 TTGCAGATAA CGCGGACAAT CTCCTGAGTT CATGGAATCA GTGGACGGCG
701 TTTCCGACAT CGAAGCTTTA CATGGGATTG CCAGCGGCAC GGGAGGCAGC
751 GCCGAGCGGG GGATTTATTC CGGCGGATGT GCTTATTTCT CAAGTTCTTC
801 CAACCATTAA AGCTTCTTCC AACTATGGAG GAGTGATGTT ATGGAGTAAG

851 GCGTTTGACA ATGGCTACAG CGATTCCATT AAAGGCAGCA TCGGCTGAAG
901 GAAGCTCCTA AGTTTAATTT TAATTAAAGC TATGAATAAA CTCCAAAGTA
951 TTATAATAAT TAAAAAGTGA GACTTCATCT TCTCCATTTA GTCTCATATT
1001 AAATTAGTGT GATGCAATAA TTAATATCCT TTTTTTCATT ACTATACTAC
1051 CAATGTTTTA GAATTGAAAA GTTGATGTCA ATAAAAACAT TCCAAGTTTA
1101 TTT

### Example 20B: Cucumber peroxidase cDNA

An oligonucleotide probe is designated to isolate cDNAs encoding the cucumber peroxidase based on the protein data presented in Example 12B. The sequence of the mixture of oligonucleotides is as follows: JR74 5' ACRAARCARTCRTGRAARTG 3'. wherein each R is independently selected from a purine G or C. This oligonucleotide mixture is 20 bases long and contains 64 species.

A cDNA library is prepared from RNA isolated from leaves of cucumber plants five days after infection with tobacco necrosis virus as described in Example 14B. This library is constructed in the Lambda ZAP cloning vector.

About 300,000 plaques of this cDNA library are screened with the oligonucleotide probe and 25 plaques are isolated. These are rescreened several times and purified. As a result of this process only six plaques remain as still positive after many rounds of purification. The inserts contained in these clones are excised using the automatic excision protocol described in the Stratagene Lambda ZAP laboratory manual. The inserts are sequenced by double stranded dideoxy sequencing and then the structures are analyzed by dot matrix analysis comparing to the sequence of an acidic, lignin-forming peroxidase isolated from tobacco (Lagrimini et al., 1987). Two of these clones, Per1 and Per25, show some limited homology to the tobacco cDNA and are chosen for further analysis. Upon complete sequencing of the clones, it is found that they encode the same protein.

A probe derived from the cucumber peroxidase is then used to rescreen the cucumber library and about 30 plaques are isolated. After purification the inserts are excised from the phage as plasmids and then the sequence is determined by DNA sequencing.

The results of this analysis is the isolation of two types of peroxidase cDNA clones from cucumber. One encoding a basic protein encoded by the plasmid pBSPER1, the nucleotide sequence is as follows, and the other encoding a related peroxidase contained in the plasmids, pBSPER24 and pBSPER25.

### Sequence 12: Cucumber Peroxidase cDNA cloned into the plasmid pBSPER1

1 ACCAGAGAAG ACCCCATTTG CAGTATCAAA AATGGGTTTA CCTAAAATGG
51 CAGCCATTGT TGTGGTGGTG GCTTTGATGC TATCACCCTC TCAAGCCCAG
101 YTTTCTCCTT TCTTCTACGC CACCACATGC CCTCAGCTGC CTTTCGTTGT
151 TCTCAACGTG GTTGCCCAAG CCCTACAGAC TGATGACCGA GCTGCTGCTA
201 AGCTCATTCG CCTCCATTTT CATGATTGCT TTGTCAATGG GTGTGATGGA
251 TCGATTCTAT TGGTAGACGT ACCGGGCGTT ATCGATAGTG AACTTAATGG
301 ACCTCCAAAT GGTGGAATCC AAGGAATGGA CATTGTGGAC AACATCAAAG
351 CAGCAGTTGA GAGTGCTTGT CCAGGAGTTG TTTCTTGCGC TGATATCTTA
401 GCCATTTCAT CTCAAATCTC TGTTTTCTTG TCGGGAGGAC CAATTTGGGT
451 TGTACCAATG GGAAGAAAAG ACAGCAGAAT AGCCAATAGA ACTGGAACCT
501 CAAACTTACC TGGTCCCTCA GAAACTCTAG TGGGACTTAA AGGCAAGTTT
551 AAAGATCAAG GGCTTGATTC TACAGATCTC GTGGCTCTAT CAGGAGCCCA
601 CACGTTTGGA AAATCAAGAT GCATGTTCTT CAGTGACCGC CTCATCAACT
651 TCAACGGCAC AGGAAGACCC GACACAACGC TTGACCCAAT ATACAGGGAG
701 CAGCTTCGAA GACTTTGTAC TACTCAACAA ACACGAGTAA ATTTCGACCC
751 AGTCACACCC ACTAGATTTG ACAAGACCTA TTACAACAAT TTGATTAGCT
801 TAAGAGGGCT TCTCCAAAGC GACCAAGAGC TCTTCTCAAC TCCCAGAGCT
851 GATACCACAG CCATTGTCAR AACTTTTGCT GCCAACGAAC GTGCCTTCTT
901 TAAACAATTT GTGAAATCAA TGATCAAAAT GGGCAACCTC AAGCCTCCCC
951 CTGGCATTGC ATCAGAAGTT AGATTGGACT GTAAGAGGGT CAACCCAGTC
1001 AGAGCCTACG ACGTTATGTA ATAACTTTAT CCCACTTCAT CCCTTCTACT
1051 TTTGCTGTCT CTTGTACTAC TTTGTTGATG TATTAGTTCA ACCGGTTAAG
1101 ATATATATAT CGTTGACCTA AATAATAGAT C

### Section 3C: Development of a novel, differential cloning and screening technology.

### Example 21: Differential Enrichment Scheme

A method has been conceived and developed which will allow efficient enrichment of sequences present in one population of molecules in greater amounts than in another population. The method's greatest utility is in situations where the populations are very similar and the differentially present sequences represent a very small proportion of the population.

If two populations of clones are similar and one wishes to isolate those clones which are present in one population in higher amounts (i.e. "induced" or differentially regulated"), past techniques involved screening with probes from the two populations (+/- screening (St. John and Davis, 1979), or enrichment of probes or mRNAs by hybridization and hydroxy-apatite (HAP) chromatography (Davis et al., 1984). The first method has a demonstrated sensitivity limitation in that only clones present in greater than about one in 2,000 will be detected. The second is laborious, technically difficult, and achieves enrichments of 20 to 50 fold at best.

The present method involves exploiting two recent developments in molecular technology: PCR (Saiki et al., 1988) and biotin-avidin chromatography (Stahl et al., 1988). PCR allows simple synthesis of large amounts of DNA of specified sequence. Biotin-avidin chromatography allows the efficient separation of molecules bearing a biotin affinity tag from those molecules which do not bear the tag.

In its general form, the technique consists of isolating single strands of cDNA representing two different populations ("induced" vs "uninduced"), but of opposite cDNA polarity for the two populations, i.e. one of "sense" polarity relative to mRNAs, and the other its complement, or "anti-sense", polarity relative to mRNAs. The isolated strands from the "induced " population would have no affinity tag, while the strands of opposite polarity from the "uninduced" populations would have stable affinity tags. When these two populations are hybridized together, hybrids will form between complementary strands present in the two populations. Those strands from the "induced" population which have no counterparts, or many fewer counterparts, in the "uninduced" population, would remain single stranded. Due to the presence of the affinity tag (in essence a handle) on the strands of the "uninduced" population molecules, those strands and, most importantly, any hybrid molecules can be removed from the mixture by affinity chromatography. This leaves only those "induced" molecules which are not significantly represented in the "uninduced" population. These "induced" molecules can then be cloned by standard means and serve as an enriched population from which to isolate "induced" clones; alternatively, the enriched molecules can be amplified individually and sequenced directly.

An alternate scheme is the same as described above except that it involves incorporating a labile affinity tag only on the "induced" population molecules, while the affinity tag on the "uninduced" population is stable. "Labile" in this case means that the affinity tag can be removed at will, or be altered at will in such a way that it no longer serves as an affinity tag. In this scheme all the molecules in the hybridization mixture could bind to the affinity matrix, but only those "induced" molecules that are not hybridized to a complementary "uninduced" counterpart could be selectively recovered from the matrix for subsequent cloning.

The following is a specific example of the first scheme described above, using cDNA populations in phage cloning vectors. The "induced" and "uninduced" cDNA populations (constructed from mRNA from TMV-induced or mock-induced tobacco leaves in this case) are cloned in two different phages so that the primers used for amplifying the clone inserts will be different (and thus not hybridize in later steps). The "induced" cDNA bank is constructed in λGEM-4 (Promega, Inc.) and the "uninduced" or "mock-induced" DNA bank is constructed in λZAP-II (Stratagene, Inc.). For each vector specific oligonucleotide primers are synthesized such that they represent sequences in the phage vector immediately adjacent to the cDNA cloning site, and that, when used in the polymerase chain reaction in the presence of the corresponding phage, the DNA cloned into the cDNA cloning site will be amplified. A population of phages representing all the members of each bank are amplified together, producing a population of cDNA inserts representing the bank. One of the oligonucleotide primers in each case is biotinylated so that a specific strand of each amplified bank DNA can be positively selected by avidin affinity chromatography and strand separation, followed by release and recovery of the biotin-selected strand; importantly, the primers used for biotinylation are selected such that strands of opposite polarity are selected from the "induced" vs "uninduced" cDNA banks.

The biotin tag in the case of the "induced" DNA is a labile one in that the spacer arm through which the biotin moiety is attached contains a disulfide linkage, and the tag in the "uninduced" DNA is a stable one. In this case the single strand from the "induced" population is released by DTT treatment, losing its biotin tag, and the single strand from the "uninduced" population is released by denaturation of the avidin molecule, while retaining its biotin tag. When the recovered "target" DNA (single strand from the "induced" library) is hybridized to the recovered "driver" DNA (single strand from the "mock-induced" or "uninduced" library), the complexes that are formed, and the excess "driver" DNA can be removed by avidin affinity chromatography. The remaing "target" DNA still bears the primer sequences, making its recovery, by subsequent repair or amplification and cloning, very simple.

In the alternate scheme, both the "target" and "driver" single stranded DNAs are recovered by denaturation of the avidin on the affinity matrix, and both retain their biotin tags. After hybridization all the molecules are bound to the affinity matrix and following washing, the non-hybridized "target" DNA, bearing the "liable" affinity tag, is selectively eluted from the matrix by DTT.
This modification allows a positive selection for the single stranded "target" DNA, avoiding potential problems with less-than-quantitative binding of the hybridization mix to the affinity matrix. The advantage of this technique over those previously described is the ability to isolate those genes which are turned on only to low levels, in specific circumstances, and which may play a causative role in some important biological phenomenon.
In both forms of the technique described above, the "target" and "driver" single stranded DNA is generated by biotin-avidin affinity chromatography.

An alternative method for generating these single-stranded populations is a method described as "asymmetric" PCR (Gyllensten and Erlich, 1988). This consists of multiple cycles of polymerase extension and denaturation, as in PCR, but in the presence of only one of the primers. The primer chosen determines the polarity of the resultant DNA, thus allowing the selective polarity critical to the technique as described above. Asymmetric PCR in this case is most easily accomplished using as template a small amount of double-stranded PCR products of the relevant population, from which the excess primers have been removed.

### SECTION 4. CHIMERIC GENES

This section describes combinations of cDNA sequences with sequences that promote transcription of the cDNAs and with those that facilitate processing of the 3' end of the mRNA in the plant cells. The first set of examples covers the construction of plasmids which contain the expression cassette and the second section describes the subcloning of cDNAs into the cassettes in both sense and anti-sense orientation.

### Section 4A: Construction of plasmids containing plant expression cassettes.

### Example 22: Construction of pCGN1509 (tobacco RUBISCO small subunit promoter cassette)

pCGN1509 is an expression cassette plasmid containing the 5' regulatory region and promoter from a tobacco RUBISCO small subunit gene, and the 3' region from the octopine synthase (*ocs*) gene of the Ti plasmid of *A. tumefaciens*, with unique restriction sites between these two parts. The 5' regulatory region is ultimately derived from a 3.4 kb EcoRI fragment containing tobacco RUBISCO small subunit gene TSSU3-8 (O'Neal et al., 1988).

The 3.4 kb EcoRI fragment of TSSU3-8 is cloned into the EcoRI site of M13 mp18 (Yanisch-Perron et al., 1985) to yield an M13 clone 8B. Single-stranded DNA is used as a template to extend oligonucleotide primer "Probe 1" (O'Neal et al., 1987) using the Klenow fragment of DNA polymerase I. Extension products are treated with mung bean nuclease and then digested with HindIII to yield a 1450 bp fragment containing the small subunit promoter region; the fragment is cloned into HindIII-SmalI digested pUC18 (Yanisch-Perron et al., 1985) to yield pCGN625. The BamHI-EcoRI fragment of pCGN625 is cloned into the large BamHI-EcoRI fragment (plasmid backbone) of BamHI-EcoRI digested pCGN607 in which the SmaI site at position 11207 (Barker et al., 1983) of the *ocs* 3' region is converted to a BglII site by ligation of a synthetic BglII linker (Facciotti et al., 1985). This yields plasmid pCGN630. The BamHI site of pCGN630 is deleted by digestion with BamHI and treatment with the Klenow fragment of DNA polymerase I to create pCGN1502. The KpnI site of pCGN1502 is replaced with a BamHI site by digestion of pCGN1502 with Kpnl, treatment with Klenow enzyme, and ligation of a synthetic BamHI linker. The resulting construction is pCGN1509.

### Example 23: Construction of pCGN1761 (a double CaMV 35S - promoter/terminator cassette containing ampicillin resistance) and pCGN1431 (a double CaMV 35S promoter /terminator cassette - containing chloramphenicol resistance)

pCGN1761 contains a double CaMV 35S promoter and the *tm-l* 3' region with an EcoRI site between contained in a pUC-derived plasmid backbone. The promoter-EcoRI-3' processing site cassette is bordered by multiple restriction sites for easy removal. The plasmid is derived by a series of steps (see below) from an initial double-35S plasmid, pCGN2113, which itself is derived from pCGN164, and pCGN638.

pCGN1431 also contains the double CaMV 35S promoter and the *tm-1* 3' region with a multiple cloning site between them. This promoter/terminator cassette is contained in a pUC-derived vector which contains a chloramphenicol rather than ampicillin resistance gene. The cassette is bordered by multiple restriction sites for easy removal.

A. Construction of pCGN986. pCGN986 contains a 35S CaMV 35 promoter and a T-DNA *tm-l* 3'-region with multiple restriction sites between them. pCGN986 is derived from another plasmid, pCGN206, containing a CaMV 35S promoter and a different 3' region, the CaMV region VI 3'-end. The CaMV 35S promoter is cloned as an AluI fragment (bp 7144-7734) (Gardner et al., 1981) into the HincII site of M13mp7 (Messing et al., 1981) to create C614. An EcoRI digest of C614 produces the EcoRI fragment from C614 containing the 35S promoter which is cloned into the EcoRI site of pUC8 (Vieira and Messing, 1982) to produce pCGN147.

pCGN148a containing a promoter region, selectable marker (Kanamycin with 2 ATGs) and 3' region, is prepared by digesting pCGN528 with BglII and inserting the BamHI-BglII promoter fragment from pCGN147. This fragment is cloned into the BglII site of pCGN528 so that the BglII site is proximal to the kanamycin gene of pCGN528.

The shuttle vector, pCGN528, used for this construct is made as follows: pCGN525 is made by digesting a plasmid containing Tn5 which harbors a kanamycin gene (Jorgensen et al., 1979) with HindIII-BamHI and inserting the HindIII-BamHI fragment containing the kanamycin resistance gene into the HindIII-BamHI sites in the tetracycline gene of pACYC184 (Chang and Cohen, 1978) pCGN526 is made by inserting the BamHI fragment 19 of pTiA6 (Thomashow et al., 1980) modified with XhoI linkers inserted into the SmaI site, into the BamHI site of pCGN525. pCGN528 is obtained by deleting the small XhoI and religating.

pCGN149a is made by cloning the BamHI Kanamycin gene fragment from pMB9KanXXI into the BamHI site of pCGN148a. pMB9KanXXI is a pUC4K variant (Vieira and Messing, 1982) which has the Xhol site missing but contains a function kanamycin gene from Tn903 to allow for efficient selection in *Agrobacterium.*

pCGN149a is digested with HindIII and BamHI and ligated to pUC8 digested with HindIII and BamHI to produce pCGN169. This removes the Tn903 kanamycin marker. pCGN565 and pCGN169 are both digested with HindIII and PstI and ligated to form pCGN203, a plasmid containing the CaMV 35S promoter and part of the 5'-end of the TN5 kanamycin gene (up to the PstI site, Jorgensen et al., 1979). A 3' regulatory region is added to pCGN203 from pCGN204 (an EcoRI fragment of CaMV (bp 408-6105) containing the 3' region of gene VI subcloned into pUC18 (Gardner et al., 1981) by digestion with HindIII and PstI and ligation. The resulting cassette, pCGN206, is the basis for the construction of pCGN986.

The pTiA6 T-DNA *tm-l* 3'-sequences are subcloned from the Bam19 T-DNA fragment (Thomashow et al., 1980) as a BamHI-EcoRI fragment (nucleotides 9062 to 12, 823, numbering as in Barker et al., 1983) and combined with the pACYC184 (Chang and Cohen, 1978) origin of replication as an EcoRI-HindII fragment and a gentamycin resistance marker (from plasmid pLB41), [D. Figurski] as a BamHI-HindII fragment to produce pCGN417.

The unique SmaI site of pCGN417 (nucleotide 11,207 of the Bam19 fragment) is changed to a SacI site using linkers and the BamHI-SacI fragment is subcloned into pCGN565 to give pCGN971. The BamHI site of pCGN971 is changed to an EcoRI site using linkers to yield pCGN971E. The resulting EcoRI-SacI fragment of pCGN971E, containing the *tm-1* 3' regulatory sequences, is joined to pCGN206 by digestion with EcoRI and SacI to give pCGN975. The small part of the Tn5 kanamycin resistance gene is deleted from the 3'-end of the CaMV 35S promoter by digestion with SalI and BglII, blunting the ends and ligation with Sail linkers. The final expression cassette pCGN986 contains the CaMV 35S promoter followed by two Sail sites, an XbaI site, BamHI, SmaI Kpnl and the *tm-1* 3'region (nucleotides 11207-9023 of the T-DNA).

B. Construction of pCGN164. The AluI fragment of CaMV (bp 7144-7735) (Gardner et al., 1981) is obtained by digestion with AluI and cloned into the HincII site of M13mp7 (Vieira and Messing, 1982) to create C614. An EcoRI digest of C614 produces the EcoRI fragment from C614 containing the 35S promoter which is cloned into the EcoRI site of pUC8 (Vieira and Messing, 1982) to produce pCGN146. To trim the promoter region, the BglII site (bp7670) is treated with BglII and Bal31 and subsequently a BglII linker is attached to the Bal31 treated DNA to produce pCGN147. PCGN147 is digested with EcoRI HphI and the resultant EcoRI-HphI fragment containing the 35S promoter is ligated into EcoRI-SmalI digested M13mp8 (Vieira and Messing, 1982) to create pCGN164.

C. Construction of pCGN638. Digestion of CaMV10 (Gardner et al. 1981) with BglII produces a BglII fragment containing a 35S promoter region (bp 6493-7670) which is ligated into the BamHI site of pUC19 (Norrander et al., 1983) to create pCGN638.

D. Construction of pCGN2113. pCGN164 is digested with EcoRV and BamHI to release a EcoRV-BamHI fragment which contains a portion of the 35S promoter (bp 7340-7433); pCGN638 is digested with HindIII and EcoRV to release a HindIII-EcoRV fragment containing a different portion of the 35S promoter (bp 6493-7340). These two fragments are ligated into pCGN986 which has been digested with HindIII and BamHI to remove the KindIII-BamHI fragment containing the 35S-promoter; this ligation produces pCGN639 which contains the backbone and *tm-l* 3' region from pCGN986 and the two 35S promoter fragments from pCGN164 and pCGN638. pCGN638 is digested with EcoRV and DdeI to release a fragment of the 35S promoter (bp 7070-7340); the fragment is treated with the Klenow fragment of DNA polymerase I to create blunt ends, and is ligated into the EcoRV site of pCGN639 to produce pCGN2113 having the fragment in the proper orientation.

E. Construction of pCGN1761. pCGN2113 is digested with EcoRI and the plasmid is ligated in the presence of a synthetic DNA adaptor containing an XbaI site and a BamHI site (the adaptor contains EcoRI sticky ends on either end, but the adjacent bases are such that an EcoRI site is not reconstructed at this location) to produce pCGN2113M. pCGN2113M is digested to completion with SacI and then subjected to partial digestion with BamHI. This DNA is then treated with T4 DNA polymerase to create blunt ends and an EcoRI linker is ligated into the blunt-ended plasmid. After transformation a plasmid clone which contains a unique EcoRI site between the promoter and the intact *tm-l* 3' region is selected and designated pCGN1761.

F. Contruction of pCGN1431. The salI-EcoRI fragment of pCGN2113, which contains the entire promoter-polylinker-3' cassette, is removed by SalI-EcoRI digestion and cloned into SalI-EcoEI digested pCGN565 to create pCGN2120; pCGN565 is a cloning vector based on pUC8-Cm [K. Buckley, PH.D. Thesis, UC San Diego 1985], but containing the polylinker from pUC18 (Yanisch-Perron et al., 1985). pCGN2120 is digested to completion with PstI and then religated. A clone is selected which has deleted only the 858 bp PstI-PstI fragment (9207-10065, Barker et al., 1983) from the *tm-l* 3'region to create pCGN1431.

### Section 4B: Chimeric Genes

### Example 24: Construction of pCGN1752A and pCGN1752B (SSU-promoter/PR-1A expression cassette (sense and anti-sense orientation))

The 807 bp EcoRI fragment of pBSPR1-207 is subcloned into EcoRI digested pCGN1509 and plasmids bearing the cDNA in each of the two possible orientations are selected; a plasmid in which the tobacco RUBISCO small subunit promoter would be expected to generate a transcript with the mRNA sense strand of PR1a is designated pCGN1752A, and a plasmid in which the tobacco RUBISCO small subunit promoter would be expected to generate a transcript with the antisense strand (i.e. complementary sequence of the mRNA) of PR1a is designated pCGN1752B.

### Example 25: Construction of pCGN1753A and pCGN1753B (SSU - promoter/PR-1B expression cassette (sense and anti-sense orientation)

The 717 bp EcoRI fragment of pBSPR1-1023 is subcloned into EcoRI digested pCGN1509 and plasmids bearing the cDNA in each of the two possible orientation are selected; a plasmid in which the tobacco RUBISCO small subunit promoter would be expected to generate a transcript with the mRNA sense strand of PR-1b is designated pCGN1753A, and a plasmid in which the tobacco RUBISCO small subunit promoter would be expected to generate a transcript with the antisense strand (i.e. complementary sequence of the mRNA) of PR-1b is designated pCGN1753B.

### Example 26: Construction of pCGN1762A and pCGN1762B (Double CaMV 35S promoter/PR-1A expression cassette (sense and anti-sense orientation))

A 807 bp EcoRI fragment bearing a tobacco PR1a cDNA is released from pBSPR1-207 by EcoRI digestion and subcloned into EcoRI digested pCGN565 to yield pCGN1750. A 717 bp EcoRI fragment bearing the entire coding region of a tobacco PR-1b cDNA is released from pBSPR1-1023 by digestion with EcoRI and subcloned into EcoRI digested pCGN565 to yield pCGN1751. These two plasmids are constructed to facilitate subsequent subcloning experiments.

The 807 bp EcoRI fragment of pCGN1750 is subcloned into EcoRI digested pCGN1761 and plasmids bearing the cDNA in each of the two possible orientations are selected; a plasmid in which the double 35S promoter would be expected to generate a transcript with the mRNA sense strand of PR-1a is designated pCGN1762A, and a plasmid in which the double 35S promoter would be expected to generate a transcript with the anti-sense strand (i.e. complementary sequence of the mRNA) of PR-1a is designated pCGN1762B.

### Example 27: Construction of pCGN1763A and pCGN1763B (Double CaMV 35S promoter/PR-1b expression cassette (sense and anti-sense orientation))

The 717 bp EcoRI fragment of pCGN1751 (see above) is subcloned into EcoRI digested pCGN1761 and plasmids bearing the cDNA in each of the two possible orientations are selected; a plasmid in which the double 35S promoter would be expected to generate a transcript with the mRNA sense strand of PR-1b is designated pCGN1763A, and a plasmid in which the double 35S promoter would be expected to generate a transcript with the anti-sense strand (i.e. complementary sequence of the mRNA) of PR-1b is designated pCGN1763B.

### Example 28: Construction of pCIB 1002 and pCIB1003 (Double CaMV 35S promoter/PR-R major expression cassettes (sense and anti-sense orientation)

The plasmid pBSPRR-401 is partially digested with EcoRI and the resulting DNA fragments are separated on a 1.0 % LGT agarose gel. A band at about 900 bp, which contains the full length PR-R cDNA insert, is excised and ligated to pCGN1761 which has been digested to completion with EcoRI and dephosphorylated using calf intestine alkaline phosphatase. The DNA is ligated and transformed as described above, positive colonies are screened and their plasmids are analyzed. One plasmid which contains the PR-R cDNA in a sense orientation relative to the double CaMV 35S promoter, is selected and designated as pCIB1002. A second plasmid, in which the PR-R cDNA is in an anti-sense orientation relative to the double CaMV 35S promoter is selected and designated pCIB1003.

### Example 29: Construction of pCIB1020 and pCIB1021 (Double CaMV 35S promoter/PR-P expression cassette (sense and anti-sense orientation)

The plasmid pBScht28 (see above) is digested with EcoRI and fragments are separated on a 0.5 % LGT agarose gel. The band containg the PR-P cDNA is excised and mixed with pCGN1761 (see above) which has been digested with EcoRI, treated with CIAP and purified on a 0.5 % LGT agarose gel. The mixture is ligated and transformed as described. Plasmids are screened for insertion of the PR-P cDNA in either orientation. One plasmid, in which the PR-P cDNA is inserted in a sense orientation relative to the double CaMV 35S promoter, is designated pCIB1020. A plasmid in which the PR-P cDNA is inserted in an anti-sense orientation relative to the double CaMV 35S promoter is designated pCIB1021.

### Example 30: Construction of pCIB1022 and pCIB1023 (Double CaMV 35S promoter/PR-O expression cassette (sense and anti-sense orientation))

The full-length cDNA sequence for PR-Q is contained on two different plasmids, pBScht15 contains the 3' end of the cDNA and pBScht5'-4 contains the 5' end of the cDNA. Because of this situation, the plasmids pCIB1022 and pCIB1023 are constructed in a three way ligation and differ by their orientation in the pCGN1761 vector. pCGN1761 is digested with EcoRI, treated with CIAP and purified on a 0.5 % LGT agarose gel. pBScht15 (see above) is digested with NsiI and EcoRI and the fragments are separated on a 0.5 % LGT agarose gel. PCR using pBScht5'-4 (see above) as a template and oligonucleotides 
(1) 5'CTATGAATGCATCATAAGTG 3' and
(2) 5' GCGGAATTCAAAAAAAAAAAAAAAACATAAG 3'
 as primers is performed to amplify the 5' end of the PR-Q cDNA. The PCR product is purified and digested with NsiI and EcoRI and the 210 bp product is purified from a 1.0 % LGT agarose gel. The purified pCGN1761 vector, the 810 bp NsiI/EcoRI fragment from pBScht15 and the NsiI/EcoRI digested PCR fragment are ligated and transformed as decribed above. Transformants are screened and selected which include the entire cDNA in either orientation. One plasmid which has the full-length PR-Q cDNA in a sense orientation relative to the double CaMV 35S promoter is designated as pCIB1022. A plasmid in which the full-length PR-Q cDNA is inserted in the anti-sense orientation relative to the promoter is designated pCIB1023.

### Example 31: Construction of pCIB 1024 and pCIB1025 (Double CaMV 35S promoter/PR-O' expression cassette (sense and anti-sense orientation)

The PR-O' cDNA cloned into pBSGL6e is truncated at the 5' end and is missing almost all of the complete signal sequence. This sequence is necessary for extracellular transport of the protein and should be replaced in order to engineer transgenic plants that secrete the protein. This example describes the subcloning of the PR-O' cDNA into the double CaMV 35S expression cassette in such a way that a signal peptide from PR-1a is added to the PR-O' protein.

This construction is carried out as a complicated three-way ligation. First a fusion of the PR-1a leader and signal peptide and the coding sequence of the mature PR-O' is made by a PCR gene fusion method. Then this piece is ligated along with the 3' end of the PR-O' cDNA into the pCGN1761 vector and transformants are selected with the insert in either orientation relative to the promoter.

A gene fusion technique based on PCR amplification has been developed by Ho et al. (1989). In this technique a gene fusion is made by creating two fragments with overlapping ends by PCR. In a subsequent reaction these two fragments are then fused also by PCR to generate a perfect fusion between the two molecules. This strategy is used to fuse the PR-1a signal peptide and leader to the PR-O' cDNA. Four oligonucleotides are synthesized with the following sequences:

GP50 - 5′ CCATAACAAACTCCTGCTTGGGCACGGCAAGAGTGGGATA 3′
GP51 - 5′ TATCCCACTCTTGCCGTGCCCAAGCAGGAGTTTGTTATGG 3′
GP52 - 5′ GATCGAATTCATTCAAGATACAACATTTCT 3′
GP53 - 5′ CATTCTCAAGGTCCGG 3′.

The GP50 and GP51 oligonucleotides are complementary to each other and contain the DNA sequence desired for the fusion between the PR-1a leader and signal and the PR-O' mature coding sequence. This is diagramed below:

GP51 5' TATCCCACTCTTGCCGTGCCCAAGCAGGAGTTTGTTATGG 3'
3' ATAGGGTGAGAACGGCACGGGTTCGTCCTCAAACAATACC 5' GP50
| PR-1A || PR-O' |

The oligonucleotide GP52 is the same sequence as the 5' end of the PR-1a cDNA and it contains on the 5' end a sequence encoding an EcoRI site.

GP52 - 5' GATCGAATTCATTCAAGATACAACATTTCT 3'
|EcoRI| PR-1a |

The oligonucleotide GP53 serves as a primer and is complementary to positions 180 to 195 of the PR-O' sequence, sequence 7.

In order to fuse the two pieces of DNA two PCRs are set up. One uses the plasmid pBSPR1-207 as a template and the two primers GP52 and GP50; the other uses pBSGL6e as a template and the primers GP51 and GP53. The PCR products are analyzed by gel electrophoresis. The PCR products are then purified and an aliquot of each is used in a second stage PCR. In this reaction the templates are both of the products from the first two reactions and the primers are GP52 and GP53. A modified PCR is established such that in the first round of synthesis the DNA templates are added without the primers and the templates are heated and allowed to cool and then extended at 65°C. The two primers are then added and the PCR is carried out normally. An aliquot of the PCR products is analyzed by gel electrophoresis. The remaining DNA is then purified and digested with SacI and EcoRI and the digest is electrophoresed on a 1.5 % LGT agarose gel. The band corresponding to the correct PCR product is excised and used for ligation.

The plasmid pBSGL6e is digested with both SacI and EcoRI and the digest is electrophoresed on a 0.5 % LGT agarose gel. The 1.0 kb band containing the large PR-O' fragment is excised and ligated with the SacI-EcoRI PCR product from above and the plasmid pCGN1761 which has been digested with EcoRI and CIAP and purified on a 0.5 % LGT agarose gel. The fragments are ligated and transformed as described above. Transformants are screened for the correct construct. A plasmid in which the PR-1a leader and signal has been fused to the PR-O' mature coding sequence and is in the sense orientation relative to the promoter is designated pCIB 1024; this construct is confirmed by DNA sequencing. A plasmid with the correct fusion but in the opposite orientation relative to the promoter is designated pCIB1025. This construct is also verified by DNA sequencing.

### Example 31A: Construction of pCIB1024A and pCIB1025A (Double 35S promoter/PR-O' expression cassette (sense and anti-sense orientation)

The plasmid pBSGL5B-12, which contains a full-length cDNA encoding the PR-O' protein is digested with EcoRI and the fragments are separated on a 0.5 % LGT agarose gel. The 1.2 kb band is excised and ligated with pCGN1761 which is digested with EcoRI, treated with CIAP and purified on a 0.5 % LGT agarose gel as described above. The ligation mixture is transformed as described and transformants are screened for a clone containing the PR-O' cDNA in either orientation. One plasmid, in which the cDNA is inserted in a sense orientation relative to the double CaMV 35S promoter, is designated pCIB1024. A plasmid in which the cDNA is inserted in an anti-sense orientation relative to the promoter is designated as pCIB1025A.

### Example 31B: Construction of pCIB1032 and pCIB1033 (Double 35S promoter/PR-2 expression cassette (sense and anti-sense orientation)

The plasmid pBSGL117 contains a full-length cDNA encoding the PR-2 protein. The PR-2 cDNA from pBSGL117 is subcloned into the pCGN1761 expression plasmid in either orientation to create pCIB1032 and pCIB1033. However, the cDNA contains an internal EcoRI site and so the cDNA has to be excised by a partial EcoRI digest.

The plasmid pBSGL117 is digested with EcoRI under conditions in which a partial digest results. The digestion products are separated on a 0.5 % LGT agarose gel and the 1.2 kb band containing the full-length cDNA for PR-2 is excised and ligated to pCGN1761 which has been digested with EcoRI, treated with CIAP and purified on a 0.5 % LGT agarose gel. The ligation and transformation is carried out as previously described. Positive transformants are isolated and screened for the presence of the large PR-2 cDNA fragment inserted in either orientation. One plasmid, with the PR-2 cDNA subcloned in a sense orientation relative to the transcriptional start site is designated as pCIB1032 and a plasmid with the fragment in an anti-sense orientation is designated as pCIB1033. The structure of these constructs can be verified by DNA sequencing.

### Example 31C: Construction of pCIB1034 and pCIB1035 (Double 35S promoter/PR-2 expression cassette (sense and anti-sense orientation)

A plasmid containing a full-length cDNA encoding the PR-N protein is used as a source to subclone the PR-N cDNA into the pCGN1761 expression plasmid in either orientation. The resulting plasmids are designated as pCIB1034 and pCIB1035. However, the cDNA contains an internal EcoRI site and so the cDNA has to be excised by a partial digest.

The plasmid containing the full-length PR-N cDNA is digested with EcoRI under conditions in which a partial digest results. The digestion products are separated on a 0.5 % LGT agarose gel and the 1.2 kb band containing the full-length cDNA for PR-N is excised and ligated to pCGN1761 which has been digested with EcoRI, treated with CIAP and purified on a 0.5 % LGT agarose gel. The ligation and transformation is carried out as previously described.

Positive transformants are isolated and screened for the presence of the large PR-N cDNA fragment inserted in either orientation. One plasmid, with the PR-N cDNA subcloned in a sense orientation relative to the transcriptional start site is designated as pCIB1034 and a plasmid with the fragment in an anti-sense orientation is designated as pCIB1035. The structure of these constructs is verified by DNA sequencing.

### Example 31D: Construction of pCIB1036 and pCIB1037 (Double 35S promoter/PR-O expression cassette (sense and anti-sense orientation)

A plasmid containing a full-length cDNA encoding the PR-O protein is used as a source to subclone the PR-O cDNA into the pCGN1761 expression plasmid in either orientation. The resulting plasmids are designated as pCIB1036 and pCIB1037. However, the cDNA contains an internal EcoRI site and so the cDNA has to be excised by a partial EcoRI digest.

The plasmid containing the full-length PR-O cDNA is digested with EcoRI under conditions in which a partial digest results. The digestion products are separated on a 0.5 % LGT agarose gel and the 1.2 kb band containing the full-length cDNA for PR-O is excised and ligated to pCGN1761 which has been digested with EcoRI, treated with CIAP and purified on a 0.5 % LGT agarose gel. The ligation and transformation is carried out as previously described. Positive transformants are isolated and screened for the presence of the large PR-O cDNA fragment inserted in either orientation. One plasmid, with the PR-O cDNA subcloned in a sense orientation relative to the transcriptional start site is designated as pCIB1036 and a plasmid with the fragment in an anti-sense orientation is designated as pCIB1037. The structure of these constructs is verified by DNA sequencing.

### Example 31E: Construction of pCIB1038 and pCIB1039 (Double 35S promoter/PR-2' expression cassette (sense and anti-sense orientation)

A plasmid (pBSGL135 from Example 18B) containing a full-length cDNA encoding the PR-2' protein is used as a source to subclone the PR-2' cDNA into the pCGN1761 expression plasmid in either orientation. The resulting plasmids are designated as pCIB1038 and pCIB1039. However, the cDNA contains an internal EcoRI site and so the cDNA has to be excised by a partial EcoRI digest.

The plasmid containing the full-length PR-2' cDNA is digested with EcoRI under conditions in which a partial digest results. The digestion products are separated on a 0.5 % LGT agarose gel and the 1.2 kb band containing the full-length cDNA for PR-2' is excised and ligated to pCGN1761 which has been digested with EcoRI, treated with CIAP and purified on a 0.5 % LGT agarose gel.

The ligation and transformation is carried out as previously described. Positive transformants are isolated and screened for the presence of the large PR-2' cDNA fragment inserted in either orientation. One plasmid, with the PR-2' cDNA subcloned in a sense orientation relative to the transcriptional start site is designated as pCIB1036 and a plasmid with the fragment in an anti-sense orientation is designated as pCIB1039. The structure of these constructs is verified by DNA sequencing.

### Example 32: Construction of pCIB1005B and pCIB1006B (Double CaMV 35S promoter/basic glucanase expression cassette (sense and anti-sense - orientation)

The plasmid pGLN17 is a hybrid cDNA encoding the basic β-1,3-glucanase from *N. tabacum* (Shinshi et al., 1988) constructed by fusing the 5' end of the pGL31 clone and the 3' end of the pGL36 clone. The sequence encoded in this hybrid cDNA is as follows:

### Sequence 13: cDNA sequence of the basic β-1,3-glucanase cDNA hybrid cloned into the plasmid pGLN17

10 20 30 40 50 60
CCTCAAATGGCTGCTATCACACTCCTAGGATTACTACTTGTTGCCAGCAGCATTGACATA
M A A I T L L G L L L V A S S I D I
(N) 80 90 100 110 120
|
GCAGGGGCTCAATCGATAGGTGTTTGCTATGGAATGCTAGGCAACAACTTGCCAAATCAT
A G A Q S I G V C Y G M L G N N L P N H
130 140 150 160 170 180
TGGGAAGTTATACAGCTCTACAAGTCAAGAAACATAGGAAGACTGAGGCTTTATGATCCA
W E V I Q L Y K S R N I G R L R L Y D P
190 200 210 220 230 240
AATCATGGAGCTTTACAAGCATTAAAAGGCTCAAATATTGAAGTTATGTTAGGACTTCCC
N H G A L Q A L K G S N I E V M L G L P
250 260 270 280 290 300
AATTCAGATGTGAAGCACATTGCTTCCGGAATGGAACATGCAAGATGGTGGGTACAGAAA
N S D V K H I A S G M E H A R W W V Q K
310 320 330 340 350 360
AATGTTAAAGATTTCTGGCCAGATGTTAAGATTAAGTATATTGCTGTTGGGAATGAAATC
N V K D F W P D V K I K Y I A V G N E I
370 380 390 400 410 420
AGCCCTGTCACTGGCACATCTTACCTAACCTCATTTCTTACTCCTGCTATGGTAAATATT
S P V T G T S Y L T S F L T P A M V N I
430 440 450 460 470 480
TACAAAGCAATTGGTGAAGCTGGTTTGGGAAACAACATCAAGGTCTCAACTTCTGTAGAC
Y K A I G E A G L G N N I K V S T S V D
490 500 510 520 530 540
ATGACCTTGATTGGAAACTCTTATCCACCATCACAGGGTTCGTTTAGGAACGATGCTAGG
M T L I G N S Y P P S Q G S F R N D A R
550 560 570 580 590 600
TGGTTTACTGATCCAATTGTTGGGTTCTTAAGGGACACACGTGCACCTTTACTCGTTAAC
W F T D P I V G F L R D T R A P L L V N

610 620 630 640 650 660

ATTTACCCCTATTTCAGCTATTCTGGTAATCCAGGGCAGATTTCTCTCCCCTATTCTCTT
I Y P Y F S Y S G N P G Q I S L P Y S L

670 680 690 700 710 720

TTTACAGCACCAAATGTGGTAGTACAAGATGGTTCACGCCAATATAGGAACTTATTTGAT
F T A P N V V V Q D G S R Q Y R N L F D

730 740 750 760 770 780

GCAATGCTGGATTCTGTGTATGCTGCCCTCGAGCGATCAGGAGGGGCATCTGTAGGGATT
A M L D S V Y A A L E R S G G A S V G I

790 800 810 820 830 840

GTTGTGTCCGAGAGTGGCTGGCCATCTGCTGGTGCATTTGGAGCCACATATGACAATGCA
V V S E S G W P S A G A F G A T Y D N A

850 860 870 880 890 900

GCAACTTACTTGAGGAACTTAATTCAACACGCTAAAGAGGGTAGCCCAAGAAAGCCTGGA
A T Y L R N L I Q H A K E G S P R K P G

910 920 930 940 950 960

CCTATTGAGACCTATATATTTGCCATGTTTGATGAGAACAACAAGAACCCTGAACTGGAG
P I E T Y I F A M F D E N N K N P E L E

970 980 990 1000 1010 (C)
|
AAACATTTTGGATTGTTTTCCCCCAACAAGCAGCCCAAATATAATATCAACTTTGGGGTC
K H F G L F S P N K Q P K Y N I N F G V

1030 1040 1050 (N-glyc) 1070 1080
|
TCTGGTGGAGTTTGGGACAGTTCAGTTGAAACTAATGCTACTGCTTCTCTCGTAAGTGAG
S G G V W D S S V E T N A T A S L V S E

1090 1100 1110 1120 1130 1140

ATGTGAGCTGATGAGACACTTGAAATCTCTTTACATACGTATTCCTTGGATGGAAAACCT
M

1150 1160 1170 1180 1190 1200

AGTAAAAACAAGAGAAATTTTTCTTTATGCAAGATACTAAATAACATTGCATGTCTCTG

1210 1220 1230 1240 1250 1260

TAAGTCCTCATGGATTGTTATCCAGTGACGATGCAACTCTGAGTGGTTTAAATTCCTTT

1270 1280 1290 1300 1310 1320

TCTTTGTGATATTGGTAATTTGGCAAGAAACTTTCTGTAAGTTTGTGAATTTCATGCATC

1330 1340 1350 1360

ATTAATTATACATCAGTTCCATGTTTGATCAAAAAAAA

This cDNA is truncated at the 5' end and does not encode the entire signal peptide. In order to make transgenic plants in which this protein is properly targeted (i.e., the central vacuole), it is necessary to add this sequence back on to the truncated cDNA. Therefore, the double CaMV 35S expression cassette is constructed in a two step process. In the first step the signal peptide of the cDNA is replaced by a signal peptide encoded in the genomic clone. In the second step, this "repaired cDNA" is moved into the expression vector.

The plasmid pSGL2 is a subclone of the pGLN17 cDNA. This plasmid is digested with ClaI and EcoRI and the 1 kb fragment containing the glucanase cDNA is isolated from a LGT agarose gel. The pBluescript plasmid is digested with EcoRI, treated with CIAP and purified on a LGT agarose gel.

The plasmid pBS-Gluc 39.1 contains a 4.4 kb insert which includes the glucanase coding sequence, about 1.5 kb of 5' flanking sequence, a 600 bp intron, and about 1 kb of 3' flanking sequence. This plasmid is used as a template in a PCR experiment containing the following two primers:

A. 5' CATCTGAATTCTCCCAACAAGTCTTCCC 3'
B. 5' AACACCTATCGATTGAGCCCCTGCTATGTCAATGCTGGTGGC 3'

The result of this amplification is to produce a fragment to replace the truncated 5' end of the glucanase cDNA. A single-base mutation creating an EcoRI site is introduced to facilitate cloning experiments. The PCR product is digested with EcoRI and ClaI and fragments are separated on a 2.0 % LGT agarose gel. A 120 bp band is excised, mixed with the 1 kb ClaI-EcoRI fragment from pSGL2 and the purified, EcoRI digested bluescript vector, ligated and transformed as described above. Transformants are screened for the presence of the insert and one plasmid with the proper structure is designated pCIB 1009.

The plasmid pCIB1009 is digested with EcoRI and the 1.2 kb fragment is purified on a LGT agarose gel. The plasmid pCGN1761 is digested with EcoRI, treated with CIAP, purified on a LGT agarose gel, mixed with the 1.2 kb EcoRI fragment, and then ligated and transformed. Transformants are screened for the presence of the insert. One plasmid, in which the glucanase cDNA is in a sense orientation relative to the CaMV promoter is designated as pCIB1005B. Another plasmid, with the cDNA insert in an anti-sense orientation is designated pCIB1006B.

### Example 33: Construction of pCIB1007 and pCIB1008 (Double CaMV 35S promoter/basic chitinase expression cassette (sense and anti-sense orientation))

The plasmid pSCH10 contains a cDNA insert of the tobacco basic chitinase which is similar to the insert in pCHN50 (Shinshi, H. et al, 1987) but with an extension of 81 bp on the 5' end. The 80 extra bp are:

5'GGATCCGTTTGCATTTCACCAGTTTACTACTACATTAAAATGAGGCTTTG
- |        |         |         |         |         |
  1        10        20        30        40        50

5′ TAAATTCACAGCTCTCTCTTCTCTACTATTT 3′
|          |          |
60         70         80

pSCH10 is digested with BamHI and then ligated with a molecular adaptor with the sequence 5'GATCCGGAATTCCG 3' as described in Example 5. The ligation product is then purified and digested with EcoRI and the 1.2 kb fragment containing the adapted chitinase cDNA is purified from a LGT agarose gel.

This fragment is mixed with EcoRI digested, CIAP treated pCGN1761 which is also purified from a LGT agarose gel and the mixture is ligated and transformed.
Transformants are screened for the chitinase cDNA insert and one plasmid which contains the chitinase cDNA in a sense orientation relative to the CaMV promoter is designated as pCIB1007.
A plasmid with the chitinase cDNA in an anti-sense orientation with respect to the promoter is designated as pCIB1008.

### Example 34: Construction of pCGN1788A and pCGN1788B (Double CaMV 35S Promoter/SAR8.2 expression cassette (sense and anti-sense orientation))

The pSAR8.2a cDNA (see above) is subcloned into the double CaMV 35S promoter/3'*tm-l* terminator cassette pCGN1431 (see above) using a PCR amplification method. Four oligonucleotides, two for each of the sense and anti-sense constructions and each one 33 nucleotides in length, are synthesized for use as primers to generate the cDNA sequence of pSAR8.2a by PCR using the plasmid pSAR8.2a as template. The primers contain additional sequences at their 5' ends that generate new restriction sites upon completion of PCR amplification.

For the sense construction the sequence of oligonucleotide 1167 is 5'-GTGACCGAGCTCAAAGAAAAATACAGTACAATA-3' which generates a SstI site proximal to the 3' end of the cDNA sequence. The sequence of oligonucleotide 1168 is 5' ACCGTGGGATCCACAGTAAAAAACTGAAACTCC-3' and generates a BamHI site proximal to the 5' end of the cDNA. For the anti-sense construction, the sequence of oligonucleotide 1224 is 5'-GTGACCGGATCCAAAGAAAAATACAGTACAATA-3' which generates a BamHI site proximal to the 3' end of the cDNA. The sequence of oligonucleotide 1225 is 5'-ACCGTGGAGCTCACAGTAAAAAACTGAAACTCC-3' and generates an SstI site proximal to the 5' end of the cDNA.

Oligonucleotide 1167 and 1168 are used in a PCR in which the plasmid pSAR8.2a serves as a DNA template. The purified PCR product generated in this reaction is digested with SstI and BamHI and cloned into pCGN1431 which is digested with SstI and BamHI. The DNA is transformed and plasmids are screened for the presence of the pSAR8.2a cDNA insert in a sense orientation relative to the double CaMV 35S promoter. Putative plasmids are then subjected to DNA sequencing and one, which has the proper orientation and contains no introduced mutations, is designated pCGN1788A.

For the anti-sense construct, oligonucleotides 1224 and 1225 are used in a PCR as decribed above. After digestion with SstI and BamHI the DNA is cloned into SstI and BamHI digested pCGN1431. Putative positive plasmids are screened for the insertion of the cDNA in an anti-sense orientation relative to the promoter and the constructs are verified by sequencing the entire cDNA. One plasmid, in which the cDNA is inserted in the correct orientation and the DNA sequence is correct, is designated pCGN1788B.

### Example 35: Construction of pCIB1000 and pCIB1001 (Double CaMV 35S promoter/cucumber chitinase/lysozyme expression cassette (sense and anti-sense orientation))

The plasmid, pBScuccht5 (also called pBScucchi/chitinase) is digested with EcoRI and the fragments separated on a 0.5 % LGT agarose gel. The 1.2 kb band is excised and ligated with pCGN1761 which has been digested with EcoRI, treated with CIAP and purified on a 0.5 % LGT agarose gel as described above. The ligation mixture is transformed as described and transformants are screened for containing the chitinase/lysozyme cDNA in either orientation. One plasmid, in which the cDNA is inserted in a sense orientation relative to the double CaMV 35S promoter, is designated pCIB1000. A plasmid in which the cDNA is inserted in an anti-sense orientation relative to the double CaMV 35S promoter is designated pCIB1001.

### SECTION 5. VECTORS

This section details the construction of binary vectors containing all of the chimeric genes. The first section explains the development of the binary vectors to be used and the second section details the subcloning of the expression cassette into the binary vector.

### Section 5A: Construction of Binary Vectors

### Example 36: Construction of pCGN783

pCGN783 is a binary plasmid containing the left and right T-DNA borders of *A. tumefaciens* octopine Ti-plasmid pTiA6 (Currier and Nester, 1976) the gentamycin resistance gene of pPh1JI (Hirsch and Beringer, 1984), the 35S promoter of CaMV (Gardner et al., 1981), the kanamycin resistance gene of Tn5 (Jorgensen et al., 1979), and the 3' region from transcript 7 of pTiA6 (Currier and Nester, 1976). The vector is constructed in a multi-step process detailed below.

A. Construction of pCGN739. To obtain the gentamycin resistance marker, the resistance gene is isolated from a 3.1 kb EcoRI-PstI fragment of pPhIJI (Hirsch and Beringer, 1984) and cloned into pUC9 (Vieira and Messing, 1982), yielding pCGN549. The pCGN549 HindIII-BamHI fragment containing the gentamycin resistance gene replaces the HindIII-BglII fragment of pCGN587 (for above) constructing pCGN594. The pCGN594 HindIII-BamHI region which contains an *ocs*-kanamycin-*ocs* fragment is replaced with the HindIII-BamHI polylinker region from pUC18 (Yanisch-Perron et al., 1985) to make pCGN739.

B. Construction of pCGN726C. pCGN566 contains the EcoRI-HindIII linker of pUC18 (Yanisch-Perron et al., 1985), inserted into the EcoRI-HindIII sites of pUC13-cm [K. Buckley, Ph.D. Thesis, UC San Diego 1985]. The HindIII-BglII fragment of pNW31c-8, 29-1 (Thomashow et al., 1980) containing ORF1 and 2 (Barker et al., 1983) is subcloned into the HindIII-BamHI sites of pCGN566 producing pCGN703. The Sau3A fragment of pCGN703 containing the 3' region of transcript 7 from pTiA6 (corresponding to bases 2396-2920 of pTi15955 (Barker et al., 1983) is subcloned into the BamHI site of pUC18 (Yanisch-Perron al., 1985) producing pCGN709. The EcoRI-SmaI polylinker region of pCGN709 is replaced with the EcoRI-SmaI fragment from pCGN587 (for production see infra) which contains the kanamycin resistance gene (APH3 'II) producing pCGN726. The EcoRI-SalI fragment of pCGN726 plus the BglII-EcoRI fragment of pCGN734 are inserted into the BamHI-SalI sites of pUC8-pUC13-cm [chloramphenicol resistant, K. Buckley, PH.D. Thesis, UC San Diego 1985] producing pCGN738. To construct pCGN734, the HindIII-SphI fragment of pTiA6 corresponding to bases 3390-3241 (Barker et al., 1983) is cloned into the HindIII-SphI site of M13mp19 (Yanisch-Perron et al., 1985: Norrander et al., 1983). Using an oligonucleotide corresponding to bases 3287 to 3300, DNA synthesis is primed from this template. Following S1 nuclease treatment and HindIII digestion, the resulting fragment is cloned in the HindIII-SmaI site of pUC19 (Yanisch-Perron et al., 1985). The resulting EcoRI-HindIII fragment corresponding to bases 3287-3390 (Barker et al., 1983), is cloned with the EcoRI-HindIII fragment of pTiA6 (corresponding to bases 3390-4494) into the EcoRI site of pUC8 (Vieira and Messing, 1982) resulting in pCGN374. pCGN726c is derived from pCGN738 by deleting the 900 bp EcoRI-EcoRI fragment.

C. Construction of pCGN766C. The HindIII-BamHI fragment of pCGN167 (see infra) containing the CaMV-35S promoter, 1 ATG-kanamycin gene and the BamHI fragment 19 of pTiA6 is cloned into the BamHI-HindIII sites of pUC19 (Norrander et al., 1983; Yanisch-Perron et al., 1985) constructing pCGN976. The 35S promoter and 3' region from transcript 7 is developed by inserting a 0.7 kb HindIII-EcoRI fragment of pCGN976 (35S promoter) and the 0.5kb EcoRI-SalI fragment of PCGN709 (transcript 7:3') into the HindIII-SalI sites of pCGN566 constructing pCGN766c. To construct pCGN167, the AluI fragment of CaMV (bp 7144-7735) (Gardner et al., 1981) is obtained by digestion with AluI and cloned into the HincII site of M13mp7 (Vieira and Messing, 1982) to create C614. An EcoRI digest of C614 produces the EcoRI fragment from C614 containing the 35S promoter which is cloned into the EcoRI site of pIC8 (Vieira and Messing, 1982) to produce pCGN146. To trim the promoter region, the BglII site (bp 7670) is treated with BglII and Bal31 and subsequently a BglII linker is attached to the Bal31 treated DNA to produce pCGN147. pCGN148a containing a promoter region, selectable marker (KAN with 2 ATG's) and 3' region is prepared by digesting pCGN528 (see below) with BglII and inserting the BamHI-BglII promoter fragment from pCGN147. This fragment is cloned into the BglII site of pCGN528 so that the BglII site is proximal to the kanamycin gene of pCGN528. The shuttle vector used for this construct, pCGN528, is made as follows. pCGN525 is made by digesting a plasmid containing Tn5 which harbors a kanamycin gene (Jorgenson et al., 1979) with HindIII-BamHI and inserting the HindIII-BamHI fragment containing the kanamycin gene into the HindIII-BamHI sites in the tetracycline gene of pACYC184 (Chang and Cohen, 1978). pCGN526 is made by inserting the BamHI fragment 19 of pTiA6 (Thomashow et al., 1980) into the BamHI site of pCGN525. pCGN528 is obtained by deleting the small XhoI fragment from pCGN526 by digesting with XhoI and religating. pCGN149a is made by cloning the BamHI kanamycin gene fragment from 9MB9KanXXI into the BamHI site of pCGN148a. pMB9KanXXI is a pUC4k variant (Vieira and Messing, 1982) which has the XhoI site missing but containing a functional kanamycin gene from Tn903 to allow for efficient selection in *Agrobacterium.* pCGN149a is digested with BglII and SphI. This small BglII-SphI fragment of pCGN149A is replaced with the BamHI-SphI fragment from MI (see below) isolated by digestion with BamHI and SphI. This produces pCGN167, a construct containing a full length CaMV promoter, 1 ATG-Kanamycin gene, 3' end and the bacterial Tn903-type kanamycin gene. MI is an EcoRI fragment from pCGN550 (see construction of pCGN587) and is cloned into the EcoRI cloning site of M13mp9 in such a way that the PstI site in the 1ATG-kanamycin gene is proximal to the polylinker region of M13mp9.

D. Construction of pCGN451. pCGN451 contains the *ocs*5'-*ocs*3' cassette cloned into a derivative of pUC8 (Vieira and Messing, 1982). The modified vector is derived by digesting pUC8 with HincII and ligating in the presence of synthetic linker DNA, creating pCGN416, and then deleting the EcoRI site of pCGN416 by EcoRI digestion followed by treatment with Klenow enzyme and self ligation to create pCGN426. The *ocs*5'*-ocs*3' cassette is constructed by a series of steps from DNA derived from the octopine Ti-plasmid pTiA6 (Currier and Nester, 1976). An EcoRI fragment of pTiA6 (bp 13362-16202; the numbering is by Barker et al. (1983) for the closely related Ti plasmid pTi 15955) is removed from pVK232 (Knauf and Nester, 1982) by EcoRI digestion and cloned into EcoRI digested pACYC184 (Chang and Cohen, 1978) to generate pCGN15. The 2.4 kb BamHI-EcoRI fragment (bp 13774-16202) of pCGN15 is cloned into EcoRI-BamHI digested pBR322 (Bolivar et al., 1977) to yield pCGN429. The 412 bp EcoRI-BamHI fragment (bp13362-13774) of pCGN15 is cloned into EcoRI-BamHI digested pBR3322 (Bolivar et al., 1977) to yield pCGN407. The cut-down promoter fragment is obtained by digesting pCGN407 with XmnI (bp 13512), followed by resection with Bal31 exonuclease, ligation of synthetic EcoRI linkers, and digestion with BamHI. Resulting fragments of approximately 130 bp are gel purified and cloned into M13mp9 (Vieira and Messing, 1982) and sequenced. A clone, I-4, in which the EcoRI linker has been inserted at bp 13642 between the transcription initiation point and the translation initiation codon is identified by comparison with the sequence of de Greve et al. (1982); the EcoRI cleavage site is at position 13639, downstream from the mRNA start site. The 141 bp EcoRI-BamHI fragment of I-4, containing the cut-down promoter, is cloned into EcoRI-BamHI digested pBR322 (Bolivar et al., 1977) to create pCGN428. The 141 bp EcoRI-BamHI promoter piece from pCGN428, and the 2.5 kb EcoRI-BamHI *ocs* 5' piece from pCGN429 are cloned together into EcoRI digested pUC9 (Vieira and Messing, 1982) to generate pCGN442, reconstructing the *ocs* upstream region with a cut-down promoter section. The HindIII fragment of pLB41 [D. Figurski] containing the gentamycin resistance gene is cloned into HindIII digested pACYC184 (Chang and Cohen, 1978) to create pcDNA413b. The 4.7 kb BamHI fragment of pTiA6 (Currier and Nester, 1976) containing the *ocs* 3' region, is cloned into BamHI digested pBR325 (Bolivar, 1978) to create 33c-19. The SmaI site at position 11207 of 33c-19 is converted to an XhoI site using synthetic XhoI linker DNA, generating pCGN401.2. The 3.8 kb BamHI-EcoRI fragment of pCGN401.2 is cloned into BamHI-EcoRI digested pCGN413b to create pCGN419. The *ocs*5'-*ocs*3' cassette is generated by cloning the 2.64 kb EcoRI fragment of pCGN442, containing the 5' region, into EcoRI digested pCGN419 to create pCGN446. The 3.1 kb Xhol fragment of pCGN446, having the *ocs* 5' region (bp13639-15208) and *ocs* 3' region (bp 11207-12823), is cloned into the XhoI site of pCGN426 to create pCGN451.

E. Construction of pCGN587. The HindIII-SmaI fragment of Tn5 containing the entire structural gene for APH3'II (Jorgensen et al., 1979) is cloned into pUC8 (Vieira and Messing, 1982), this converts the fragment into a HindIII-EcoRI fragment, since there is an EcoRI site immediately adjacent to the SmaI site. The PstI-EcoRI fragment of pCGN300, containing the 3'-portion of the APH3'II gene, is then combined with an EcoRI-BamHI-SalI-PstI linker into the EcoRI site of pUC7 to make pCGN546W. An ATG codon is upstream from and out of reading frame with the ATG initiation codon of APH3'II. The undesired ATG is avoided by inserting a Sau3A-PstI fragment from the 5'-end of APH3'II, which fragment lacks the superfluous ATG, into the BamHI-PstI site of pCGN546W to provide plasmid pCGN550. The EcoRI fragment of pCGN550 containing the APH3'II gene is then cloned into the EcoRI site of pUS8-pIC13-cm [K. Buckley (1985), supra] to give pCGN551. The plasmid pCGN451 (described above) having the *ocs* 5' and the *ocs 3'* in the proper orientation is digested with EcoRI and the EcoRI fragment from pCGN551 containing the intact kanamycin resistance gene inserted into the EcoRI site to provide pCGN552 having the kanamycin resistance gene in the proper orientation. This *ocs*/KAN gene is used to provide a selectable marker for the trans type binary vector pCGN587. The 5' portion of the engineered octopine synthase promoter cassette consists of pTiA6 DNA from the XhoI at bp 15208-13644 (Barker et al., 1983) which also contains the T-DNA boundary sequence (border) implicated in T-DNA transfer. In the plasmid pCGN587, the *ocs*/KAN gene from pCGN552 provides a selectable marker as well as the right border. The left boundary region is first cloned in M13mp9 as a HindIII-SmaI piece (pCGN502) (bp 602-2212) and recloned as a KpnI-EcoRI fragment in pCGN565 to provide pCGN580. pCGN565 is a cloning vector based on pUC-pUC13-Cm, [K. Buckley, Ph.D. Thesis, UC San Diego 1985] but containing pUC18 linkers (Yanisch-Perron et al., 1985) pCGN580 is linerarized with BamHI and used to replace the smaller BglI fragment of pVCK102 (Knauf and Nester, 1982) creating pCGN585. By replacing the smaller SalI fragment of PCGN585 with the XhoI fragment from pCGN552 containing the *ocs*/KAN gene, pCGN587 is obtained.

F. Final construction of pCGN783. The 0.7 kb HindIII-EcoRI fragment of pCGN766c (CaMV-35S promoter) is ligated to the 1.5 kb EcoRI-SalI fragment of pCGN726c (2 ATG-KAN-3' region) into the HindIII-SalI sites of pUC119 [Sambrook J. et al, 1989) to produce pCGN778. The 2.2 kb region of pCGN778, HindIII-SalI fragment containing the CaMV 35S promoter (1-ATG-KAN-3' region) replaces the HindIII-SalI polylinker region of pCGN739 to produce pCGN783.

### Example 37: Construction of pCGN1539 and pCGN1540

pCGN1539 and pCGN1540 are binary plant transformation vectors containing the left and right T-DNA borders of *A. tumefaciens* octopine Ti-plasmid pTiA6 (Currier and Nester, 1976), the gentamycin resistance gene of pPHiJI (Hirsch and Beringer, 1984), an *A. rhizogenes* Ri plasmid origin of replication from pLJB11 (Jouanin et al, 1985), the *mas* promoter region an *mas* 3' *region* of pTiA6 with the kanamycin resistance gene of Tn5 (Jorgensen et al., 1979) a ColEl origin of replication from pBR322 (Bolivar et al., 1977), and a *lac*Z' screenable marker gene from pUC18 (Norrander et al., 1983). The backbone of pCGN1539-1540, containing the gentamycin resistance gene and the Ri and ColEl origins, is derived from pCGN1532 (see below). The Ti borders and plant selectable marker gene (*mas* 5'-*kan-mas*3'), are from pCGN1537; the plant selectable marker cassette is in turn taken from pCGN1536, while the right border and the *lac*Z' fragments are derived from pCGN565RBx2X, and the left border is derived from pCGN65.

A. pCGN1532 construction. The 3.5 kb EcoRI-PstI fragment containing the gentamycin resistance gene is removed from pPhUI (Hirsch and Beringer, 1984) by EcoRI-PstI digestion and cloned into EcoRI-PstI digested pUC9 (Vieira and Messing, 1982) to generate pCGN549. HindIII-PstI digestion of pCGN549 yields a 3.1 kb fragment bearing the gentamycin resistance gene, which is made blunt ended by the Klenow fragment of DNA polymerase I and cloned into PvuII digested pBR322 (Bolivar et al., 1977) to create pBR322Gm. pBR322Gm is digested with DraI and SphI, treated with Klenow enzyme to create blunt ends, and the 2.8 kb fragment cloned into the Ri origin containing plasmid pLJbB11 (Jouanin et al., 1985) which has been digested with ApaI and made blunt ended with Klenow enzyme, creating pLHbB11Gm. The extra ColEl origin and the kanamycin resistance gene are deleted from pLHbB11GM by digestion with BamHI followed by self closure to create pGMB11. The HindII site of pGmB11 is deleted by HindII digestion followed by treatment with Klenow enzyme and self closure, creating pGmB11-H. The PstI site of pGmB11-H is deleted by PstI digestion followed by treatment with Klenow enzyme and self closure, creating pCGN1532.

B. pCGN1536 construction. The 5.4 kb EcoRI fragment is removed from pVK232 (Knauf and Nester, 1982) by EcoRI digestion and cloned into EcoRI digested pACYC184 (Chang and Cohen, 1978) to create pCGN14. The 1434 bp ClaI-SphI fragment of pCGN14, containing the *mas* 5' region (bp20128-21562 according to numbering of Barker et al., 1983) is cloned into AccI-SphI digested pUC19 (Yanisch-Perron et al., 1985) to generate pCGN50. A 746 bp EcoRV-NaeI fragment of the *mas* 5' region is replaced by an XhoI site by digesting pCGN40 with EcoRV and Nael followed by ligation in the presence of a synthetic Xho I linker DNA to create pCGN1036. The 765 bp SstI-HindIII fragment (bp 18474-19239) of pCGN14, containing the *mas* 3' region, is cloned into SstI-HindIII digested pUC18 (Norrander et al., 1983) to yield pCGN43. The HindIII site of pCGN43 is replaced with an EcoRI site by digestion with HindIII, blunt ending with Klenow enzyme, and ligation of synthetic EcoRI linker DNA to create pCGN1034. The 767 bp EcoRI fragment of pCGN 1034 is cloned into EcoRI-digested pCGN1036 in the orientation that places bp 19239 of the *mas* 3' region proximal to the *mas* 5' region to create pCGN1040. pCGN1040 is subjected to partial digestion with SstI, treated with T4 DNA polymerase to create blunt ends, and ligated in the presence of synthetic XhoI linker DNA; a clone is selected in which only the SstI site at the junction of bp 18474 and vector DNA (constructed in pCGN43 and carried into pCGN1040) is replaced by an XhoI site to generate pCGN1047. pCGN565 [see above] is digested with EcoRI and HindIII, treated with Klenow enzyme to create blunt ends, and ligated in the presence of synthetic Xhol linker DNA to create pCGN1003; this recreates the EcoRI site adjacent to the XhoI linker. pCGN1003 is digested with EcoRI, treated with Klenow enzyme to create blunt ends, and ligated in the presence of synthetic PstI linker DNA to create pCGN1007. The 1.5 kb Xhol fragment of pCGN1047, containing the *mas* 5' region and the *mas* 3' region with a multiple cloning site between, is cloned into XhoI digested pCGN1007 to construct pCGN1052.

A portion of the multiple cloning site of pCGN1052 is deleted by digestion with XbaI and SstI, treated with Klenow enzyme to make blunt ends, and ligated to generate pCGN1052δXS. The 1 kb EcoRI-SmaI fragment of pCGN550 [pCGN783description], containing the 1 ATG-kanamycin resistance gene, is cloned into EcoRI-SmaI digested Bluescript M13-KS (Stratagene, Inc.) to create pBSKm; this plasmid contains an M13 region allowing generation of single stranded DNA. Single stranded DNA is generated according to the supplier's recommendations, and in vitro mutagenesis is performed (Adelman et al., 1983) using a synthetic oligonucleotide with the sequence 5'GAACTCCAGGACGAGGC3' to alter a PstI site within the kanamycin resistance gene and make it undigestable, creating pCGN1534. pCGN1534 is digested with SmaI and ligated in the presence of synthetic EcoRI linker DNA to generate pCGN1535. The 1 kb EcoRI fragment of pCGN1536 is cloned into EcoRI digested pCGN1052δXS to create the *mas*5'-*kan mas*3' plant selectable marker cassette pCGN1536.

C. pCGN565RAx2X construction. pCGN451 [pCGN783 description] is digested with HpaI and ligated in the presence of synthetic SphI linker DNA to generate pCGN55. The XhoI-SphI fragment of pCGN55 (bp13800-15208, including the right border, of *A. tumefaciens* T-DNA; Barker et al., 1977) is cloned into SalI-SphI digested pUC19 (Yanisch-Perron et al., 1985) to create pCGN60. The 1.4 kb HindIII-BamHI fragment of pCGN60 is cloned into HindIII-BamHI digested pSP64 (Promega, Inc.) to generate pCGN1039. pCGN1039 is digested with SmaI and NruI (deleting bp14273-15208; Barker et al., 1977) and ligated in the presence of synthetic BglII linker DNA creating pCGN1039δNS. The 0.47 kb EcoRI-HindIII fragment of pCGN1039δNS is cloned into Eco-RI-HindIII digested pCGN565 [described in pCFN783 description] to create pCGN565RB. The HindIII site of pCGN565RB is replaced with an XhoI site by HindIII digestion, treatment with Klenow enzyme, and ligation in the presence of synthetic XhoI linker DNA to create pCGN565RB-H+X. pUC18 (Norrander et al., 1983) is digested with HaeII to release the *lac*Z' fragment, treated with Klenow enzyme to create blunt ends, and the *lac*Z'-containing fragment ligated into pCGN565RB-H+X, which has been digested with AccI and SphI and treated with Klenow enzyme, in such an orientation that the *lac*Z' promoter is proximal to the right border fragment; this construct, pCGN565RBx2x is positive for *lac*Z' expression when plated on an appropriate host and contains bp 13990-14273 of the right border fragment (Barker et al., 1983) having deleted the AccI-SphI fragment (bp 13800-13990).

D. pCGN65 construction. pCGN501 is constructed by cloning a 1.85 kb EcoRI-XhoI fragment of pTiA6 (Currier and Nester, 1976) containing bases 13362-15208 (Barker et al., 1983) of the T-DNA (right border), into EcoRI-SalI digested M13mp9 (Vieira and Messing, 1982). PCGN502 is constructed by cloning a 1.6 kb HindIII-SmaI fragment of pTiA6, containing bases 602-2212 of the T-DNA (left border), into HindIII-SmaI digested M13mp9. pCGN501 and pCGN502 are both digested with EcoRI and HindIII and both T-DNA-containing fragments cloned together into HindIII digested pUC9 (Vieira and Messing, 1982) to yield pCGN503, containing both T-DNA border fragments. pCGN503 is digested with HindIII and EcoRI and the two resulting HindIII-EcoRI fragments (containing the T-DNA borders) are cloned into EcoRI digested pHC79 (Hohn and Collins, 1980) to generate pCGN518. The KpnI-EcoRI fragment from pCGN518, containing the left T-DNA border, is cloned into KpnI-EcoRI digested pCGN565 to generate pCGN580. The BamHI-BglII fragment of pCGN580 is cloned into the BamHI site of pACYC184 (Chang and Cohen, 1978) to create pCGN51. The 1.4 kb BamHI-SphI fragment of pCGN60 (see pCGN65x2X section above) containing the T-DNA right border fragment, is cloned into BamHI-SphI digested pCGN51 to create pCGN65.

E. pCGN1537 construction. pCGN65 is digested with KpnI and XbaI, treated with Klenow enzyme to create blunt ends, and ligated in the presence of synthetic BglII linker DNA to create pCGN65δKX. pCGN65δKX is digested with SalI, treated with Klenow enzyme to create blunt ends, and ligated in the presence of synthetic XhoI linker DNA to create pCGN65δKX-S+X. The 728 bp BglII-Xhol fragment of pCGNRBx2X, containing the T-DNA right border piece and the *lac*Z' gene, is cloned into BglII-XhoI digested pCGN65δKX-S+K, replacing pCGN65x2X. The ClaI fragment pCGN65x2X is deleted and replaced with an XhoI linker by digesting with ClaI, treated with Klenow enzyme to create blunt ends, and ligated in the presence of synthetic XhoI linker DNA to create pCGN65δ2XX. pCGN65δ2XX is digested with BglII and fused with BglII digested pCGN549 (see pCGN1532 section above) to create pCGN1530 which contains both plasmid backbones. pCGN1530 is digested with XhoI and religated, then a gentamycin-resistant chloramphenicol-sensitive clone is chosen which has deleted the pACYC184-derived backbone, creating pCGN1530A. The 2.43 kb XhoI fragment of pCGN1536, containing the *mas*5'-*kan-mas*3' cassette, is cloned into XhoI digested pCGN1530A to create pCGN1537.

F. Final assembly of pCGN1540. The BglII fragment of pCGN1537, containing the plant selectable marker gene and the *lac*Z' screenable marker gene (with multiple cloning site), all between the T-DNA borders, is cloned into BamHI digested pCGN1532. A clone of the orientation bearing the T-DNA right border adjacent to the pBR322 origin of replication is designated pCGN1539, and the orientation bearing the T-DNA right border adjacent to the Ri plasmid origin of replication is designated pCGN1540. This binary vectors have several advantageous features, including a minimal amount of DNA between the T-DNA borders, high stability in *Agrobaclerium* hosts, high copy number in *E. coli* hosts, and a blue/white screen with multiple restriction sites for ease of cloning target DNA.

### Section 5B: Construction of Binary Vectors containing chimeric genes

In the previous section the construction of pCGN783 and pCGN1540 are detailed. These are binary vectors which can be used in *Agrobacterium* mediated transformation experiments to transform plants. The vectors are designed to cotransform a chimeric gene of interest into a plant, however, the chimeric gene first must be subcloned into the binary vector. The following section details the subcloning of the chimeric genes constructed in Section 4 into either the pCGN783 or the pCGN1540 binary vectors. The resulting vectors are capable of transforming plants with the chimeric gene.

### Example 38: Construction of pCGN1754 and pCGN1760 (pCGN783 containing the empty SSU promoter cassette in either orientation)

The BamHI site of pCGN783 lies near the right T-DNA border, with the plant selectable marker gene lying between the left T-DNA border and the BamHI site. Cloning a chimeric gene construct into the BamHI site places the chimeric gene between the plant selectable marker gene and the right T-DNA border. The unique BglII site of pCGN1509 lies in a non-essential portion of the *ocs* 3' region.

pCGN1509 is digested with BglII and the entire vector is cloned into the BamHI site of pCGN783, and both possible orientations are recovered. A plasmid in which the RUBISCO small subunit promoter and *ocs* 3' regions are proximal to the right T-DNA border of pCGN783 is designated pCGN1754, and a plasmid in which the RUBISCO small subunit promoter and *ocs* 3' regions are proximal to the plant selectable marker gene of pCGN783 is designated pCGN1760.

### Example 39: Construction of pCGN1755A, pCGN1755B, pCGN1755C, and pCGN1755D (pCGN783 containing the SSU/PR-1a (sense and anti-sense) expression cassette in either orientation)

pCGN1752A is digested with BglII and the entire vector is cloned into BamHI digested pCGN783, and both possible orientations are recovered. A plasmid in which the RUBISCO small subunit promoter-PR1-*ocs* 3' regions are proximal to the right T-DNA border of pCGN783 is designated pCGN1755C, and a plasmid in which the RUBISCO small subunit promoter-PR1-*ocs* 3' regions are proximal to the plant selectable marker gene of pCGN783 is designated pCGN1755A.

pCGN1752B is digested with BglII and the entire vector is cloned into BamHI digested pCGN783, and both possible orientations are recovered. A plasmid in which the RUBISCO small subunit promoter-PR1-*ocs* 3' regions are proximal to the right T-DNA border of pCGN783 is designated pCGN1755B, and a plasmid in which the RUBISCO small subunit promoter-PR1-*ocs* 3' regions are proximal to the plant selectable marker gene of pCGN783 is designated pCGN1755B.

### Example 40: Construction of pCGN1756A, pCGN1756B, pCGN1756C, and pCGN1756D (pCGN783 containing the SSU promoter/PR-1b (sense and anti-sense) expression cassette in either orientation)

pCGN1753A is digested with BglII and the entire vector is cloned into BamHI digested pCGN783, and both possible orientations are recovered. A plasmid in which the RUBISCO small subunit promoter-PR1-*ocs* 3' regions are proximal to the right T-DNA border of pCGN783 is designated pCGN1756C, and a plasmid in which the RUBISCO small subunit promoter-PR1-*ocs* 3' regions are proximal to the plant selectable marker gene of pCGN783 is designated pCGN1756A.

pCTN1753B is digested with the BglII and cloned into BamHI digested pCGN783, and both possible orientations are recovered. A plasmid in which the RUBISCO small subunit promoter-PR1-*ocs* 3' regions are proximal to the right T-DNA border of pCGN783 is designated pCGN1756D, and a plasmid in which the RUBISCO small subunit promoter-PR1-*ocs* 3 regions are proximal to the plant selectable marker gene pCGN783 is designated pCGN1756B.

### Example 41: Construction of pCGN1766 and pCGN1767 (pCGN783 containing an empty double CaMV 35S promoter cassette in either orientation)

pCGN1761 is digested with BamHI and the entire vector is cloned into BamHI digested pCGN783, and both possible orientations are recovered. A plasmid in which the double CaMV 35S promoter and *tm-l* 3' regions are proximal to the plant selectable marker gene of pCGN783 is designated pCGN1767, and a plasmid in which the double 35S promoter and *tm-l* 3' regions are proximal to the right T-DNA border of pCGN783 is designated pCGN1766.

### Example 42: Construction of pCGN1764A, pCGN1764B, pCGN1764C, and pCGN1764D (Double CaMV 35S promoter/PR-1a (sense and anti-sense) into pCGN783)

pCGN1762A is digested with BamHI and cloned into BamHI digested pCGN783, and both possible orientations are recovered. A plasmid in which the double 35S-PR1-*tm-l* 3' regions are proximal to the plant selectable marker gene of pCGN783 is designated pCGN1764A, and a clone in which the double35S-PR1-*tm-l* 3' regions are proximal to the right T-DNA border of pCGN783 is designated pCGN1764C.

pCGN1762B is digested with BamHI and cloned into BamHI digested pCGN783, and both orientations are recovered. A plasmid in which the double 35S promoter is proximal to the plant selectable marker is designated pCGN1764B. A plasmid in the opposite orientation is designated pCGN1764D.

### Example 43: Construction of pCGN1765A, pCGN1765B, pCGN1765C, and pCGN1765D (Double CaMV 35S promoter/PR-1b (sense and anti-sense) into pCGN783 in either orientation)

pCGN1763A is digested with BamHI and cloned into BamHI digested pCGN783, and both possible orientations are recovered. A plasmid in which the double 35S-PR1-*tm-l* 3' regions are proximal to the plant selectable marker gene of pCGN783 is designated pCGN1765A, and a plasmid in which the double35S-PR1-*tm-l* 3' regions are proximal to the right T-DNA border of pCGN783 is designated pCGN1765C.

pCGN1763B is digested with BamHI and cloned into BamHI digested pCGN783 and both possible orientations are recovered. A plasmid in which the double35S-PR1-*tm-l* 3' regions are proximal to the plant selectable marker gene of pCGN783 is designated pCGN1765B, and a plasmid in which the double35S-PR1-*tm-l* 3' regions are proximal to the right T-DNA border of pCGN783 is designated pCGN1765D.

### Example 44: Construction of pCGN1780A, pCGN1780B, pCGN1780C, pCGN1780D (double CaMV35S promoter/cucumber chitinase/lysozyme (sense and anti-sense) into pCGN783 in either - orientation)

pCIB1000 is digested with BamHI and cloned into BamHI site in pCGN783, and both possible orientations are recovered. A plasmid in which the double35S-chitinase-*tm-l* 3' regions are proximal to the plant selectable marker gene of pCGN783 is designated pCGN1780A, and a plasmid in which the double35S-chitinase-*tm-l* 3'regions are proximal to the right T-DNA border of pCGN783 is designated pCGN1780C.

pCIB1001 is digested with BamHI and cloned into BamHI digested pCGN783 in either orientation. A plasmid in which the double 35S-chitinase-*tm-l* 3' regions are proximal to the plant selectable marker gene of pCGN783 is designated pCGN1780B. A plasmid in which the double 35S-chitinase-*tm-l* 3' regions are proximal to right T-DNA border of pCGN783 is designated pCGN1780D.

### Example 45: Construction of pCGN1789 (Double CaMV 35S promoter empty cassette into pCGN1540 in either orientation)

The 2.36 kb XbaI-PstI fragment of pCGN1431 is subcloned into XbaI-PstI digested pCGN1540 to create the plasmid pCGN1789. This plasmid has the insert oriented in a direction such that the double 35S CaMV promoter is proximal to the plant selectable marker.

### Example 46: Construction of pCGN1774A, pCGN1774B, pCGN1774C, and pCGN1774D (Double CaMV 35S promoter/PR-1a (sense and anti-sense) into pCGN1540 in either orientation)

The 4.2 kb XbaI fragment of pCGN1762A is subcloned into XbaI digested pCGN1540, and both possible orientations are recovered. A plasmid in which the double 35S promoter fragment is proximal to the right T-DNA border of pCGN1540 is designated pCGN1774A, and a plasmid in which the double 35S promoter fragment is proximal to the plant selectable marker gene of pCGN1540 is designated pCGN1774C.

The 4.2 kb XbaI fragment of pCGN1762B is cloned into XbaI digested pCGN1540, and both possible orientations are recovered. A plasmid in which the double 35S promoter fragment is proximal to the right T-DNA border of pCGN1540 is designated pCGN1774B, and a plasmid in which the double 35S promoter fragment is proximal to the plant selectable marker gene of pCGN1540 is designated pCGN1774D.

### Example 47: Construction of pCGN1775A, pCGN1775B, pCGN1775C, and pCGN1775D (Double CaMV 35S promoter/PR-1b (sense and anti-sense) into pCGN1540 in either orientation)

The 4.1 kb XbaI fragment of pCGN1763A is cloned into XbaI digested pCGN1540, and both possible orientations are recovered. A plasmid in which the double 35S promoter fragment is proximal to the right T-DNA border of pCGN1540 is designated pCGN1775A, and a plasmid in which the double 35S promoter fragment is proximal to the plant selectable marker gene of pCGN1540 is designated pCGN1775C.

The 4.1 kb XbaI fragment of pCGN1763B is cloned into Xbal digested pCGN1540, and both possible orientations are recovered. A plasmid in which the double 35S promoter fragment is proximal to the right T-DNA border of pCGN1540 is designated pCGN1775B, and a clone in which the double 35S promoter fragment is proximal to the plant selectable marker gene of pCGN1540 is designated pCGN1775D.

### Example 48: Construction of pCGN1783C and pCGN1783D (Double CaMV 35S promoter/PR-R major (sense and anti-sense) into pCGN1540 in either orientation)

The 4.5 kb XbaI fragment of pCIB1002 is subcloned into XbaI digested pCBN1540 in such an orientation that the double 35S promoter is proximal to the plant selectable marker gene of 1540. This plasmid is designated as pCGN1783C.

The 4.5 kb XbaI fragment of pCIB1003 is subcloned into XbaI digested pCGN1540 in such an orientation that the double 35S promoter fragment is proximal to the plant selectable marker gene of pCGN1540 to create pCGN1783D.

### Example 49: Construction of pCIB1026 and pCIB1027 (Double CaMV 35S promoter/PR-P (sense and anti-sense) into pCGN1540 in either orientation)

The XbaI fragment of pCIB1020, which contains the chimeric PR-P gene, is subcloned into XbaI digested pCGN1540 in such an orientation that the promoter fragment is proximal to the plant selectable marker gene. This plasmid is designated as pCIB1026.

The XbaI fragment of pCIB1021, which contains the chimeric PR-P anti-sense gene, is subcloned into XbaI digested pCGN1540 in such an orientation that the promoter fragment is proximal to the plant selectable marker gene. This plasmid is designated as pCIB1027.

### Example 50: Construction of pCIB1028 and pCIB1029 (Double CaMV 35S promoter/PR-Q (sense and anti-sense) into pCGN1540 in either orientation)

The XbaI fragment from pCIB 1022, which contains the chimeric PR-Q gene, is subcloned into XbaI digested pCGN1540 in an orientation such that the promoter fragment is proximal to the plant selectable marker. This plasmid is designated as pCIB1028.

The XbaI fragment from pCIB1023, which contains the chimeric PR-Q anti-sense gene, is subcloned into XbaI digested pCGN1540 in such an orientation that the promoter fragment is proximal to the plant selectable marker. The plasmid is designated pCIB1029.

### Example 51: Construction of pCIB1030 and pCIB1031 (Double CaMV 35S promoter/PR-O' (sense and anti-sense) into pCGN1540 in either orientation)

The XbaI fragment of pCIB1024, which contains the chimeric PR-O' gene is subcloned into XbaI digested pCGN1540 in such a way that the 35S promoter is proximal to the plant selectable marker. The plasmid is designated pCIB1030.

The XbaI fragment of pCIB1025, which contains the chimeric PR-O' anti-sense gene, is subcloned into XbaI digested pCGN1540 in a similar orientation as pCIB 1030. The new plasmid is designated pCIB 1031.

### Example 51A: Construction of pCIB1030 and pCIB1031 (double CaMV 35S promoter/PR-O' (sense and anti-sense) into pCGN 1540)

The XbaI fragment of pCIB1024A, which contains the chimeric PR-O' gene, is subcloned into XbaI digesteed pCGN1540 in such a way that the 35S promoter is proximal to the plant selectable marker. This plasmid is designated as pCIB1030A.

The XbaI fragment of pCIB1025A, which contains the chimeric PR-O' gene in an anti-sense orientation, is subcloned into XbaI digested pCGN1540 in such a way that the 35S promoter is proximal to the plant selectable marker. The resulting plasmid is designated pCIB1031A.

### Example 51B: Construction of pCIB1042 and pCIB1043 (double CaMV 35S promoter/PR-O' (sense and anti-sense) into pCGN1540)

The XbaI fragment of pCIB1032, which contains the chimeric PR-O' gene, is subcloned into XbaI digested pCGN1540 in such a way that the 35S promoter is proximal to the plant selectable marker. This plasmid is designated as pCIB1042.

The XbaI fragment of pCIB1033, which conains the chimeric PR-O' gene in an anti-sense orientation, is subcloned into XbaI digested pCGN1540 in such a way that the 35S promoter is proximal to the plant selectable marker. The resulting plasmid is designated pCIB1043.

### Example 51C: Construction of pCIB1044 and pCIB1045 (double CaMV 35S promoter/PR-N (sense and anti-sense) into pCGN1540)

The XbaI fragment of pCIB 1034, which contains the chimeric PR-O' gene is subcloned into XbaI digested pCGN1540 in such a way that the 35S promoter is proximal to the plant selectable marker. This plasmid is designated as pCIB1044.

The XbaI fragment of PCIB1035, which contains the chimeric PR-O' gene in an anti-sense orientation, is subcloned into XbaI digested pCGN1540 in such a way that the 35S promoter is proximal to the plant selectable marker. The resulting plasmid is designated pCIB1045.

### Example 51D: Construction of pCIB1046 and pCIB1047 (double CaMV 35S promoter/PR-O (sense and anti-sense) into pCGN1540)

The XbaI fragment of pCIB1036, which contains the chimeric PR-O' gene, is subcloned into XbaI digested pCGN1540 in such a way that the 35S promoter is proximal to the plant selectable marker. This plasmid is designated as pCIB1046.

The XbaI fragment of PCIB1037, which contains the chimeric PR-O' gene in an anti-sense orientation, is subcloned into XbaI digested pCGN1540 in such a way that the 35S promoter is proximal to the plant selectable marker. The resulting plasmid is designated pCIB1047.

### Example 51E: Construction of pCIB1048 and pCIB1049 (double CaMV 35S promoter/PR-2' (sense and anti-sense) into pCGN1540)

The XbaI fragment of pCIB1038, which contains the chimeric PR-O' gene, is subcloned into XbaI digested pCGN1540 in such a way that the 35 S promoter is proximal to the plant selectable marker. This plasmid is designated as pCIB1048.

The XbaI fragment of PCIB 1039, which contains the chimeric PR-O' gene in an anti-sense orientation, is subcloned into XbaI digested pCGN1540 in such a way that the 35S promoter is proximal to the plant selectable marker. The resulting plasmid is designated pCIB1049.

### Example 52: Construction of pCGN1781C and pCGN1781D (Double CaMV 35S promoter/basic glucanase (sense and anti-sense) into pCGN1540 in either orientation)

The 4.9 kb XbaI fragment of pCIB1005B is subcloned into XbaI digested pCGN1540 in such an orientation that the double 35S promoter fragment is proximal to the plant selectable marker gene of pCGN1540 to create pCGN1787C.

The 4.5 kb XbaI fragment of pCIB1006B is subcloned into XbaI digested pCGN1540 in such an orientation that the double 35S promoter fragment is proximal to the plant selectable gene of pCGN1540 to create pCGN1787D.

### Example 53: Construction of pCGN1782C and pCGN1782D (Double CaMV 35S promoter/basic chitinase (sense and anti-sense) into pCGN1540 in either orientation)

The 4.8 kb XbaI fragment of pCGN1007 is cloned into XbaI digested pCGN1540 in such an orientation that the double 35S promoter fragment is proximal to the plant selectable marker gene of pCGN1540 to create pCGN1782C.

The 4.8 kb fragment of pCIB1008 is cloned into Xbal digested pCGN1540, similarly, to create pCGN1782D.

### Example 54: Construction of pCGN1790C and pCGN1790D (Double CaMV 35S promoter/SAR8.2 (sense and anti-sense) into pCGN1540 in either - orientation)

The 2.89 kb XbaI-PstI fragment of pCGN1788A is subcloned into XbaI-PstI digested pCGN1540 to create pCGN1790C. The orientation of the double 35S promoter in this construct is the same as the other C type constructs in the examples above.

The 2.89 kb fragment of pCGN1788B is subcloned into XbaI-PstI digested pCGN1540 to create pCGN1790D. The orientation of the promoter in the construct is the same as in pCGN1790C.

### Example 55: Construction pCGN1779C, and pCGN1779D (Double CaMV 35S promoter/cucumber chitinase/lysozyme (sense and anti-sense) into pCGN1540 in either orientation)

The 4.6 kb Xbal fragment of pCIB 1000 is cloned into XbaI digested pCGN1540 in such an orientation that the double 35S promoter fragment is proximal to the plant selectable marker gene of pCGN1540 to create pCGN1779C. The 4.6 kb XbaI fragment of pCIB1001 is cloned into XbaI digested pCGN1540 in such an orientation that the double 35S promoter fragment is proximal to the plant selectable marker gene of pCGN1540 to create pCGN1779D.

### Example 56: Vectors having Hygromycin-Resistance as the Plant-selectable Marker Gene

Plant transformation vectors having the hygromycin- resistance gene instead of the kanamycin gene as used above have been constructed (Rothstein et al., 1987). The vector pCIB743 is one such vector. The chimeric gene for expression in the plant is cut from any of the vectors described above using a suitable restriction enzyme, for example XbaI, and inserted into the polylinker of pCIB743. This constructs the chimeric gene(s) for plant expression in the broad host range transformation vector conferring hygromycin-resistance to transformed plant tissue. This allows one skilled in the art to utilize either hygromycin-resistance, or kanamycin-resistance, or both, as selection for transformed plant tissue.

### SECTION 6. TRANSFORMATION OF A. TUMEFACIENS

### Example 57: Transformation of A. tumefaciens with binary vectors

The binary vectors described in Section 5 are transformed into *A. tumefaciens* strain LB4404 by the following method. The *Agrobacterium* strain is grown at 30°C overnight in 5 ml of LBMG medium (50 % L broth, 50 % mannitol-glutamate broth (Garfinkel and Nester, 1980). The 5 ml culture is added to 250 ml of LBMG and shaken vigorously until the culture density reaches an OD=0.6 at 600 nm wavelength. The cells are then collected by centrifugation at 8000 X g and resuspended in 5 ml of LBMG. 200 µl of cells are added to 0.2 to 1 µg of binary plasmid DNA in LBMG and the mix is frozen immediately in a dry ice/ethanol bath. After 5 minutes the tube is placed in a 37°C water bath for 5 minutes and then 2 ml of LBMG is added. The suspension is kept in a 30°C water bath for 2 to 3 hours and then the cells are collected by centrifugation. The cells are resuspended in a minimal volume of LBMG and then plated on selective media (LBMG plates with 100 µg/ml gentamycin). Colonies appear after 2 to 3 days at 30°C.

### SECTION 7. TRANSFORMATION OF PLANTS

### Example 58: A. tumefaciens-mediated transformation of N. tabacum

Explants roughly 5 to 10 mm are cut from young leaves 3 to 5 cm long and third to sixth from the apex of *N. tabacum* cv 'Xanthi nc' grown under axenic conditions (Facciotti and Pilet, 1979) in solid MS medium (Murashige and Skoog, 1962) containing 0.7 % phytagar (Gibco-BRL), 1 mg/l IAA, 0.15 mg/l kinetin. These explants are plated on solid MS medium containing 0.6 % phytagar, 40 mg/l adenine sulfate, 2 mg/l IAA, and 2 mg/l kinetin on the surface of which is placed a #1 Whatman filter and incubated for 24 hr in the dark at 24°C. *Agrobacterium* strains (bearing chimeric gene constructions prepared in Section 5 are grown overnight in LBMG at 30°C on a shaker at 180 rmp. Explants are dipped into a bacterial suspension of 3.3 X 10⁸ cells/ml for approximately 5 minutes, blotted on sterile paper towels, and re-plated on the same plates. After 48 hours explants are placed on selection medium containing the same plate medium as above plus 350 mg/l cefotaxime and 100 mg/l kanamycin. Co-cultivated control tissue is placed on the same medium but without kanamycin. The explants are transferred to fresh media every two weeks. Shoots are harvested 4 to 8 weeks after co-cultivation, placed on 50 ml culture tubes with 25 ml of solid MS medium containing 0.6 % phytagar, 1 mg/l IBA, 350 mg/l cefotaxime, and 100 mg/l kanamycin. All tissue is grown at 24° to 28°C, 12 hours light, 12 hours dark, light intensity 6700 to 8400 lx. Shoots root in 1 to 2 weeks and are then transplanted to planting mix in 4" pots and placed in the "transgenic plant phytotron".

### Example 59: Leaf Disk Transformation of Tobacco

*Agrobacterium* strains containing the binary vectors described above are grown 18 to 24 hours in glutamate salts media adjusted to pH 5.6 and supplemented with 0.15 % mannitol, 50 µg/ml kanamycin, 50 µg/ml spectinomycin and 1 mg/ml streptomycin before they are diluted to an OD₆₀₀ of 0.2 in the same media without the antibiotics. The bacteria are then grown for three to five hours before dilution to an OD₆₀₀ of 0.2 to 0.4 for inoculation of discs of 5 to 7 mm punched from leaves *of N. tabacum* cv. xanthi that have been grown aseptically in GA7 containers, following a modification of the method of Horsch et al. (1985).

The leaf disks are maintained on 0.7 % agar containing Murashige and Skoogs major and minor salts (MS), 1 mg/l benzyladenine and I mg/ml NAA for two days before transfer to the same media containing 50 µg/ml kanamycin, 100 µg/ml carbenicillin and 100 µg/ml mefoxin. Shoots which form on the discs are excised and propagated until six plantlets are obtained by subculturing the shoot tips on MS media containing 50 µg/ml kanamycin in GA7 containers.

The plantlets are rooted on medium containing no hormones and 50 µg/ml kanamycin, transferred to soil and hardened in a phytotron before transfer to the greenhouse for induction treatment with chemical regulators. At flowering time flowers are induced to selfpollinate. Seeds are harvested following maturation.

### Example 60: Production of Transgenic Tobacco Callus and Plants

*Agrobacterium* strains containing the binary vectors are used to transform callus forming from the leaf disks (Example 34). Callus forming on kanamycin-containing MSBN selection medium is maintained on a callus growth medium comprised of MS major, minor salts and Fe-EDTA (Gibco # 500-1117; 4.3 g/l), MS vitamins, 100 mg/l myo-inositol, 20 g/l sucrose, 2 mg/l NAA and 0.3 mg/l kinetin.

The callus can be used to regenerate transgenic plants by transferring callus pieces to MSBN medium and following methods described.

### Example 61: Transformation of Carrot

*Agrobacterium* strains containing the binary vectors are grown as described in Example 59. The bacteria, diluted to an OD₆₀₀ of 0.2 to 0.4, are then used for inoculation of discs cut from surface sterilized carrots.

To surface sterilize the carrots they are peeled and then soaked 20 minutes in a 10 % solution of chlorox. The carrots are rinsed with sterile water, sliced into 5 mm pieces and placed basal side up onto water agar. 20 to 50 µl of bacteria are then applied to the upper surface of the discs. After 7 days the discs are transferred to 0.7 % agar containing MS salts, 3 % sucrose, 0.1 mg/l 2,4-D, 50 µg/ml kanamycin, 100 µg/ml carbenicillin, and 100 µg/ml mefoxin. Callus forming around the cambial ring is excised and placed on 0.7 % MS agar supplemented with 3 % sucrose, 0.1 mg/12,4-D, 50 µg/ml kanamycin, 100 µg/ml carbenicillin, and 100 µg/ml mefoxin. After the callus has been grown it is cut into small pieces and randomized onto four plates of the same media.

### Example 62: Transformation of Sunflower

*Agrobacterium* strains containing the binaray vectors are grown as described. The bacteria, diluted to an OD₆₀₀ of 0.2 to 0.4, are then used for inoculation of stems of sunflower plants prepared as follows:

Sunflower seeds are soaked 10 min in 10 % captan followed by 10 min in 10 % chlorox and rinsing with sterile water. The seed coats are removed and the seeds are germinated on 0.7 % water agar in the dark for three days, after which they are placed into a labline incubator set at 23°C with a 12 hour day and night. The seedlings are grown for one week before decapitation and inoculation of the bacteria onto the cut stem surface. After one week the inoculated stems are cut and placed on 0.7 % agar containing MS salts, 3 % sucrose, 2 mg/ml NAA, 1 mg/ml BAP, 100 µg/ml carbenicillin, 100 µg/ml mefoxim and 50 µg/ml kanamycin. The callus is transferred to fresh media every two weeks until sufficient quantity is obtained for 4 plates. Half of the callus growing from the virulent *Agrobaclerium* strains is transferred to media without hormones containing 50 µg/ml kanamycin.

### Example 63: Transformation of Tomato

*Agrobacterium* strains containing the binary vectors are grown as described in Example 59. The bacteria, diluted to an OD₆₀₀ of 0.2 to 0.4, are then used for inoculation of stems of tomato seedlings prepared as follows:

Tomato seeds are soaked 20 min in 10 % chlorox and rinsed with sterile water. The seeds are germinated on 0.7 % water agar in the dark for three days, after which they are placed into a labline incubator set at 23°C with a 12 hour day and night. The seedlings are grown for one week before decapitation and inoculation of the bacteria onto the cut stem surface.

After one week the inoculated stems are cut and placed on 0.7 % agar containing MS salts, 3 % sucrose, 2 mg/ml NAA, 1 mg/ml BAP, 100 µg/ml carbenicillin, 100 µg/ml mefoxim, and 50 µg/ml kanamycin. The callus is transferred to fresh media every two weeks until sufficient quantity is obtained for 4 plates.

### Example 64: Transformation of Cotton

*Agrobacterium* strains containing the binary vectors are grown as described. The bacteria, diluted to an OD₆₀₀ of 0.2 to 0.4, are then used for inoculation of cotton cotyledons prepared as follows:

The cotton seeds are soaked 20 min in 10 % chlorox and rinsed with sterile water. The seeds are germinated on 0.7 % water agar in the dark. The seedlings are grown for one week before inoculation of the bacteria onto the cotyledon surface.

The inoculated cotyledons are allowed to form callus before they are cut and placed on 0.7 % agar containing MS salts, 3 % sucrose, 100 µg/ml carbenicillin, and 100 µg/ml mefoxim. The callus is transferred to fresh media every three weeks until sufficient quantity is obtained for 4 plates. Half of the callus growing from the virulent *Agrobacterium* strains is transferred to media without hormones containing 50 µg/ml kanamycin.

### Example 65: Preparation of a Special Type of Callus of Zea mays, Elite Inbred line Funk 2717

*Zea mays* plants of the inbred line Funk 2717 are grown to flowering in the greenhouse, and self pollinated. Immature ears containing embryos approximately 2 to 2.5 mm in length are removed from the plants and sterilized in 10 % Chlorox solution for 20 minutes. Embryos are aseptically removed from the kernels and plated with the embryo axis downwards on OMS medium containing 0.1 mg/l 2,4-D, 6 % sucrose and 25 mM L-proline solidified with 0.24 % Gelrite® (initiation medium). After two weeks' culture in the dark at 27°C, the callus developing on the scutellum is removed from the embryo and plated on B5 medium (Gamborg et al., 1968) containing 0.5 mg/l 2,4-D and solidified with 0.24 % Gelrite®. The callus is subcultured every two weeks to fresh medium. After a total of eight weeks after placing the embryos on the initiation medium, the special type of callus is identified by its characteristic morphology. This callus is subcultured further on the same medium. After a further period of two months, the callus is transferred to, and serially subcultured on, N6 medium containing 2 mg/l 2,4-D and solidified with Gelrite®.

### Example 66: Preparation of a Suspension Culture of Zea mays, Elite Inbred Funk 2717

The callus described above is subcultured for a total of at least six months. The type of callus chosen for subculture is relatively non-mucilaginous, granular and very friable, such that it separates into small individual cell aggregates upon placing into liquid medium. Cultures containing aggregates with large, expanded cells are not retained. Approximately 500 mg aliquots of the special callus of *Zea mays* elite inbred funk 2717 are placed into 30 ml of N6 medium containing 2 mg/l 2,4-D in 125 ml Delong flasks. After one week of culture at 26°C in the dark on a gyratory shaker (130 rpm, 2.5 cm throw), the medium is replaced with fresh medium. The suspensions are again subcultured in this way after another week. At that time, the cultures are inspected, and those which do not show large numbers of expanded cells are retained. Suspension cultures containing aggregates with large, expanded cells are discarded. The preferred tissue consists of densely cytoplasmic dividing cell aggregates which have a characteristically smoother surface than the usual type of cell aggregates. The cultures retained have at least 50 % of the cells represented in these small aggregates. This is the desired morphology. These suspensions also have a rapid growth rate, with a doubling time of less than one week. The suspension cultures are subcultured weekly by transferring 0.5 ml PCV into 25 ml of fresh medium. After four to six weeks of subculture in this fashion, the cultures increase two- to three-fold per weekly subculture. Cultures in which more than 75 % of the cells are of the desired morphology are retained for further subculture. The lines are maintained by always choosing for subculture the flask whose contents exhibit the best morphology. Periodic filtration through 630 µm pore size stainless steel sieves every two weeks is used in some cases to increase the dispersion of the cultures, but is not necessary.

### Example 67: Preparation of Protoplasts from Suspension Cultures of Zea mays

1 to 1.5 ml PCV of the suspension culture cells from above are incubated in 10 to 15 ml of a filter-sterilized mixture consisting of 4 % cellulase RS with 1 % Rhozyme in KMC (8.65 g/l KCI, 16.47 g/l MgCl₂·6 H₂O and 12.5 g/l CaCl₂·2 H₂O, 5 g/l MES, pH 5.6) salt solution. Digestion is carried out at 30°C on a slow rocking table for a period of 3 to 4 hours. The preparation is monitored under an inverted microscope for protoplast release. The protoplasts which are released are collected as follows: The preparation is filtered through a 100 µm mesh sieve, followed by a 50 µm mesh sieve. The protoplasts are washed through the sieves with a volume of KMC salt solution equal to the original volume of enzyme solution. 10 ml of the protoplast preparation is placed in each of several disposable plastic centrifuge tubes, and 1.5 to 2 ml of 0.6 M sucrose solution (buffered to pH 5.6 with 0.1 % MES and KOH) layered underneath. The tube is centrifuged at 60 to 100 x g for 10 minutes, and the protoplasts banding at the interface collected using a pipette and placed in a fresh tube. The protoplast preparation is resuspended in 10 ml of fresh KMC salt solution, and centrifuged for five minutes at 60 to 100 x g. The supernatant is removed and discarded, and the protoplasts resuspended gently in the drop remaining, and then 10 ml of a 13/14 strength KMC solution gradually added. After centrifuging again for five minutes, the supernatant is again removed and the protoplasts resuspended in a 6/7 strength KMC solution. An aliquot is taken for counting, and the protoplasts again sedimented by centrifugation. The protoplasts are resuspended at 10⁷ per ml in KM-8p medium or in 0.5 M mannitol containing 6 mM MgCl₂ or other suitable medium for use in transformation as described in the following examples. This protoplast suspension is used for transformation and is cultured as described below.

### Example 68: Transformation of Zea mays Protoplasts by Electroporation

A. All steps except the heat shock are carried out at room temperature (22 to 28°C). The protoplasts are resuspended in the last step of above in 0.5 M mannitol containing 0.1 % MES and 6 mM MgCl₂. The resistance of this suspension is measured in the chamber of a Dialog Electroporator and adjusted to 1 to 1.2 kΩ using a 300 mM MgCl₂ solution. The protoplasts are heat-shocked by immersing the tube containing the sample in a water bath at 45°C for five minutes, followed by cooling to room temperature on ice. 4 µg of linearized plasmid containing a plant-selectable hygromycin resistance gene such as described by Rothstein et al. (1987) or chimeric gene constructs as described and 20 µg of calf thymus carrier DNA are added to aliquots of 0.25 ml of this suspension. 0.125 ml of a 24 % PEG solution (MW 8000) in 0.5 M mannitol containing 30 mM MgCl₂ are added to the protoplasts. The mixture is mixed well but gently, and incubated for 10 minutes. The sample is transferred to the chamber of the electroporator and samples pulsed three times at 10 second intervals, at initial voltages of 1500, 1800, 2300 or 2800 Vcm⁻¹, and an exponential decay time of 10 msec.

The protoplasts are cultured as follows. The samples are plated in 6 cm petri dishes at room temperature. After a further 5 to 15 minutes, 3 ml of KM-8p medium containing 1.2 % SeaPlaque agarose and 1 mg/l 2,4-D are added. The agarose and protoplasts are mixed well and the medium allowed to gel.

B. This is repeated with one or more of the following modifications:
(1) The resistance of the protoplast preparation is adjusted to 0.5 to 0.7 kΩ.
(2) The PEG used is PEG with a MW of 4000.
(3) No PEG is added, or one-half volume of 12 % PEG is added.
(4) The pulses are applied at intervals of three seconds.
(5) The protoplasts are plated after the electroporation in dishes placed on a plate cooled to a temperature of 16°C.
(6) The protoplasts are placed in tubes after the electroporation step, washed with 10 ml of 6/7 strength KMC solution or with W5 solution (comprised of 380 mg/l KCl, 18.375 g/l CaCl₂·2 H₂O, 9 g/l NaCl; 9 g/l glucose, pH 6.0), then collected by centrifugation at 60 x g for 10 minutes, resuspended in 0.3 ml of KM medium, and plated as in A.
(7) The calf thymus carrier DNA is not added.

### Example 69: Transformation of Zea mays Protoplasts by Treatment with PEG

A. The protoplasts are resuspended at the last step of above in a 0.5 M mannitol solution containing 12 to 30 mM MgCl₂. A heat shock of 45°C for five minutes is given as described. The protoplasts are distributed in aliquots for transformation in centrifuge tubes, 0.3 ml of suspended protoplasts per tube. During the next 10 minutes the following are added: DNA and PEG solution (MW 6000, 40 % containing 0.1 M Ca(NO₃)₂ and 0.4 M mannitol; pH 8 to 9 with KOH) to give a final concentration of 20 % PEG. The aliquots are incubated for 30 minutes with occasional gentle shaking, and then the protoplasts are placed in petri dishes (0.3 ml original protoplast suspension per 6 cm diameter dish) and cultured as described.

B. This is repeated and the protoplasts are washed after 30 minutes of incubation in the PEG solution of above, by adding 0.3 ml of W5 solution five times at two- to three-minute intervals. The protoplast suspension is centrifuged, the supernatant removed, and the protoplasts are cultured as described.

C. The above is repeated with the modification that the final concentration of PEG is between 13 and 25 %.

### Example 70: Regeneration of Callus From Protoplasts

The plates containing the protoplasts in agarose are placed in the dark at 26°C. After 14 days, colonies arise from the protoplasts. The agarose containing the colonies is transferred to the surface of a 9 cm diameter petri dish containing 30 ml of N6 medium containing 2 mg/12,4-D, solidified with 0.24 % Gelrite®. This medium is referred to as 2N6. The callus is cultured further in the dark at 26°C and callus pieces subcultured every two weeks onto fresh solid 2N6 medium.

### Example 71: Selection of Transformed Callus of Zea mays

The above example is repeated with the modification that 100 mg/l or 200 mg/l hygromycin B is added to the 2N6 medium in order to select for transformed cells.

### Example 72: Regeneration of Corn Plants

A. Callus is placed on 2N6 medium for maintenance and on ON6 (comprising N6 medium lacking 2,4-D) and N61 medium (comprising N6 medium containing 0.25 mg/l 2,4-D and 10 mg/l kinetin) to initiate regeneration. Callus growing on ON6 and N61 media is grown in the light (16 hours/day light of 840 to 8400 lx from white fluorescent lamps). Callus growing on N61 medium is transferred to ON6 medium after two weeks, as prolonged time on N61 medium is detrimental. The callus is subcultured every two weeks even if the callus is to be transferred again on the same medium formulation. Plantlets appear in about four to eight weeks. Once the plantlets are at least 2 cm tall, they are transferred to ON6 medium in GA7 containers. Roots form in two to four weeks, and when the roots look well-formed enough to support growth, the plantlets are transferred to soil in peat pots, under a light shading for the first four to seven days. It is often helpful to invert a clear plastic cup over the transplants for two to three days to assist hardening off. Once the plants are established, they are treated as normal corn plants and grown to maturity in the greenhouse. In order to obtain progeny plants are self pollinated or crossed with wild type.

B. The above example is repeated with the modification that 100 mg/l or 200 mg/l hygromycin B is added to the medium used to maintain the callus.

### Example 73: Preparation of Embryogenic Suspensions from Tissue of Dactylis glomerata L. (Orchardgrass)

A. Embryogenic callus is initiated from basal sections of the youngest leaves of greenhouse-grown orchardgrass plants (*Dactylis glomerata* L.) as described by Hanning and Conger (1982). The leaves are surface sterilized by immersion in a 1:10 dilution of Chlorox solution (5.25 % sodium hypochlorite; The Clorox Company, Oakland, Ca.) for about 10 minutes and then cut aseptically into small segments of 1 to 5 mm in length or in diameter. These segments are plated on sterile SH-30 medium containing 0.8 % agarose as a gelling agent. Callus and/or embryogenic structures appear within 2 to 6 weeks after plating, upon culture at about 25°C. Embryogenic callus is maintained by subculturing onto fresh SH-30 medium every 2 to 4 weeks and culturing in the dark at 25°C.

B. Embryogenic suspension cultures are initiated by placing approximately 0.5 g fresh weight of embryogenic callus into 50 ml of liquid medium described by Gray and Conger (1985) containing 45 µM dicamba and 4 g/liter casein hydrolysate. The suspension cultures are grown at 27°C under a 16 hours light (3300 lx), 8 hours dark photoperiod on a gyratory shaker at about 130 rpm in 125 ml Delong flasks sealed with a metal cap and parafilm®. After approximately four weeks the large clumps are allowed to settle for about 30 seconds and 10 ml aliquots of the supernatant medium containing small cell clusters are removed and transferred to 50 ml of fresh medium. This process is repeated every 3 to 4 weeks using the most successful cultures as judged by smaller clump size and better quality based on the presence of small, cytoplasmic cells. After 5 to 8 transfers the suspensions are essentially free of non embryogenic cells and the majority of the embryogenic cell clusters are quite small (150 to 2000 µm).

### Example 74: Isolation and Purification of Dactylis glomerata L. Protoplasts

Protoplasts are prepared from embryogenic suspension cultures of above by aseptically filtering the cells on a Nalgene® 0.2 µm filter unit and then adding 0.5 g fresh weight cells to each 12.5 ml of protoplast enzyme mixture in a petri dish. The enzyme mixture consists of 2 % Cellulase RS, 7 mM CaCl₂ x H₂O, 0.7 mM NaH₂PO₄ x H₂O, 3 mM MES (pH 5.6), glucose (550 mOs/kg H₂O of pH 5.6), and is filter sterilized. The mixture is swirled on an orbital shaker at about 50 rpm in dim (< 420 lx) light for about 4 to 5 hours. The digest is then sieved through a stainless steel sieve (100 µm mesh size) and distributed into 12 ml centrifuge tubes which are centrifuged at about 60 to 100 x g for about 5 minutes. The protoplast-containing sediment is then washed three times with protoplast culture medium KM-8p adjusted to 550 mOs/kg H₂O with glucose. At this point a flotation step may be included for further purification of the protoplasts. In this case, the washed protoplasts are layered atop 10 ml of KM-8p culture medium adjusted to 700 mOs/kg H₂O with sucrose. After centrifugation at 60 to 100 x g for about 10 minutes, protoplasts banding at the interface are collected using a fine pipette. Finally, the protoplasts are resuspended in 1 to 2 ml KM-8p culture medium and sieved through a stainless mesh screen (20 µm mesh size). The protoplasts released are collected and washed and resuspended in KM-8p medium for culture or in osmotically adjusted medium suitable for transformation according to the examples below.

### Example 75: Dactylis glomerata L. Protoplast Culture and Growth of Callus

A. The purified protoplasts are plated at a density of about 5 x 10⁵ protoplasts per ml in KM-8p culture medium containing 1.3 % SeaPlaque® agarose (FMC Corp., Marine Colloids Division, Rockland, Maine, USA) and 30 to 40 % of conditioned medium (obtained from 3 to 4 week-old *Dactylis glomerata* L. embryogenic suspension cultures by filtering the medium through a sterile Nalgene® 0.2 µm filter, making the medium 550 mOs/kg H₂O by addition of glucose, and again filter sterilizing). The plates are then placed in the dark at a constant temperature of 28°C. After 10 to 14 days the agarose is cut into wedges and placed into 'bead culture' as described by Shillito et al. (1983) using 20 ml SH-45 suspension culture medium with 3 % sucrose per 3 ml original agarose embedded culture. The plates are put on a platform shaker and agitated at about 50 rpm in light at 670 lx. New suspension cultures are formed as the colonies grow out of the agarose and release cells into the liquid medium. The resultant suspension cultured cells are plated onto agar-solidified SH-30 medium and placed in the dark at 25°C until callus is formed.

B. Protoplasts are cultured as described above except that the culture media contains no conditioned medium.

### Example 76: Transformation of Dactylis glomerata L. Protoplasts by Means of Electroporation

A. Immediately after purification of the protoplasts, electroporation is performed according to Shillito et al. (1985) using linearized plasmid. The protoplasts are resuspended after the last wash at a density of about 7 x 10⁶ protoplasts per ml in the electroporation buffer (0.4 M mannitol, 6 mM MgCl₂). The protoplasts are placed in 0.7 ml aliquots in 10 ml plastic centrifuge tubes. Plasmid DNA and sonicated calf thymus DNA (Sigma) to give final concentrations of 10 µg/ml and 50 µg/ml respectively is added to the tubes. Then 0.38 ml PEG solution [24 % PEG 6000 in 0.4 M mannitol, 30 mM MgCl₂, 0.1 % MES (pH 5.6)] is added and the solution gently mixed. The protoplast suspension is transferred into the chamber of a Dialog® Electroporator and 10 pulses of 3250 Vcm⁻¹ initial voltage and exponential decay constant of 10 msec applied at 30 sec intervals. The sample is removed from the chamber, and placed in a 10 cm diameter petri dish. 10 ml of KM-8p medium containing 1.2 % SeaPlaque® agarose is added, the protoplasts distributed evently throughout the medium, and the agarose allowed to gel.

B. The above is repeated except that the initial voltage used is 3500 Vcm⁻¹, 4000 Vcm⁻¹, 5000 Vcm⁻¹, 3000 Vcm⁻¹, or 2500 Vcm⁻¹.

### Example 77: Transformation of Dactylis glomerata L. Protoplasts by Treatment with PEG

A. PEG mediated direct gene transfer is performed according to Negrutiu, I. et al., (1987). The DNA used is linearized plasmid described.

The protoplasts are suspended following the last wash in 0.5 M mannitol containing 15 mM MgCl₂ at a density of about 2 x 10⁶ per ml. The protoplast suspension is distributed as 1 ml aliquots into 10 ml plastic centrifuge tubes. The DNA is added as described above, and then 0.5 ml of the PEG solution added (40 % PEG 4000 in 0.4 M mannitol, 0.1 M Ca(NO₃)₂, pH 7.0).

The solutions are mixed gently and incubated for 30 minutes at room temperature (about 24°C) for 30 minutes with occasional shaking. 1.4 ml of the wash solution is then added, and the contents of the tube gently mixed. The wash solution consists of 87 mM mannitol, 115 mM CaCl₂, 27 mM MgCl₂, 39 mM KCl, 7 mM Tris-HCl and 1.7 g/l myo-inositol, pH 9.0. Four further 1.4 ml aliquots of wash solution are added at 4 minute intervals, with mixing after each addition. The tube is then centrifuged at about 60 x g for about 10 minutes, and the supernatant discarded. The sedimented protoplasts are taken up in 1 ml KM-8p culture medium, and placed in a 10 cm petri dish. 10 ml of KM-8p medium containing 1.2 % SeaPlaque® agarose is added. The protoplasts are evenly distributed throughout the medium, and the agarose allowed to gel.

B. This is repeated with one or more of the following modifications:
(1) The pH of the wash solution is adjusted to 5.6 or 7.0.
(2) The PEG used is PEG of MW 6000, PEG of MW 2000 or PEG of MW 8000.
(3) The wash medium consists of 154 mM NaCl, 125 mM CaCl₂, 5 mM KCl, 5 mM glucose, pH to 6.0 with KOH, of 0.2 M CaCl₂, 0.1 % MES, pH 6.0 with KOH, or of 0.2 M CaCl₂, 7 mM Tris/HCl, pH 9.0 with KOH.

### Example 78: Transformation of Dactylis glomerata L. Protoplasts by Electroporation or PEG Treatment

Transformation is carried out as described above except that the protoplasts are treated at 45°C for about 5 minutes prior to distribution of the aliquots into tubes for transformation or after distribution of the aliquots, and before addition of the PEG.

### Example 79: Selection of Transformed Colonies

A. The culture plates (petri dishes) containing the protoplasts are incubated for 10 days in the dark at about 25°C and then cut into 5 equal slices for 'bead cultures' (Shillito et al., 1983). Four of the slices are placed each into 20 ml SH-45 culture medium with 4 g/l casein hydrolysate and 20 µg/ml hygromycin B. The fifth slice is put into 20 ml of the same medium but without hygromycin B as a non-selected control. After 4 to 5 weeks the putative transformed protoplast-derived cell colonies growing in hygromycin B are cut out of the agarose and placed into a 19 mm petri dish with 2 ml of liquid SH-45 medium containing 20 µg/ml hygromycin B, which is agitated at about 50 rpm on an orbital shaker. After another 4 to 5 weeks all colonies which grow to make new suspensions are transferred into 125 ml Erlenmeyer flasks and grown in a manner similar to the parent suspension culture, except that 20 µg/ml hygromycin B is included in the medium.

The new suspensions are subcultured every 1 to 3 weeks using SH-45 medium containing 4 g/l casein hydrolysate and 20 µg/ml hygromycin B. Cells from these suspensions are also plated on solidified SH-30 medium containing 20 µg/ml hygromycin B and incubated at about 25°C in the dark. Calli grown from the plated cells are subcultured every two weeks onto fresh medium. The cells which grow in the presence of hygromycin B are presumed to be transformants.

B. Selection is carried out as described except that the protoplast-derived cell colonies growing in hygromycin B containing medium are placed on agar plates of SH-30 medium containing 20 µg/ml hygromycin B and incubated at about 25°C in the dark.

### Example 80: Regeneration of Transformed Dactylis glomerataL, Plants

A. *Dactylis glomerata* L. callus (obtained as described) derived from protoplasts is grown on solidified SH-30 medium, and subcultured every two weeks. Any embryos which form are removed and plated on germination medium (SH-0) and placed in the light (3800 to 4600 lx). Germination of these embryos occurs in 1 to 4 weeks and the resultant plantlets are placed on SH-0 medium in the light to form root systems. They are moved into the greenhouse at the six to twelve leaf stage, and hardened off gradually.

B. Callus (obtained as described) derived from protoplasts is grown on SH-0 medium solidified with 0.24 % Gelrite® in the light (3800 to 4600 lx), and subcultured every two weeks. The resultant plantlets are placed on a 1:1 mixture of SH-0 and OMS media solidified with a combination of 0.12 % Gelrite® and 0.4 % agar in the light to form root systems. They are moved to the greenhouse at the six to twelve leaf stage, and hardened off gradually.

C. Small plantlets are obtained as described in Examples 44A and 44B, and are placed on OMS medium solidified with 0.8 % agar in the light to form root systems. They are moved to the greenhouse at the six to twelve leaf stage, and hardened off gradually.

D. Small plantlets are obtained as described in Example 44A above and are placed on a 1:1 mixture of SH-0 and OMS media solidified with a combination of 0.12 % GelRite® and 0.4 % agar in the light to form root systems. They are moved to the greenhouse at the six to twelve leaf stage, and hardened off gradually.

### Example 81: Introduction of DNA into Protoplasts of N. tabacum by Treatment with PEG

A. Preparation of protoplasts of *N. tabacum* can be carried out in accordance with Paszkowski et al. (1984); GB 2 159 173, EP 0 129 668; Shillito and Potrykus (1987) or by other methods known in the art.

B. DNA is introduced into protoplasts by a modification of the method of Negrutiu et al. (1987). The protoplasts prepared as described are resuspended following the last washing step in a solution consisting of 0.4 M mannitol, 15 to 30 mM CaCl₂, 0.1 % MES at a density of 1.6 to 2 x 10⁶ per ml. The protoplast suspension is distributed as 0.5 ml aliquots into 10 ml plastic centrifuge tubes. The DNA is added in 10 µl sterile distilled water, sterilized as described by Paszkowski et al. (1984), and then 0.5 ml of the PEG solution (40 % PEG MW 8000 in 0.4 M mannitol, 0.1 M Ca(NO₃)₂, pH 7.0) is added. The solutions are mixed gently and incubated for 30 minutes at room temperature (about 24°C) with occasional shaking. 1 ml of the wash solution is then added, and the contents of the tube gently mixed. The wash solution consists of 154 mM NaCl, 125 mM CaCl₂, 5 mM KCl, 5 mM glucose, pH to 6.0 with KOH. Further aliquots of 2 ml, 3 ml and 4 ml of wash solution are added sequentially at 5 minute intervals, with mixing after each addition. The tube is then centrifugated at about 10 to 100 x g for about 10 minutes, and the supernatant discarded. The pelleted protoplasts are taken up in sufficient K3 culture medium with 0.3 M glucose as the osmoticum, and no sucrose, to achieve a final density of 10⁵ per ml and cultured in a 10 cm petri dish.

C. The above is repeated with one or more of the following modifications:
(1) The pH of the wash solution is adjusted to 5.6 or 7.0.
(2) The PEG used is PEG with a MW of 4000.
(3) The wash medium consists of 0.2 M CaCl₂, 0.1 % MES, pH 6.0 with KOH, or of 0.2 M CaCl₂, 7 mM Tris/HCl, pH 9.0 with KOH.
(4) 50 µg of sheared calf thymus DNA in 25 µl sterile water is added together with the plasmid DNA.
(5) The plasmid DNA is linearized before use by treatment with an appropriate restriction enzyme (e.g. BamHI).

### Example 82: Introduction of DNA into Protoplasts of N. tabacum by Electroporation

A. Introduction of DNA into protoplasts of *N. tabacum* is effected by treatment of the protoplasts with an electric pulse in the presence of the appropriate DNA, in a modification of the methods of Fromm et al. (1987); and Shillito and Potrykus (1987).

Protoplasts are isolated as described. The protoplasts are resuspended following the last wash in the following solution: 0.2 M mannitol, 0.1 % MES, 72 mM NaCl, 70 mM CaCl₂, 2.5 mM KCI, 2.5 mM glucose, pH to 5.8 with KOH, at a density of 1.6 to 2 x 10⁶ per ml. The protoplast suspension is distributed as 1 ml aliquots into plastic disposable cuvettes and 10 µg of DNA added as described. The resistance of the solution at this point when measured between the electrodes of the 471 electrode set of the electroporation apparatus described below is in the range of 6 Ω.

The DNA is added in 10 µl sterile distilled water, sterilized as described by Paszkowski et al. (1984). The solution is mixed gently and then subjected at room temperature (24 to 28°C) to a pulse of 400 Vcm⁻¹ with an exponential decay constant of 10 ms from a BTX-Transfector 300 electroporation apparatus using the 471 electrode assembly. The protoplasts are left undisturbed for 5 minutes, and then placed in a petri dish and K3 medium as described and added to bring the density of protoplasts to 10⁵ per ml.

B. The above is repeated with one or more of the following modifications:
(1) The voltage used is 200 Vcm⁻¹, or between 100 Vcm⁻¹ and 800 Vcm⁻¹.
(2) The exponential decay constant is 5 ms, 15 ms or 20 ms.
(3) 50 µg of sheared calf thymus DNA in 25 µl sterile water is added together with the plasmid DNA.
(4) The plasmid DNA is linearized before use by treatment with an appropriate restriction enzyme (e.g. BamHI).

### Example 83: Introduction of DNA into Protoplasts of Zea mays Line 2717

Protoplasts of maize inbred line Funk 2717 are prepared as described in Examples 67 to 69 and resuspended in either of the solutions described for resuspension of the *N. tabacum* protoplasts above at a density of 10⁷ per ml. Trans- formation is carried out essentially as described above. The protoplasts are cultured following transformation at a density of 2 x 10⁶ per ml in KM-8p medium with no solidifying agent added and containing 1 mg/l 2,4-D.

### Example 84: Introduction of DNA into Protoplasts of Sorghum.bicolor

Protoplasts of sorghum suspension FS 562 are prepared essentially as described for *Zea mays* above, and resuspended in either of the solutions described for resuspension of the *N. tabacum* protoplasts above at a density of 107 per ml. Transformation is carried out. The protoplasts are cultured following transformation at a density of 2 x 106 per ml in KM-8p medium, with no solidifying agent added.

### Example 85: Introduction of DNA into Protoplasts of N. plumbaginifolia, Petunia hybrida and Lolium multiflorum

Protoplasts of *N. plumbaginifolia, P. hybrida* or *L. multiflorum* are prepared as described in Shillito and Potrykus (1987) and treated as described above. They are cultured in the medium described by Shillito and Potrykus (1987) without addition of agarose or any other gelling agent.

### Example 86: Introduction of DNA into Protoplasts of Glycine max

Protoplasts of *Glycine max* are prepared by the methods as described by Tricoli et al. (1986), or Chowhury and Widholm, (1985), or Klein et al. (1981). DNA is introduced into these protoplasts essentially as described above. The protoplasts are cultured as described in Klein et al. (1981), Chowhury and Widholm (1986) or Tricoli et al. (1986) without the addition of alginate to solidify the medium.

### SECTION 8. ANALYSIS OF TRANSGENIC PLANTS

In the previous sections, the creation of transgenic plants expressing chimeric disease resistance genes has been described. In this section the development of transgenic seed lines and characterization of those lines with respect to chimeric gene expression is explained. Essentially, this characterization process comprises a preliminary screening of the transgenic plants for expression of the chimeric gene, segregation of the chimeric gene into stable homozygous lines and further characterization of the gene expression.

### Example 87: Development of transgenic T3 seed lines

Genotype designations for transgenic plants are used herein according to the following convention: the initial plant resulting from a transformation event and having grown from tissue culture is designated a T1 plant. Plants resulting from self pollination of the natural flowers of the T1 plant, are designated T2, having acquired a new genotype during the normal meiotic process. Likewise, seeds borne from self-pollination of the natural flowers of T2 plants (i.e. grown from T2 seed) are designated T3, etc.

Transgenic plants (T1) are grown to maturity. Flowers are allowed to self-pollinate and seed pods are collected after normal dessication. Seeds from each individual plant are collected and stored separately. Each seed lot is tested by genetic segregation analysis to determine the number of Mendelian loci bearing the kanamycin resistance trait. T2 seeds are surface-sterilized by multiple washing in 2 % hypochlorite containing 0.02 % (v/v) Tween-20, followed by rinses in sterile water. Approximately 150 of the seeds are placed on filter paper saturated with 0.2 X MS salts (Murashige and Skoog, 1962) containing 150 µg/ml kanamycin. Following germination and expansion of the cotyledons to approximately 5 mm, the ratio of normal-green (*kan-r*) versus bleached (*kan-s*) cotyledons is determined. Only those T2 seed lots exhibiting an approximately 3:1 (*kan-r*:*kan-s*) ratio are kept for further analysis; this segregation ratio is indicative of a single Mendelian locus bearing the kanamycin marker gene.

Four to ten plants are grown to maturity from each T2 seed lot (using the same conditions described above), and are allowed to self-pollinate. T3 seed collection, seed sterilization, and seed germination are as described above for the T2 seed. T3 seed lots in which 100 % of the tested seeds (n = 150) exhibit the kan-r phenotype are assumed to be homozgous for the trait (i.e. resulting from a homozygous T2 parent plant) and are kept for phenotypic analysis.

### Example 88: Assays for the analysis of transgenic plant tissue expressing sense or anti-sense PR-1

The expression of PR-1a in either sense or anti-sense orientation is assayed in transgenic plant material using either an ELISA assay for PR-1a protein or a primer extension assay for PR-1 mRNA as described.

A. ELISA for PR1 protein. Assays are performed in Immunolon II microtiter plates (Dynatech) which have been rinsed with ethanol and allowed to air dry. Tobacco leaf material is ground with a plastic tissue homogenizer (Kontes) in a buffer consisting of 50 mM Tris-HCl, pH 8.5, 200 mM 2-mercaptoethanol, 2 mM PMSF (Sigma), 2mM BAM (Sigma), 10 mM ACA (Sigma), and 0.048 % Leupeptine (hemisulfate salt) (Sigma); 3 ml of extraction buffer are used per gram of leaf tissue. A sufficient sample of healthy-leaf (untreated tobacco) extract is made so that a 1/10 dilution of this extract can serve as a diluent for all the other samples. Extracts are centrifuged in a microcentrifuge in 1.5 ml polypropylene tubes at 12,000 x g (max) for 15 minutes to remove debris. Wells are coated with a solution of a monoclonal antibody specific for PR1 protein (tobacco). Following washing the wells are blocked for 30 to 120 minutes with a solution of 1 % BSA and then washed again. The unknown samples and the standard curve samples, diluted to appropriate concentrations in a 1/10 solution of healthy-plant extract, are added to the wells and incubated for 1 hour at 37°C. (A standard curve is performed using highly purified PR-1a protein). Following washing, a rabbit polyclonal antiserum (5 µg/ml) specific for PR1 is added to the wells and incubated for an additional hour at 37°C, and then the wells are washed again. A goat anti-rabbit IgG antibody (133 ng/ml), to which is conjugated the indicator enzyme alkaline phosphatase (Promega), is added and the indicator reaction is developed according to the manufacturer's recommendations. The reaction is stopped after 30 minutes by the addition of NaOH and the absorbance is read at 405 nm.

B. Primer extension assay for PR1. RNA is extracted from tobacco leaf tissue by a method previously described (Ecker and Davis, 1987). Primer extension assays are performed as described in Example 6 using a synthetic oligonucleotide of the sequence

5'GTAGGTGCATTGGTTGAC3'.

The complement of this sequence occurs in both PR-1a and PR-1b mRNA, resulting in priming of both types of mRNA in the assay. The primer extension products of the chimeric gene product are distinguishable from the products of the endogenous PR-1 genes using PAGE. The chimeric PR-1a transcript generated from the tobacco RUBISCO small subunit promoter of pCGN1509 derivatives results in a primer extension product which is 90 bp longer than that of the endogenous PR-1a gene, while the chimeric PR-1b transcript generated from the tobacco RUBISCO small subunit promoter of pCGN1509 derivatives produces a primer extension product 95 bp longer than that of the endogenous PR-1b gene. The chimeric PR-1a transcript generated from the double 35S promoter of pCGN1761 derivatives yields a product 4 bp longer than that of the endogenous PR-1a gene, while the chimeric PR-1b transcript generated from the double 35S promoter of pCGN1761 derivatives yields a product 10 bp longer than that of the endogenous PR1b gene.

### Example 89: Analysis of seed lines derived from transformation of tobacco with pCGN1755 and pCGN1756 series vectors (RUBISCO SSU/PR-1a or PR-1b/ocs 3')

A leaf tissue sample is taken from T1 plants transformed with either: pCGN1754 or pCGN1760 (as empty cassette controls); one of the pCGN1755 binary vector series (SSU promoter/PR-1a in all orientations); or one of the pCGN1756 binary vector series (SSU promoter/PR-1b). The expression of PR-1 protein in this tissue is determined by ELISA for PR-1 protein and in some cases the RNA level is monitored by primer extension assay.

It is predicted that tissue transformed with the control plasmids, pCGN1754 and pCGN1760 (empty cassette) will result in a certain basal level of PR-1 protein which would be due to endogenous synthesis. Tissue transformed with the plasmids pCGN1755A, pCGN1755C, pCGN1756A or pCGN1756C (PR-1a or PR-1b in a sense orientation) should produce plants expressing PR-1 at a level significantly higher than the basal level. Tissue transformed with pCGN1755B, pCGN1755D, pCGN1756B or pCGN1756D (PR-1a or PR-1b in an anti-sense orientation) should produce significantly lower levels of PR-1 protein relative to the control. When the transformed T1 tissue is screened for PR-1 protein by ELISA those plants which conform to the expectation are promoted to T2 analysis. The intent in this screening is to eliminate transformants that do not cotransform the chimeric PR-1 gene with the antibiotic resistance gene. Many plants from each transformation do conform to the expectation and the protein result is confirmed by primer extension analysis of the RNA to make sure that the higher level of expression in sense plants is due to chimeric gene expression.

The assays are repeated at the T2 generation and at this point several lines are chosen for further characterization. The lines are chosen for based on: (1) a 3:1 segregation of the antibiotic resistance, which indicates a Mendelian inherited (single insertion event) trait; (2) high levels of expression of PR-1 for sense construct, low levels for anti-sense constructs and intermediate levels for control plants. The lines chosen for further study and the data for PR-1 expression and segregation are shown below.

| Seed Line | T2 Segregation Analysis (% *Kan-R*) | T2 PR-1 expression ELISA (ng/ml) |
|---|---|---|
| 1754-12 | 77% | 300 |
| 1755A-4 | 75% | 175 (Av=515 in T3) |
| 1755B-2 | 83% | ≤ 2 |
| 1755B-3 | 76% | ≤ 2 |
| 1760-1 | 93% | 240 |

The seed line nomenclature is designed such that the transforming plasmid is designated first and the individual transformant is designated second. For example, 1755A-4 represents a transgenic plant resulting from transformation with the plasmid pCGN1755A and this is the fourth individual transformant selected. In many of these experiments the control level of PR-1 expression seems artificially high (i.e. 1754-12 above at a level of 300 ng/ml is about 30 times higher than normal). This level decreases in the control, but not in the experimental plants with sucessive generations.

The T2 seed lines above are mixed genotypes in respect to the chimeric PR-1 gene. For instance, some of the plants from a seed line are homozygous and some are heterozygous for the trait. In order to isolate homozygous seed lines, between four and ten plants of each line are allowed to self pollinate and set seed. This seed is collected and segregation is determined as explained above. These segregation data for several lines is shown below.

| T3 Seed Line | Segregation (% *Kan-R*) |
|---|---|
| 1754-12-10 | 100 |
| 1755A-4-2 | 100 |
| 1755B-2-1 | 100 |
| 1755B-3-1 | 100 |
| 1760-1 | ND |

These homozygous seed lines are then analyzed for generalized disease resistance as described below. The general conclusion from the analysis of this series of plants is that the sense constructs (both PR-1a and PR-1b) produce 6 to 150 times the amount of PR-1 in healthy tobacco tissue and anti-sense constructs usually produce much less PR-1 than in healthy tobacco.

### Example 90: Analysis of seed lines derived from transformation of tobacco with pCGN1764, pCGN1765, pCGN1774 and pCGN1775 series of - vectors (Double CaMV 35S promoter/PR-1a)

The development of seed lines in this example include the transformations of tobacco with the double CaMV 35S promoter linked to PR-1a (pCGN1764 and pCGN1774 series) in sense and anti-sense orientation and the double CaMV 35S promoter linked to PR-1b (pCGN1765 and pCGN1775 series) in sense and anti-sense orientation. The difference between the pCGN1764/pCGN1765 and pCGN1774/pCGN1775 constructs is that the binary vector is different (see relevant examples above). Empty cassette controls for pCGN1764 and pCGN1765 are pCGN1766 and pCGN1767. The empty cassette control for pCGN1774 and pCGN1775 is pCGN1789.

PR1 protein expression in the "sense" T1 plants (all events) range from undetectable levels up to approximately 13,000 ng/ml extract; this maximum level is within two fold of the levels seen in a highly infected primary leaf bearing many lesions. The levels seen in secondary tissue, even under optimal conditions, is several fold lower than this. The average expression level for all the "sense" T1 plants is approximately, 4,200 ng/ml, which is more than 20-fold higher than the average for the small subunit-PR1 transgenic plants.

No significant differences are seen in the expression levels of the chimeric gene between the pCGN783 binary vector (pCGN1764 and pCGN1765 series). Both sets of plants have a wide range of expression levels, as is common in transgenic experiments of this type. The highest expression in both types is similar, and the number of plants expressing at a low level is about the same. The average for the pCGN783 binary plants is higher than the average for the small subunit-PR1 transgenic plants.

No significant differences are seen in the expression levels of the chimeric gene between the pCGN783 binary vector (pCGN1764 and pCGN17065 series) and the pCGN1540 binary vector (pCGN1774 and pCGN1775 series). The average for the pCGN783 binary plants is 3,955 ng/ml, and for the pCGN1540 binary plants is 4,415 ng/ml, but considering the variation, this difference is not significant. Similarly, the orientation of the genes in the binary has no major effect on expression. The "C" orientation (head to tail) gives three of the four highest expressing plants, but it also gives more low level expressing plants. The "A" (head to head) orientation plants tend to group more in the moderate expression range, but again the variation and the small sample size prevent the attachment of any statistical significance to these differences. Primer extension analysis of a limited number of samples shows that the chimeric gene mRNA is the dominant or the only PR1 mRNA present.

The conclusion from the T1 data is that the level of PR-1 protein in plants transformed with double CaMV 35S promoter/PR-1a or PR-1b sense constructs is several hundred fold higher than the level of control plants. The level of expression of PR-1 in plants transformed with the anti-sense construct is very low. The binary vector used for transformation (pCGN783 or pCGN1540) does not significantly effect the level of PR-1 expression in the transgenic plants. Likewise, the orientation of the expression cassette within the vector has no significant effect on the level of PR-1 expression. Therefore, one line is selected that produces high levels of PR-1a due to sense expression and one is selected that produces low levels of PR-1a due to anti-sense expression for further development. A control line with an empty cassette is included. The results of PR-1 expression and antibiotic segregation for the selected lines is shown below.

| T2 Seed Line | Segregation Analysis (% Kan-R) | T2 PR-1 expression ELISA (ng/ml) |
|---|---|---|
| 1774A-10 | 409/553 | 9000 |
| 1774B-3 | 109/142 | ≤ 2 |
| 1789-10 | 371/459 | 5.6 |

Homozygous T3 seed lines are generated from each of the selected lines as described in the previous example. The results of segregation analysis are shown below.

| T3 Seed Line | Segregation (% Kan-R) |
|---|---|
| 1774A-10-1 | 100 |
| 1774B-3-2 | 100 |
| 1789-10-3 | 100 |

These homozygous seed lines are evaluated for PR-1 expression and disease resistance as described below.

### Example 91: Analysis of seed lines derived from transformation of tobacco with the pCGN1779 plasmid series (Double CaMV 35S - promoter/cucumber chitinase/lysozyme)

A leaf tissue sample is taken from T1 plants transformed with either of the binary vectors pCGN1779C or pCGN1779D. The cucumber chitinase/lysozyme protein content is determined using an ELISA assay essentially as described above except that the monoclonal and polyclonal antibodies are directed against the cucumber chitinase/lysozyme protein.

Eight of thirteen T1 "sense" plants produce very high amounts (>10,000 ng/ml extract) of the cucumber chitinase foreign gene product. Again a wide range, from undetectable up to 31,500 ng/ml extract, is observed, with an average of 12,500 ng/ml extract.

The conclusion from the T1 data is that the transformed T1 plants produce several thousand times more of the transgenic protein than is present in control plants. T3 seed lines are derived from the high expressing T1 plants as described in Example 87 and these T3 seed lines maintain their high levels of chitinase/lysozyme expression.

### Example 91B: Analysis of seed lines derived from transformation of tobacco with the pCGN1782 plasmid series (Double CaMV 355 promoter/tobacco basic chitinase)

A leaf tissue sample is taken from T1 plants transformed with either of the binary vectors pCGN1782C or pCGN1782D. The tobacco basic chitinase protein content is estimated by an immunoblot technique (Towbin et al., 1979) as modified by Johnson et al. (1984), following SDS-PAGE (Laemmli, 1970). The antibodies used are raised against the tobacco basic chitinase protein by standard methods and are specific for the tobacco basic chitinase protein. T1 plants with the pCGN1782C plasmid (containing the sense expression cassette) showing high levels of expression relative to control and anti-sense plants, are advanced to T3 seed lines as described in Example 87. Homozygous T2 plants which yield these T3 seed continue to express the protein at high levels. T1 plants transformed by the pCGN1782D plasmid (containing the anti-sense expression cassette) which give low levels of expression are also advanced to T3 seed as described in Example 87.

### Example 91C: Analysis of seed lines derived from transformation of tobacco with the pCGN1781 plasmid series (Double CaMV 35S promoter/tobacco basic glucanase)

A leaf tissue sample is taken from T1 plants transformed with either of the binary vectors pCGN1781C or pCGN1781D. The tobacco basic glucanase protein content is estimated by an immunoblot technique as described in Example 91B. The antibodies used are raised against the tobacco basic glucanase protein by standard methods and are specific for the tobacco basic glucanase protein. T1 plants with the pCGN1781C plasmid (containing the sense expression cassette) showing high levels of expression relative to control and anti-sense plants, are advanced to T3 seed lines as described in Example 87. Homozygous T2 plants which yield these T3 seed continue to express the protein at high levels. T1 plants transformed by the pCGN1781D plasmid (containing the anti-sense expression cassette) which give low levels of expression are also advanced to T3 seed as described in Example 87.

### SECTION 9. EVALUATION OF PHENOTYPE

The development of stable, transgenic seed lines of tobacco which express chimeric PRP genes in sense and anti-sense orientation is explained in Section 1 to 8. Once the seed lines are developed they are evaluated quantitatively for resistance to various diseases.

### Example 92: Evaluation of transgenic tobacco expressing PR-1 in a sense and anti-sense orientation for disease resistance

The seed lines 1755A-4-2 and 1755B-2-1 are analyzed for resistance to TMV. The result of these experiments is that there is no significant difference in lesion size or lesion number due to either elevated or depressed levels of PR-1 protein.

The seed lines 1755A-4-2 and 1755B-2-1 are analyzed for resistance to the fungal pathogen *Peronospora nicotiana* (Bluemold egg downy mildew) by spraying a spore suspension on the leaves of the plants and incubating under standard conditions for seven days. The plants are then scored for resistance to bluemold based on the percentage of leaf surface area infected by the pathogen. Six plants of the 1755A-4-2 line which are expressing an average of 1454 ng/ml PR-1 protein are showing 98 ± 3 % infected surface area. Six plants of the 1755B-2-1 line, which are expressing an average of 370 ng/ml PR-1 protein are showing 45 % ± 26 % infected surface area. Six plants derived from untransformed Xanthi.nc tobacco which are producing 559 ng/ml PR-1 protein are showing 99 % ± 1 % infected surface area. This result indicates that the anti-sense expression of PR-1a results in a significant and valuable resistance to downy mildew in transgenic plants.

### BIBLIOGRAPHY

**Adelman et al.,** DNA 2: 183-193 (1983)
**Allen, G.,** "Sequencing of proteins and peptides, in: Laboratory Techniques in Biochemistry and Molecular Biology, Vol. 9, ed. T.S. Work and R.H. Bordon, Elsevier, North-Holland Biomedical Press, Amsterdam (1981)
**Alexander**, Methods in Enzymology 154:41-64 (1987)
**Barker et al.,** Plant Mol. Biol. 2: 335-350 (1983)
**Bevan, M.W. and Chilton, M.-D.,** Ann. Rev. Genet. 16, 357 (1982)
**Bolivar et al.,** Gene 2: 95-113 (1977)
**Bolivar**, Gene 4: 121-136 (1978)
**Buckley**, PH.D. Thesis, UC San Diego 1985
**Chang and Cohen,** J. Bacteriol. 134: 1141-1156 (1978)
**Chowhury, V.K. and Widholm, J.M.,** Plant Cell Reports 4, 289-292 (1985)
**Chu et al.,** Scientia Sinica 18, 659 (1975)
**Cornelissen, B.J.C. et al.,** EMBO J., Vol. 5(1), 37 (1986)
**Currier and Nester**, J. Bact. 126: 157-165 (1976)
**de Block**, M. et al., EMBO J 6, 2513 (1987)
**de Greve et al**., J. Mol. Appl. Genet. 1: 499-512 (1982)
**Ecker and Davis**, Proc. Natl. Acad. Sci., USA 84: 5203-5206 (1987)
**Evans D.A. and Bravo et al.**, in: Handbook of Plant Cell Culture, Vol. 1, MacMillan Publ. Co., 1983, p. 124
**Facciotti et al.**, Bio/Technology 3: 241-246 (1985)
**Facciotti and Pilet**, Plant Science Letters, 15: 1-7 (1979)
**Fromm, M.E. et al.**, In: Methods in Enzymology, eds. Wu, R. and Grossman, L. **Academic Press,** Orlando, Florida, Vol. 153, 307, (1987)
**Gamborg, O.L. et al.,** Experimental Cell Research 50, 151-158 (1968)
**Gardner et al.,** Nucl. Acids Res. 9: 2871-2888 (1981)
**Garfinkel and Nester,** J. Bact. 144: 732-743 (1980)
**Gray, D.J. and Conger et al.,** Plant Tissue Organ Cult., 4, 123-133 (1985)
**Gubler, U. and Hoffman, B.J.,** Gene 25, 263 (1983)
**Glover, D.M.,** DNA Cloning, IRL Press, Oxford (1985)
**Guiseley and Renn**, "The Agarose Monograph", Marine Colloids Division FMC Corp., (1975)
**Gyllensten, U. and Erlich,** H. Proc. Natl. Acad. Sci. USA, Vol. 85, 7652-7656 (1988)
**Hall et al.,** PNAS USA 75:3196-3200 (1978)
**Hanahan, D.,** J. Mol. Biol. 166, 557-580 (1983)
**Hanning, G.E. and Conger et al.,** Theor. Appl. Genet., 63, 155-159 (1982)
**Hirsch and Beringer,** Plasmid 12: 139-141 (1984)
**Ho, S. et al.,** Gene 77, 51-59 (1989)
**Holsters et al.,** Mol. Gen. Genet. 163: 181-187 (1978)
**Hohn and Collins,** Gene 11: 291-298 (1980)
**Horsch, R. et al.,** Science 227, 1229-1232 (1985)
**Johnson, D. et al.,** Gene Anal. Techn., Vol. 1: 3-8 (1984)
**Jorgensen et al.**, Mol. Gen. Genet. 177: 65 (1979)
**Jouanin et al.,** Mol. Gen. Genet. 201: 370-374 (1985)
**Kao K.N. and Michayluk et al.,** Planta 126, 105-110 (1975)
**Klein, T.M. et al.,** Nature 327, 70 (1987)
**Klein, A.S. et al.,** Planta 152, 105-114 (1981)
**Knauf and Nester**, Plasmid 8: 45 (1982)
**Lagrimini, L.M. et al.**, Proc. Natl. Acad. Sci. USA 84, 7542 (1987)
**Lucas, J. et al.**, EMBO J. 4, 2745 (1985)
**Laemmli, E.**, Nature, Vol. 227: 680-685 (1970)
**Maniatis, T. et al.**, Molecular Cloning, Cold Spring Harbor Laboratory, New York (1982);
**Matsudaira, P., J.** Biol. Chem., vol261, 10035-10038 (1987)
**Messing et al.**, Nucl Acids Res. 9: 309-321 (1981)
**Methods in Enzymology,** Volumes 68, 100, 101 and 118 (1979, 1983 and 1986);
**Metraux, J.P. et al.,** Physiological and Molecular Plant Pathology, 33, 1-9 (1988)
**Miller, J.H.,** Experiments in Molecular Genetics, Cold Spring Harbor Laboratory, Cold Spring Harbor (1972)
**Murashige T. and Skoog, F.,** Physiologia Plantarum 15, 473 (1962)
**Negrutiu, I. et al.,** Plant Mol. Biol. 8, 363 (1987)
**Norrander et al.,** Gene 26: 101-106 (1983)
**Okayama and Berg, Mol.** and Cell Biol. 2:161-170 (1982)
**O'Neal et al**., Nucl. Acids Res. 15: 8661-8677 (1987)
**Parent, J.G. and Asselin**, Can. J. Bot. 62, 564 (1984)
**Paszkowski, J. et al.,** EMBO J. 3, 2717 (1984)
**Pfitzner U.M. et al.,** Nucl. Acid. Res., 15(11), 4449 (1987)
**Pierpoint, W.S. et al.,** Physiol. Plant Pathol. 31, 291 (1987)
**Potrykus, I. et al.,** Mol. Gen. Genet. 199, 169 (1985)
**Redolfi and Neth, J** Plant Pathol 89: 245-254, (1983)
**Reich, T.J. et al.,** Bio/Technology 4, 1001 (1986)
**Richardson M et al.**, Nature 327: 432 (1987)
**Rothstein, et al.,** Gene 53: 153-161 (1987)
**Saiki et al.,** Science 239: 487-491 (1988)
**Sambrook J. et al.,** Molecular Cloning, A Loboratory Manual, 2nd Ed., Cold Spring Harbor **Laboratory** Press (1989)
**Schenk, R.U. et al.,** Can. J. Bot., 50, 199-204 (1972)
**Shinshi et al., Proc.** Natl. Acad. Sci. USA 84, 89-93 (1987)
**Shinshi, H. et al.,** Proc. Natl. Acad. Sci. USA 85: 5541-5545, (1988)
**Shillito, R.D. et al.,** Plant Cell Reports, 2, 244-247 (1983)
**Shillito, R.D. et al.,** Bio/Technology 3, 1099-1103 (1985)
**Shillito, R.D. and Potrykus,** I., In: Methods in Enzymology, eds. Wu, R. and Grossman, L. **Academic Press,** Orlando, Florida, Vol. 153, 313-306, (1987)
**Simpson, R.J. and Nice,** Biochem. Intl. 8, 787 (1984)
**Smith, J.A.,** PH.D. Thesis, Department of Botany and Plant Pathology, Michigan State University **Lansing,** Michigan (1988)
**Stahl et al.,** Nuc. Acids Res. 16: 3026-3038 (1988)
**St. John and Davis,** Cell, 16: 443-452 (1979)
**Stratagene Lambda** Zap laboratory manual, Stratagene, San Diego, USA
**Thomashow et al.,** Cell 19: 729-739 (1980)
**Towbin, H. et al.,** Proc. Natl. Acad. Sci. USA, Vol. 76: 4350-4354 (1979)
**Tricoli, D.M.** et al., Plant Cell Reports, 5, 334-337 (1986),
**Vaeck, M. et al., Nature** 328, 33 (1987)
**van Loon, Plant Mol. Biol 4:** 111-116, (1985)
**Vieira and Messing,** Gene 19:259-268 (1982)
**Yamada, Y. et al.,** Plant Cell Reports, 5, 85-88 (1986)
**Yanisch-Perron et al.,** Gene 33: 103-119 (1985)
**Yuen, S.W. et al.,** "Microanalysis of SDS-PAGE Electroblotted Proteins, in: Applied Biosystems User Bulletin No. 36, March 21, 1988

### Patent Literature

EP-A 129 668;
EP-A 223 452
EP-A 240 332
EP-A 0249432
WO 87/07299
US P 4,795,855
GB 2 159 173

## Claims

1. A method for producing a plant having a disease-resistant phenotype, comprising:
(a) transforming plant tissue or cells with a chimeric DNA sequence comprising a first DNA sequence comprising a promoter which promotes in a plant the constitutive transcription of an associated DNA sequence, operably linked to a second DNA sequence comprising a coding sequence encoding an inducible plant pathogenesis-related protein, wherein the said pathogenesis-related protein is not a chitinase;
(b) constitutively expressing the pathogenesis-related protein at a level sufficient to provide a disease-resistant phenotype to the plant transformed in accordance with step (a); and
(c) selecting those plants that exhibit a disease-resistant phenotype, wherein said disease is caused by pathogenic fungi.

2. A method according to claim 1, wherein said pathogenic fungi are selected from the group consisting of *Phytophtora* and *Peronospora*.

3. A method according to claim 1, wherein the first DNA sequence is of heterologous origin with respect to the second DNA sequence.

4. A method according to claim 1, wherein the first DNA sequence is prepared from a gene encoding the small subunit of tobacco ribulose bis-phosphate carboxylase (RUBISCO).

5. A method according to claim 1, wherein the first DNA sequence, being prepared from the CaMV genome, comprises double CaMV 35S promoter.

6. A method according to claim 1, wherein the second DNA sequence is obtainable from a DNA library prepared from a plant tissue induced to systemic acquired resistance or localized acquired resistance using biological inducers.

7. A method according to claim 1, wherein the second DNA sequence encodes a plant pathogenesis-related protein selected from the group consisting of PR-1A encoded by Sequence 1, PR-1B encoded by Sequence 2, PR-1C encoded by Sequence 3, PR-R major encoded by Sequence 4, PR-R minor, PR-P encoded by Sequence 6, PR-Q encoded by Sequence 5, PR-2 encoded by Sequence 8, PR-N, PR-O, PR-O' encoded by Sequence 7A, SAR8.2a encoded by Sequence 9, SAR8.2b encoded by Sequence 10, cucumber basic peroxidase encoded by Sequence 12, and tobacco basic glucanase.

8. A method according to claim 1, wherein linking the first and the second DNA sequence has been carried out in a way that the second DNA sequence is oriented with respect to the first DNA sequence such that the first DNA sequence promotes transcription of the sense strand of the second DNA sequence.

9. A method of claim 1, wherein the chimeric DNA sequence further comprises a third DNA sequence which is a plant-selectable gene marker.

10. A transgenic plant including the progeny thereof which has been transformed with a chimeric DNA molecule comprising a promoter which promotes in a plant the constitutive transcription of an associated DNA sequence, operably linked to a coding sequence encoding an inducible plant pathogenesis- related protein, wherein the said pathogenesis-related protein is not a chitinase, such as to constitutively express a pathogenesis-related protein at a level sufficient to provide a disease-resistant phenotype, wherein said progeny comprises said chimeric DNA molecule, wherein said disease is caused by pathogenic fungi.

11. A transgenic plant according to claim 10, wherein said pathogenic fungi are selected from the group consisting of *Phytophtora* and *Peronospora.*

12. A transgenic plant according to claim 10, wherein the plant pathogenesis-related protein is selected from the group consisting of PR-1A encoded by Sequence 1, PR-1B encoded by Sequence 2, PR-1C encoded by Sequence 3, PR-R major encoded by Sequence 4, PR-R minor, PR-P encoded by Sequence 6, PR-Q encoded by Sequence 5, PR-2 encoded by Sequence 8, PR-N, PR-O, PR-O' encoded by Sequence 7A, SAR8.2a encoded by Sequence 9, SAR8.2b encoded by Sequence 10, cucumber basic peroxidase encoded by Sequence 12, and tobacco basic glucanase.

13. A transgenic plant according to any one of claims 11 or 12, which is a plant selected from the group consisting of tobacco, carrot, sunflower, tomato, cotton, sorghum, *Petunia* and *Glycine.*

14. A transgenic plant according to any one of claims 11 or 12, which is a plant selected from the group consisting of *Zea mays, Dactylis* and *Lolium.*

15. A transgenic plant according to claim 14, which is a maize plant.

16. A transgenic plant seed obtained from a plant of any one of claims 10 to 15, wherein said seed comprises said chimeric DNA molecule.

17. A disease-resistant plant produced by a method of claim 1, wherein said disease is caused by pathogenic fungi.

18. A disease-resistant plant according to claim 17, wherein said pathogenic fungi are selected from the group consisting of *Phytophtora* and *Peronospora.*

19. A disease-resistant plant produced by a method of any one of claims 3 to 9, wherein said disease is caused by pathogenic fungi.

20. A disease-resistant plant according to claim 19, wherein said pathogenic fungi are selected from the group consisting of *Phytophtora* and *Peronospora.*

21. A plant tissue constitutively expressing induced levels of plant pathogenesis-related proteins or constitutively expressing levels of pathogenesis-related proteins which provide a disease-resistant phenotype, with the proviso that the pathogenesis-related proteins are of heterologous origin with respect to the plant to be transformed and are not a chitinase, wherein said disease is caused by pathogenic fungi.

22. A plant tissue according to claim 21, wherein said pathogenic fungi are selected from the group consisting of *Phytophtora* and *Peronospora.*

23. A method of protecting a plant against damage caused by a plant disease comprising transforming plant tissue or cells with a chimeric DNA sequence comprising a first DNA sequence which promotes in a plant the constitutive transcription of a second DNA sequence, and a second DNA sequence comprising a coding sequence of an inducible plant pathogenesis-related protein or a coding sequence having substantial sequence homology to a coding sequence of an inducible plant pathogenesis-related protein such that the transformed plant exhibits a disease-resistant phenotype and planting the said transformed plant in an environment, where the disease may occur, wherein the said pathogenesis-related protein is not a chitinase, wherein said disease is caused by pathogenic fungi.

24. A method according to claim 23, wherein the second DNA sequence encodes a plant pathogenesis-related protein selected from the group consisting of PR-1A encoded by Sequence 1, PR-1B encoded by Sequence 2, PR-1C encoded by Sequence 3, PR-R major encoded by Sequence 4, PR-R minor, PR-P encoded by Sequence 6, PR-Q encoded by Sequence 5, PR-2 encoded by Sequence 8, PR-N, PR-O, PR-O' encoded by Sequence 7A, SAR8.2a encoded by Sequence 9, SAR8.2b encoded by Sequence 10, cucumber basic peroxidase encoded by Sequence 12, and tobacco basic glucanase.

25. A method according to claim 23, wherein the pathogenic fungi are those selected from the group consisting of *Phytophtora* and *Peronospora.*

26. A method according to claim 23, wherein the plant to be protected is a plant according to any one of claims 10 to 20.

27. A method according to claim 26 wherein the plant to be protected is a plant selected from the group consisting of tobacco, carrot, sunflower, tomato, cotton, sorghum, *Petunia* and *Glycine*.

28. A method according to claim 27, wherein the plant to be protected is a plant selected from the group consisting of *Zea mays, Dactylis* and *Lolium.*

29. A method according to claim 28, wherein the plant to be protected is a maize plant.

## Patentansprüche

1. Verfahren zur Herstellung einer Pflanze mit einem krankheitsresistenten Phänotyp, umfassend:
(a) Transformieren von Pflanzengewebe oder Zellen mit einer chimeren DNA-Sequenz, die eine erste DNA-Sequenz mit einem Promotor, welcher in einer Pflanze als Promotor für die konstitutive Transkription einer assoziierten DNA-Sequenz dient und operativ an eine zweite DNA-Sequenz geknüpft ist, welche eine kodierende Sequenz umfasst, die für ein induzierbares, Pflanzenpathogenese-bezogenes Protein kodiert, wobei das Pathogenese-bezogene Protein keine Chitinase ist;
(b) konstitutives Exprimieren des Pathogenese-bezogenen Proteins in einer ausreichenden Menge, um einen krankheitsresistenten Phänotyp der gemäß Schritt (a) transformierten Pflanze bereitzustellen; und
(c) Auswählen derjenigen Pflanzen, die einen krankheitsresistenten Phänotyp zeigen, wobei die Krankheit durch pathogene Pilze hervorgerufen wird.

2. Verfahren nach Anspruch 1, worin die pathogenen Pilze aus der Gruppe aus *Phytophtora* und *Peronospora* ausgewählt sind.

3. Verfahren nach Anspruch 1, worin die erste DNA-Sequenz von heterologem Ursprung in Bezug auf die zweite DNA-Sequenz ist.

4. Verfahren nach Anspruch 1, worin die erste DNA-Sequenz aus einem Gen hergestellt wird, welches für die kleine Untereinheit der Tabak-Ribulose-Bisphosphatcarboxylase (RUBISCO) kodiert.

5. Verfahren nach Anspruch 1, worin die aus dem CaMV-Genom hergestellte erste DNA-Sequenz einen doppelten CaMV-35S-Promotor enthält.

6. Verfahren nach Anspruch 1, worin die zweite DNA-Sequenz aus einer DNA-Bank erhältlich ist, welche aus einem Pflanzengewebe hergestellt ist, das mit Hilfe biologischer Induktoren dazu induziert wurde, eine systemisch erworbene Resistenz oder eine lokal erworbene Resistenz zu entfalten.

7. Verfahren nach Anspruch 1, worin die zweite DNA-Sequenz für ein Pflanzenpathogenese-bezogenes Protein kodiert, welches aus der Gruppe aus: von Sequenz 1 kodiertem PR-1A, von Sequenz 2 kodiertem PR-1B, von Sequenz 3 kodiertem PR-1C, von Sequenz 4 kodiertem PR-R-Haupt, PR-R-Neben, von Sequenz 6 kodiertem PR-P, von Sequenz 5 kodiertem PR-Q, von Sequenz 8 kodiertem PR-2, PR-N, PR-O, von Sequenz 7A kodiertem PR-O', von Sequenz 9 kodiertem SAR8.2a, von Sequenz 10 kodiertem SAR8.2b, von Sequenz 12 kodierter basischer Gurken-Peroxidase und basischer Tabak-Glukanase ausgewählt ist.

8. Verfahren nach Anspruch 1, worin die erste und die zweite DNA-Sequenz in einer Weise verknüpft sind, dass die zweite DNA-Sequenz in Bezug auf die erste DNA-Sequenz so ausgerichtet ist, dass die erste DNA-Sequenz als Promotor für die Transkription vom kodierenden Strang der zweiten DNA-Sequenz dient.

9. Verfahren nach Anspruch 1, worin die chimere DNA-Sequenz ferner eine dritte DNA-Sequenz umfasst, welche ein pflanzenselektierbarer Genmarker ist.

10. Transgene Pflanze, einschließlich die Abkömmlinge davon, welche mit einem chimeren DNA-Molekül, umfassend einen Promotor, welcher in einer Pflanze als Promotor fiir die konstitutive Transkription einer assoziierten DNA-Sequenz dient und operativ an eine kodierende Sequenz geknüpft ist, welche für ein induzierbares, Pflanzenpathogenese-bezogenes Protein kodiert, transformiert wurde,
wobei das Pathogenese-bezogene Protein keine Chitinase ist, so dass in konstitutiver Weise ein Pathogenese-bezogenes Protein in einer ausreichenden Menge exprimiert wird, um einen krankheitsresistenten Phänotyp bereitzustellen,
wobei die Abkömmlinge dieses chimere DNA-Molekül umfassen und die Erkrankung durch pathogene Pilze verursacht wird.

11. Transgene Pflanze nach Anspruch 10, worin die pathogenen Pilze aus der Gruppe aus *Phytophtora* und *Peronospora* ausgewählt sind.

12. Transgene Pflanze nach Anspruch 10, worin das Pflanzenpathogenese-bezogene Protein aus der Gruppe aus: von Sequenz 1 kodiertem PR-1A, von Sequenz 2 kodiertem PR-1B, von Sequenz 3 kodiertem PR-1C, von Sequenz 4 kodiertem PR-R-Haupt, PR-R-Neben, von Sequenz 6 kodiertem PR-P, von Sequenz 5 kodiertem PR-Q, von Sequenz 8 kodiertem PR-2, PR-N, PR-O, von Sequenz 7A kodiertem PR-O', von Sequenz 9 kodiertem SAR8.2a, von Sequenz 10 kodiertem SAR8.2b, von Sequenz 12 kodierter basischer Gurken-Peroxidase und basischer Tabak-Glukanase ausgewählt ist.

13. Transgene Pflanze nach einem der Ansprüche 11 oder 12, welches eine Pflanze ist, die aus der Gruppe aus Tabak, Karotte, Sonnenblume, Tomate, Baumwolle, Sorghum, *Petunie* und *Glycine* ausgewählt ist.

14. Transgene Pflanze nach einem der Ansprüche 11 oder 12, welches eine Pflanze, ausgewählt aus der Gruppe aus *Zea Mays, Dactylis* und *Lolium,* ist.

15. Transgene Pflanze nach Anspruch 14, welches eine Maispflanze ist.

16. Transgener Pflanzensamen, der von einer Pflanze nach einem der Ansprüche 10 bis 15 gewonnen wurde, wobei der Samen das chimere DNA-Molekül enthält.

17. Nach dem Verfahren gemäß Anspruch 1 hergestellte krankheitsresistente Pflanze,
wobei die Erkrankung durch pathogene Pilze verursacht wird.

18. Krankheitsresistente Pflanze nach Anspruch 17, wobei die pathogenen Pilze aus der Gruppe aus *Phytophtora* und *Peronospora* ausgewählt sind.

19. Nach dem Verfahren gemäß einem der Ansprüche 3 bis 9 hergestellte krankheitsresistente Pflanze, worin die Erkrankung durch pathogene Pilze verursacht wird.

20. Krankheitsresistente Pflanze nach Anspruch 19, worin die pathogenen Pilze aus der Gruppe aus *Phytophtora* und *Peronospora* ausgewählt sind.

21. Pflanzengewebe, welches auf konstitutive Weise induzierte Mengen Pflanzenpathogenese-bezogener Proteine exprimiert oder auf konstitutive Weise eine Menge Pathogenese-bezogener Proteine exprimiert, so dass ein krankheitsresistenter Phänotyp bereitgestellt wird, mit der Vorgabe, dass die Pathogenese-bezogenen Proteine von heterologem Ursprung mit Bezug auf die zu transformierende Pflanze sind und keine Chitinase sind, wobei die Erkrankung durch pathogene Pilze verursacht wird.

22. Pflanzengewebe nach Anspruch 21, wobei die pathogenen Pilze aus der Gruppe aus *Phytophtora* und *Peronospora* ausgewählt sind.

23. Verfahren zum Schutz einer Pflanze vor Schädigung durch eine Pflanzenkrankheit, umfassend
Transformieren von Pflanzengewebe oder Zellen mit einer chimeren DNA-Sequenz, welche eine erste DNA-Sequenz enthält, die in einer Pflanze als Promotor fiir die konstitutive Transkription einer zweiten DNA-Sequenz dient, und eine zweite DNA-Sequenz, welche die kodierende Sequenz eines induzierbaren, Pflanzenpathogenese-bezogenen Proteins oder eine kodierende Sequenz mit einer wesentlichen Sequenzhomologie zu der kodierenden Sequenz eines induzierbaren, Pflanzenpathogenese-bezogenen Proteins umfasst, so dass die transformierte Pflanze einen krankheitsresistenten Phänotyp zeigt, und
Anpflanzen der transformierten Pflanze in einer Umgebung, wo die Erkrankung auftreten kann,
wobei das Pathogenese-bezogene Protein keine Chitinase ist und die Erkrankung durch pathogene Pilze verursacht wird.

24. Verfahren nach Anspruch 23, worin die zweite DNA-Sequenz für ein Pflanzenpathogenese-bezogenes Protein, ausgewählt aus der Gruppe aus: von Sequenz 1 kodiertem PR-1A, von Sequenz 2 kodiertem PR-1B, von Sequenz 3 kodiertem PR-1C, von Sequenz 4 kodiertem PR-R-Haupt, PR-R-Neben, von Sequenz 6 kodiertem PR-P, von Sequenz 5 kodiertem PR-Q, von Sequenz 8 kodiertem PR-2, PR-N, PR-O, von Sequenz 7A kodiertem PR-O', von Sequenz 9 kodiertem SAR8.2a, von Sequenz 10 kodiertem SAR8.2b, von Sequenz 12 kodierter basischer Gurken-Peroxidase und basischer Tabak-Glukanase ausgewählt ist.

25. Verfahren nach Anspruch 23, worin die pathogenen Pilze aus der Gruppe aus *Phytophtora* und *Peronospora* ausgewählt sind.

26. Verfahren nach Anspruch 23, worin die zu schützende Pflanze eine Pflanze gemäß einem der Ansprüche 10 bis 20 ist.

27. Verfahren nach Anspruch 26, worin die zu schützende Pflanze eine Pflanze aus der Gruppe aus Tabak, Karotte, Sonnenblume, Tomate, Baumwolle, Sorghum, *Petunie* und *Glycine* ist.

28. Verfahren nach Anspruch 27, worin die zu schützende Pflanze eine aus der Gruppe aus *Zea Mays, Dactylis* und *Lolium* ausgewählte Pflanze ist.

29. Verfahren nach Anspruch 28, worin die zu schützende Pflanze eine Maispflanze ist.

## Revendications

1. Procédé pour la production d'une plante ayant un phénotype résistant à des maladies, comprenant
(a) la transformation de cellules ou de tissu végétaux à l'aide d'une séquence d'ADN chimère comprenant une première séquence d'ADN comprenant un promoteur qui active chez une plante la transcription constitutive d'une séquence d'ADN associée, fonctionnellement liée à une seconde séquence d'ADN comprenant une séquence codante codant pour une protéine inductible en relation avec la pathogenèse de la plante, ladite protéine en relation avec la pathogenèse n'étant pas une chitinase ;
(b) l'expression constitutive de la protéine en relation avec la pathogenèse, à un taux suffisant pour procurer à la plante transformée selon l'étape (a) un phénotype résistant à la maladie ; et
(c) la sélection des plantes qui manifestent un phénotype résistant à la maladie, ladite maladie étant provoquée par des champignons pathogènes.

2. Procédé selon la revendication 1, dans lequel lesdits champignons pathogènes sont choisis dans le groupe constitué par *Phytophtora* et *Peronospora*.

3. Procédé selon la revendication 1, dans lequel la première séquence d'ADN est d'origine hétérologue par rapport à la seconde séquence d'ADN .

4. Procédé selon la revendication 1, dans lequel la première séquence d'ADN est produite à partir d'un gène codant pour la petite sous-unité de la ribulose bis-phosphate carboxylase (RUBISCO) de tabac.

5. Procédé selon la revendication 1, dans lequel la première séquence d'ADN, qui est préparée à partir du génome de CaMV, comprend le double promoteur 3SS de CaMV.

6. Procédé selon la revendication 1, dans lequel la seconde séquence d'ADN peut être obtenue à partir d'une banque d'ADN construite à partir d'un tissu végétal induit à une résistance acquise systémique ou une résistance acquise localisée, au moyen d'inducteurs biologiques.

7. Procédé selon la revendication 1, dans lequel la seconde séquence d'ADN code pour une protéine en relation avec une phytopathogenèse, choisie dans le groupe constitué par PR-1A codée par la séquence 1, PR-1B codée par la séquence 2, PR-1C codée par la séquence 3, PR-R majeure codée par la séquence 4, PR-R mineure, PR-P codée par la séquence 6, PR-Q codée par la séquence 5, PR-2 codée par la séquence 8, PR-N, PR-O, PR-O' codée par la séquence 7A, SAR8.2a codée par la séquence 9, SAR8.2b codée par la séquence 10, la peroxydase basique de concombre codée par la séquence 12 et la glucanase basique de tabac.

8. Procédé selon la revendication 1, dans lequel la liaison de la première séquence d'ADN et de la seconde séquence d'ADN a été effectuée de telle façon que la seconde séquence d'ADN est orientée par rapport à la première séquence d'ADN de manière que la première séquence d'ADN active la transcription du brin sens de la seconde séquence d'ADN.

9. Procédé de la revendication 1, dans lequel la séquence d'ADN chimère comprend en outre une troisième séquence d'ADN qui est un marqueur de gène sélectionnable chez une plante.

10. Plante transgénique, y compris sa descendance, qui a été transformée par une molécule d'ADN chimère comprenant un promoteur qui active chez une plante la transcription constitutive d'une séquence d'ADN associée, fonctionnellement liée à une séquence d'ADN codante qui code pour une protéine inductible en relation avec une phytopathogenèse, ladite protéine en relation avec la pathogenèse n'étant pas une chitinase, de manière à exprimer constitutivement une protéine en relation avec la pathogenèse à un taux suffisant pour procurer un phénotype résistant à la maladie, ladite descendance comprenant ladite molécule d'ADN chimère, et ladite maladle étant provoquée par des champignons pathogènes.

11. Plante transgénique selon la revendication 10, dans laquelle lesdits champignons pathogènes sont choisis dans le groupe constitué par *Phytaphtora* et *Peronospora.*

12. Plante transgénlque selon la revendication 10, dans laquelle la protéine en relation avec la phytopathogenèse est choisie dans le groupe oonstitué par PR-1A codée par la séquence 1, PR-1B codée par la séquence 2, PR-1C codée par la séquence 3, PR-R majeure codée par la séquence 4, PR-R mineure, PR-P codée par la séquence 6, PR-Q codée par la séquence 5, PR-2 codée par la séquence 8, PR-N, PR-O, PR-O' codée par la séquence 7A, SAR8.2a codée par la séquence 9, SAR8.2b codée par la séquence 10, la peroxydase basique de concombre codée par la séquence 12 et la glucanase basique de tabac.

13. Plante transgénique selon la revendication 11 ou 12, qui est une plante choisie dans le groupe constitué par le tabac, la carotte, le tournesol, la tomate, le coton, le sorgho, *Petunlia* et *Glycine*.

14. Plante transgénique selon la revendication 11 ou 12, qui est une plante choisie dans le groupe constitué par *Zea mays, Dactylis* et *Lolium.*

15. Plante transgénique selon la revendication 14, qui est une plante de maïs.

16. Graine de plante transgénique obtenue à partir d'une plante de l'une quelconque des revendications 10 à 15, ladite graine comprenant ladite molécule d'ADN chimère.

17. Plante résistante à une maladie, produite par un procédé de la revendication 1, ladite maladie étant provoquée par des champignons pathogènes.

18. Plante résistante à une maladle selon la revendication 17, lesdits champignons pathogènes étant choisis dans le groupe constitvé par *Phytophtora* et *Peronospora*.

19. Plante résistante à une maladie produite par un procédé de l'une quelconque des revendications 3 à 9, ladite maladie étant provoquée par des champignons pathogènes.

20. Plante résistante à une maladie selon la revendication 19, lesdits champignons pathogènes étant choisis dans le groupe constitué par *Phytophtora* et *Peronospora*.

21. Tissu de plante exprimant constitutivement des taux induits de protéines en relation avec la phytopathogenèse ou exprimant constitutivement des taux de protéines en relation avec une pathogenèse, qui procurent un phénotype résistant à une maladle, étant entendu que les protéines en relation avec la pathogenèse sont d'origine hétérologue par rapport à la plante à transformer et ne sont pas une chitinase, ladite maladie étant provoquée par des champignons pathogènes.

22. Tissu de plante selon la revendication 21, dans lequel lesdits champignons pathogènes sont choisis dans le groupe constitué par *Phytophtora* et *Peronospora.*

23. Procédé de protection d'une plante contre l'endommagement provoqué par une maladie végétale, comprenant la transformation de cellules ou d'un tissu de plante par une séquence d'ADN chimère comprenant une première séquence d'ADN qui active chez une plante la transcription constitutive d'une seconde séquence d'ADN, et une seconde séquence d'ADN comprenant une séquence codante d'une protéine Inductible en relation avec la phytopathogenèse, ou une séquence codante ayant une homologle notable de séquence avec une séquence codante d'une protéine inductible en relation avec la phytopathogenèse, de sorte que la plante transformée manifeste un phénotype résistant à la maladie, et le fait de planter ladite plante transformée dans un environnement où la maladie peut apparaître, ladite protéine en relation avec la pathogenèse n'étant pas une chitinase et ladite maladie étant provoquée par des champignons pathogènes.

24. Procédé selon la revendication 23, dans lequel la seconde séquence d'ADN code pour une protéine en relation avec la phytopatnogenèse, choisie dans le groupe constitué par PR-1A codée par la séquence 1, PR-1B codée par la séquence 2, PR-1C codée par la séquence 3, PR-R majeure codée par la séquence 4, PR-R mineure, PR-P codée par la séquence 6, PR-Q codée par la séquence 5, PR-2 codée par la séquence 8, PR-N, PR-O, PR-O' codée par la séquence 7A, SAR8.2a codée par la séquence 9, SAR8.2b codée par la séquence 10, la peroxydase basique de concombre codée par la séquence 12 et la glucanase basique de tabac.

25. Procédé selon la revendication 23, dans lequel les champignons pathogènes sont ceux choisis dans le groupe constitué par *Phytophtora* et *Peronospora*.

26. Procédé selon la revendication 23, dans lequel la plante à protéger est une plante selon l'une quelconque des revendications 10 à 20.

27. Procédé selon la revendication 26, dans lequel la plante à protéger est une plante choisie dans le groupe constitué par le tabac, la carotte, le tournesol, la tomate, le coton, le sorgho, *Petunia* et *Glycine*.

28. Procédé selon la revendication 27, dans lequel la plante à protéger est une plante choisie dans le groupe constitué par *Zea mays, Dactylis* et *Lolium.*

29. Procédé selon la revendication 28, dans lequel la plante à protéger est une plante de maïs.
